# EUROPEAN PATENT APPLICATION

(11) **EP 3 412 669 A1**
(43) Date of publication of application: **12.12.2018**
(21) Application number: 17747001.0
(22) Date of filing: 04.02.2017
(51) Int. Cl.: C07D 471/14, C07D 471/04, A61K 31/4375, A61K 31/437, A61P 35/00, A61P 31/12, A61P 25/28, A61P 29/00, A61P 9/10

(54) **TRICYCLIC COMPOUND FOR BROMODOMAIN-CONTAINING PROTEIN INHIBITOR AND PREPARATION, PHARMACEUTICAL COMPOSITION, AND APPLICATION THEREOF**

(30) Priority: 05.02.2016 CN 201610080167
(71) Applicant: Chia Tai Tianqing Pharmaceutical Group Co.,Ltd, Lianyungang, Jiangsu 222062 (CN); Centaurus BioPharma Co., Ltd., Beijing 100195 (CN); Lianyungang Runzhong Pharmaceutical Co., Ltd., Lianyungang, Jiangsu 222069 (CN)
(72) Inventor: LI, Jijun, Beijing 100195 (CN); WU, Wei, Beijing 100195 (CN); ZHU, Yan, Beijing 100195 (CN); WANG, Huting, Beijing 100195 (CN); ZHAO, Lijia, Beijing 100195 (CN); HE, Weinan, Beijing 100195 (CN); SUN, Yinghui, Beijing 100195 (CN); PENG, Yong, Beijing 100195 (CN); HAN, Yongxin, Beijing 100195 (CN)
(74) Representative: Maiwald Patent- und Rechtsanwaltsgesellschaft mbH
(86) International application number: PCT/CN2017/072878
(87) International publication number: WO 2017/133681

(57) **Abstract**

The present applicationpresent application relates to a compound represented by Formula (III) or a pharmaceutically acceptable salt, solvent compound, active metabolite, crystal polymorph, ester, isomer, or prodrug thereof. The application further provides a pharmaceutical composition comprising the compound represented by Formula (III) and a use thereof for preparing a bromodomain inhibitor for preventing or treating various diseases, such as inflammation and cancer, related to the bromodomain.

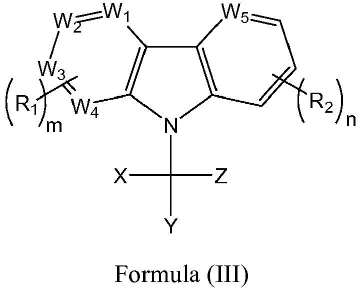

## Description

### CROSS-REFERENCE TO RELATED APPLICATIONS

The present application claims the priority and benefit of Chinese Patent Application No. 201610080167.2 filed before the State Intellectual Property Office of China on February 5, 2016, the disclosure of which is incorporated herein by reference in their entirety.

### TECHNICAL FIELD

The present application belongs to the field of pharmaceutical chemistry, specifically relates to a series of polysubstituted tricyclic compounds, and particularly relates to tricyclic compounds as bromodomain protein inhibitors, methods for preparing the same, and pharmaceutical compositions comprising the tricylic compounds. The present application further relates to use of the tricyclic compounds for preventing and treating hyperproliferative diseases, especially tumor diseases, and use thereof for treating viral infection, neurodegenerative diseases, inflammatory diseases and atherosclerotic diseases.

### BACKGROUND

The epigenetic regulation of transcriptional genes plays an important role in the development of tumors, inflammations, and metabolic diseases. The acetylation of N-terminal residues of nucleosomal histone lysine is particularly important for the regulation of epigenetic genes. However, histone acetylation is usually most commonly associated with the activation of gene transcription, and the recognition of histone-lysine acetylation is a key step in the epigenetic regulation by histone acetylation. Bromodomains (BRDs) are a kind of conserved protein domains that can specifically recognize acetylated lysine (KAc) in histones, promote the related proteins, such as chromatin remodeling factors, transcription factors and the like, to be enriched in specific gene transcription sites by binding to acetylated lysine, and change the activity of RNA II polymerase, thereby synergistically completing gene expression regulation (Filippakopoulos P, Picaud S, Mangos M, et al. Cell, 2012, 149 (1): 214-231).

BET (Bromodomain and Extra Terminal) proteins include two mutually associated bromodomain centers and an extra-terminal domain, and are classified into four kinds of proteins: Brd2, Brd3, Brd4 and BrdT, according to different amino acid sequences, wherein Brd2-Brd4 are widely distributed in human organs. BET is a kind of transcriptional regulatory proteins, and plays a very important role in regulating gene expression through the interaction with chromatins. BET proteins have bi-directional regulatory functions for co-activation or co-suppression of intracellular reticular signal transduction pathways, such as transcription of insulin, adipogenesis in lipid tissues, and differentiation of the hematopoietic system, etc. (Belkina A C, Denis G V, Nature Reviews Cancer, 2012, 12(7): 465-477). In recent years, researches have shown that BET protein-targeted drugs can be used to treat cancers (Zuber J, Shi J, Wang E, et al. Nature, 2011, 478(7370): 524-528), inflammation (Huang B, Yang X D, Zhou M M, et al. Molecular and Cellular Biology, 2009, 29(5): 1375-1387), nephrosis (Zhang G, Liu R, Zhong Y, et al. Journal of Biological Chemistry, 2012, 287(34): 28840-28851), autoimmune diseases (Denis G V. Discovery Medicine, 2010, 10(55): 489) and male antifertility (Matzuk M M, McKeown M R, Filippakopoulos P, et al. Cell, 2012, 150(4): 673-684), etc. Therefore, BET proteins have increasingly become one of the important targets in the epigenetic field, and have attracted great attentions from many giant pharmaceutical companies and scientific research institutions.

So far, a plurality of small molecular inhibitors selectively targeting the bromodomain of BET proteins have been reported (WO2006129623, WO2009084693, WO2009158404, WO2011143669, WO2011161031, WO2012075383, WO2013030150, WO2013097601, US2015246923, etc.), but only a few candidate drugs (RVX-208, CPI-0610, OTX015, GSK525762, TEN-010 etc.) are in the clinical trial study phase.

### SUMMARY OF THE INVENTION

The present application targets BET proteins and has developed a novel tricyclic small molecular compound for the treatment of tumors, inflammations, and metabolic diseases, etc. Compared with previous BET protein inhibitors, the compounds of the present application have higher activities.

The present application provides a series of compounds represented by general Formula (III) and use thereof for preventing and treating hyperproliferative diseases, especially tumor diseases. The present application further relates to a pharmaceutical composition comprising a compound represented by Formula (III), methods for preparing the same, and use of the compound for treating viral infection, neurodegenerative diseases, inflammatory diseases and atherosclerotic diseases.

In one aspect of the present application, the present application provides a compound represented by Formula (III) as shown below or a pharmaceutically acceptable salt, solvate, active metabolite, polymorph, ester, isomer or prodrug thereof, 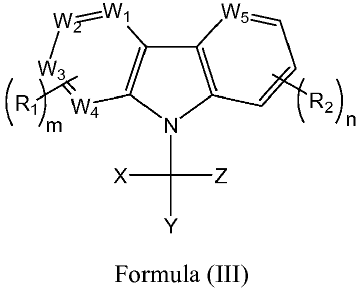 wherein
W₁, W₂, W₃ and W₄ are each independently selected from the group consisting of CH and N, and at least one of them is N;
W₅ is N or CR₃;
X and Y are each independently selected from the group consisting of optionally substituted C₁₋₆ alkyl, optionally substituted C₃₋₈ cycloalkyl, optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S;
Z is selected from the group consisting of hydrogen and C₁₋₆ alkyl;
R₁ is independently selected from the group consisting of halogen, -CN, -OH, -NH₂, -NH-optionally substituted C₁₋₆ alkyl, -C(=O)NH₂, -C(=O)-optionally substituted C₁₋₆ alkyl, - C(=O)N(CH₃)-OCH₃, -C(=O)NH-optionally substituted C₁₋₆ alkyl, -COOH, -C(=O)O-optionally substituted C₁₋₆ alkyl, -OC(=O)NH-optionally substituted C₁₋₆ alkyl, -NHOC(=O)-optionally substituted C₁₋₆ alkyl, -NHC(=O)NH-optionally substituted C₁₋₆ alkyl, -NHSO₂NH-optionally substituted C₁₋₆ alkyl, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₈ cycloalkyl, optionally substituted C₃₋₈ cycloalkyl-CO-, optionally substituted C₃₋₈ cycloalkyl-SO₂-, optionally substituted aryl C₁₋₆ alkoxy, optionally substituted C₃₋₈ cycloalkyl-C₁₋₆ alkoxy, optionally substituted heterocycloalkyl-CO-, optionally substituted heterocycloalkyl, optionally substituted C₁₋₆ alkyl-SO₂-, -NHSO₂-optionally substituted C₁₋₆ alkyl, -N(SO₂-optionally substituted C₁₋₆ alkyl)₂, -NHSO₂-optionally substituted heterocycloalkyl, optionally substituted C₁₋₆ alkyl-NHSO₂- and optionally substituted heterocycloalkyl-NHSO₂-;
R₂ is independently selected from the group consisting of optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S;
R₃ is selected from the group consisting of hydrogen, halogen, -CN, -OH, -NH₂, - NH-C₁₋₆ alkyl, -C(=O)NH-C₁₋₆ alkyl, -COOH, -C(=O)O-C₁₋₆ alkyl, C₁₋₆ alkyl, C₁₋₆ alkoxy and C₃₋₈ cycloalkyl;
m is selected from the group consisting of 0, 1, 2 and 3; and
n is selected from the group consisting of 1, 2 and 3.

In some embodiments, W₁ and W₃ are CH, and one of W₂ and W₄ is N and the other is CH.

In some embodiments, one of W₁, W₂, W₃ and W₄ is N, and the others are CH.

In some embodiments, X and Y are each independently selected from the group consisting of C₁₋₆ alkyl, C₃₋₈ cycloalkyl, 6- to 10-membered aryl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, aryl, heteroaryl and heterocycloalkyl may be optionally substituted by halogen, C₁₋₆ alkyl or C₁₋₆ alkoxy;
preferably, X and Y are each independently selected from the group consisting of C₁₋₆ alkyl, C₃₋₈ cycloalkyl, phenyl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, phenyl, heteroaryl and heterocycloalkyl may be optionally substituted by halogen, C₁₋₆ alkyl or C₁₋₆ alkoxy; and
more preferably, X and Y are each independently selected from the group consisting of C₁₋₆ alkyl, C₃₋₈ cycloalkyl, phenyl, pyridyl, thiazolyl and 3- to 8-membered heterocycloalkyl containing 1-3 heteroatoms independently selected from the group consisting of N, O and S, wherein the alkyl, cycloalkyl, phenyl, pyridyl, thiazolyl and heterocycloalkyl may be optionally substituted by halogen, C₁₋₆ alkyl or C₁₋₆ alkoxy.

In some embodiments, X is selected from the group consisting of C₁₋₆ alkyl, phenyl and pyridyl, and Y is selected from the group consisting of C₁₋₆ alkyl, C₃₋₈ cycloalkyl, phenyl, 5-to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, phenyl, pyridyl, cycloalkyl, heteroaryl and heterocycloalkyl may be optionally substituted by halogen, C₁₋₆ alkyl or C₁₋₆ alkoxy.

In some embodiments, X is selected from the group consisting of phenyl and pyridyl, and Y is selected from the group consisting of C₁₋₆ alkyl, C₃₋₈ cycloalkyl, and 3- to 8-membered heterocycloalkyl containing 1-3 heteroatoms independently selected from the group consisting of N, O and S, wherein the phenyl, pyridyl, alkyl, cycloalkyl and heterocycloalkyl may be optionally substituted by halogen, C₁₋₆ alkyl or C₁₋₆ alkoxy.

In some embodiments, X is selected from the group consisting of 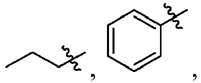 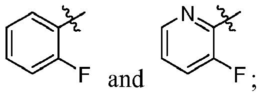 and Y is selected from the group constisting of

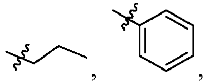

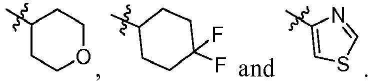

In some embodiments, X is selected from the group consisting of 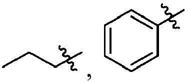 and 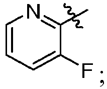 and Y is selected from the group consisting of

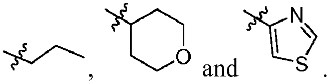

In some embodiments, Z is hydrogen.

In some embodiments, R₁ is independently selected from the group consisting of halogen, -CN, -OH, -NH₂, -NH-C₁₋₆ alkyl, -C(=O)NH₂, -C(=O)-C₁₋₆ alkyl, -C(=O)N(CH₃)-OCH₃, -C(=O)NH-C₁₋₆ alkyl, -COOH, -C(=O)O-C₁₋₆ alkyl, C₁₋₆ alkyl, C₂₋₆ alkenyl, -C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, -NHSO₂-C₁₋₆ alkyl and - N(SO₂-C₁₋₆ alkyl)₂, wherein the alkyl, alkenyl, cycloalkyl and heterocycloalkyl may be optionally substituted by -F, -Cl, -Br, -OH or -NH₂;
preferably, R₁ is independently selected from the group consisting of -OH, -NH₂, - NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, - SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or -NH₂;
more preferably, R₁ is independently selected from the group consisting of -OH, - NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and -N(SO₂-C₁₋₆ alkyl)₂, wherein the alkyl and alkenyl may be optionally substituted by -F, -Cl, -Br, -OH or - NH₂; and
further preferably, R₁ is selected from the group consisting of -F, -OH,

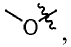

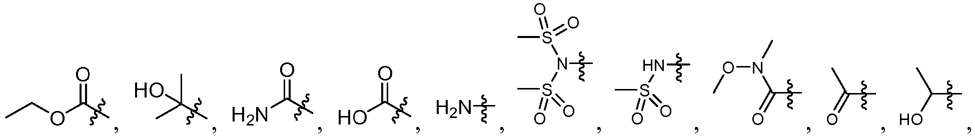

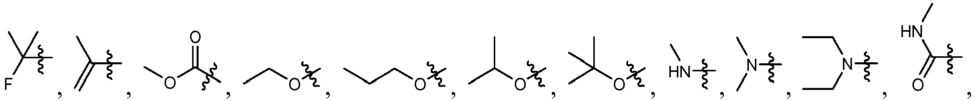

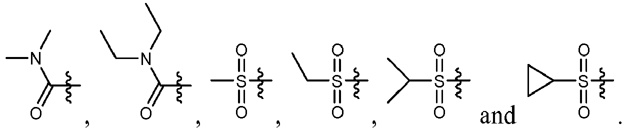

In some embodiments, R₂ is independently selected from the group consisting of 5-to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl, C₂₋₆ alkenyl or C₃₋₈ cycloalkyl; and
preferably, R₂ is independently selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl.

In some embodiments, R₂ is independently selected from the group consisting of the following structures: 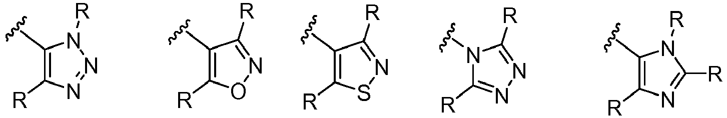 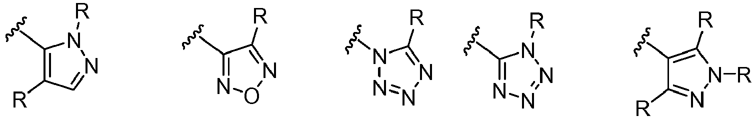 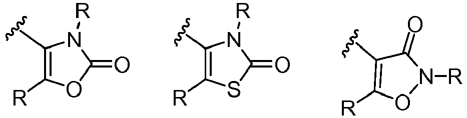 wherein R is independently selected from the group consisting of hydrogen, optionally substituted C₁₋₆ alkyl, optionally substituted C₃₋₈ cycloalkyl-C₁₋₆ alkyl, optionally substituted aryl-C₁₋₆ alkyl, optionally substituted heteroaryl-C₁₋₆ alkyl, optionally substituted heterocycloalkyl-C₁₋₆ alkyl, optionally substituted C₁₋₆ alkyl-CO-, optionally substituted aryl-CO-, optionally substituted C₃₋₈ cycloalkyl-CO-, optionally substituted heteroaryl, optionally substituted heterocycloalkyl-CO-, optionally substituted aryl-SO₂-, optionally substituted C₁₋₆ alkyl-SO₂-, optionally substituted C₃₋₈ cycloalkyl-SO₂-, optionally substituted heteroaryl-SO₂-, optionally substituted C₁₋₆ alkyl-OCO- and optionally substituted C₃₋₈ cycloalkyl-OCO-;
preferably, R is independently selected from the group consisting of hydrogen, C₁₋₆ alkyl, C₃₋₈ cycloalkyl, C₁₋₆ alkyl-CO-, aryl-CO-, C₃₋₈ cycloalkyl-CO-, heteroaryl, heterocycloalkyl-CO-, aryl-SO₂-, C₁₋₆ alkyl-SO₂-, C₃₋₈ cycloalkyl-SO₂-, heteroaryl-SO₂-, C₁₋₆ alkyl-OCO- and C₃₋₈ cycloalkyl-OCO-;
more preferably, R is independently selected from the group consisting of hydrogen, C₁₋₆ alkyl and heteroaryl; and
most preferably, R is independently selected from the group consisting of hydrogen and C₁₋₆ alkyl.

In some embodiments, R₂ is selected from the group consisting of 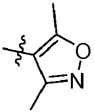 and

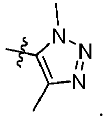

In some embodiments, R₃ is selected from the group consisting of hydrogen, halogen, C₁₋₆ alkyl and C₃₋₈ cycloalkyl; and
preferably, R₃ is selected from the group consisting of hydrogen, halogen and C₁₋₆ alkyl.

In some embodiments, R₃ is hydrogen or F.

In some embodiments, m is selected from the group consisting of 0, 1 and 2, and n is selected from the group consisting of 1 and 2.

In some embodiments, m and n are 1.

In some embodiments, m is 1, and R₁ is connected to W₂ or W₃.

In some embodiments, n is 1, and R₂ is at a meta-position of W₅.

In some embodiments, in the compound represented by Formula (III) or a pharmaceutically acceptable salt, solvate, active metabolite, polymorph, ester, isomer or prodrug thereof,
W₁, W₂, W₃ and W₄ are each independently selected from the group consisting of CH and N, and at least one of them is N;
W₅ is N or CR₃;
X and Y are each independently selected from the group consisting of C₁₋₆ alkyl, C₃₋₈ cycloalkyl, 6- to 10-membered aryl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, heteroaryl and heterocycloalkyl may be optionally substituted by halogen, C₁₋₆ alkyl or C₁₋₆ alkoxy;
Z is selected from the group consisting of hydrogen and C₁₋₆ alkyl;
R₁ is independently selected from the group consisting of halogen, -CN, -OH, -NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, - SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -OH or -NH₂;
R₂ is independently selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl, C₂₋₆ alkenyl or C₃₋₈ cycloalkyl;
R₃ is selected from the group consisting of hydrogen, halogen, C₁₋₆ alkyl and C₃₋₈ cycloalkyl;
m is selected from the group consisting of 0, 1 and 2; and
n is selected from the group consisting of 1 and 2.

In some embodiments, in the compound represented by Formula (III) or a pharmaceutically acceptable salt, solvate, active metabolite, polymorph, ester, isomer or prodrug thereof,

W₁ and W₃ are CH, and one of W₂ and W₄ is N and the other is CH;
W₅ is N or CR₃, and R₃ is selected from the group consisting of hydrogen, halogen and C₁₋₆ alkyl;
X and Y are each independently selected from the group consisting of C₁₋₆ alkyl, C₃₋₈ cycloalkyl, phenyl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, phenyl, heteroaryl and heterocycloalkyl may be optionally substituted by halogen, C₁₋₆ alkyl or C₁₋₆ alkoxy; and
Z is hydrogen.

In some embodiments, in the compound represented by Formula (III) or a pharmaceutically acceptable salt, solvate, active metabolite, polymorph, ester, isomer or prodrug thereof,
R₁ is independently selected from the group consisting of -OH, -NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and -N(SO₂-C₁₋₆ alkyl)₂, wherein the alkyl and alkenyl may be optionally substituted by -OH; and
R₂ is independently selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl.

In some embodiments, the compound represented by Formula (III) according to the present application does not comprise the following compounds or stereoisomers thereof:

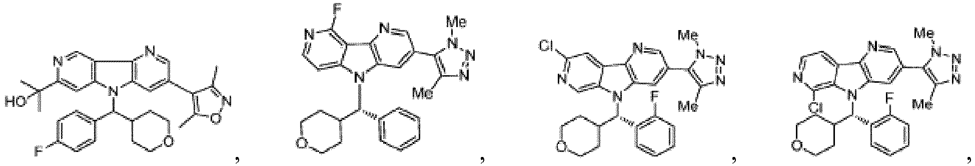

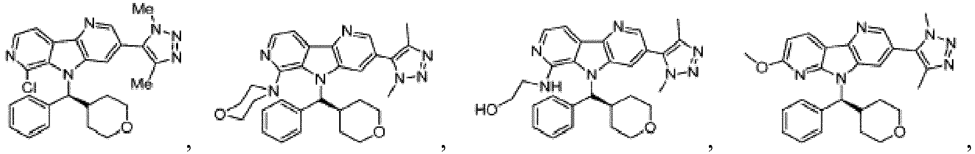

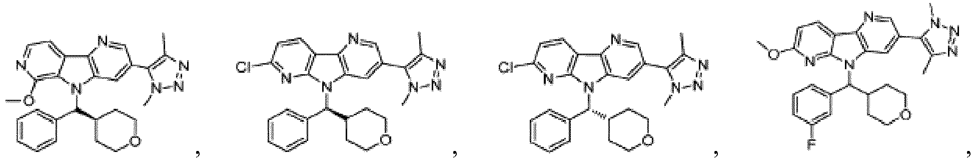

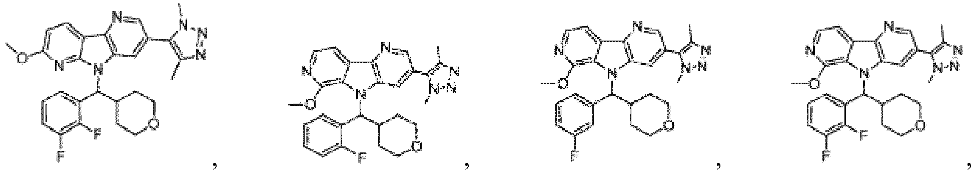

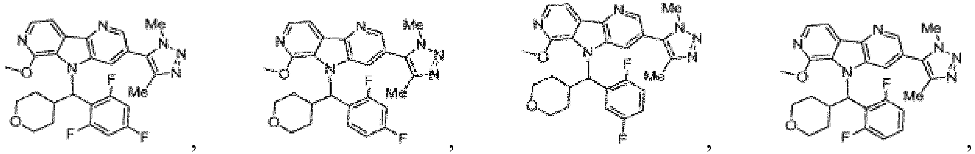

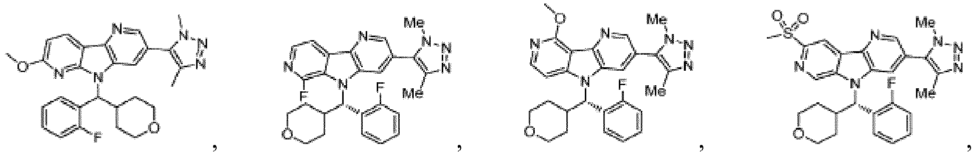

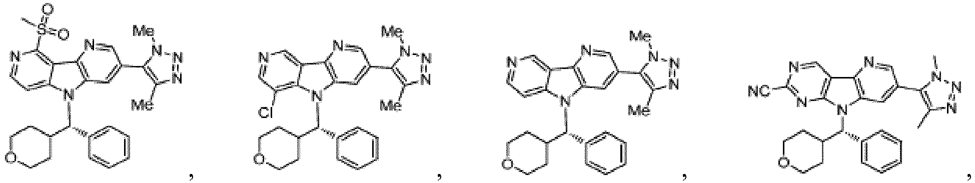

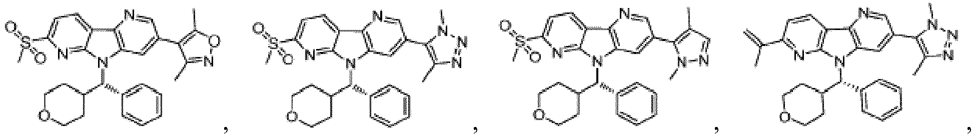

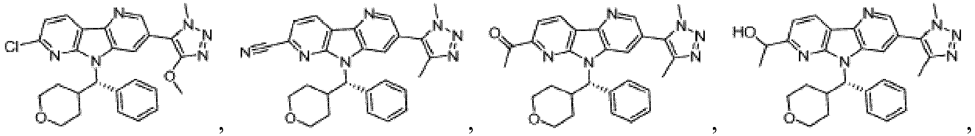

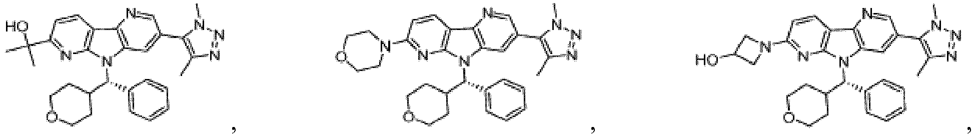

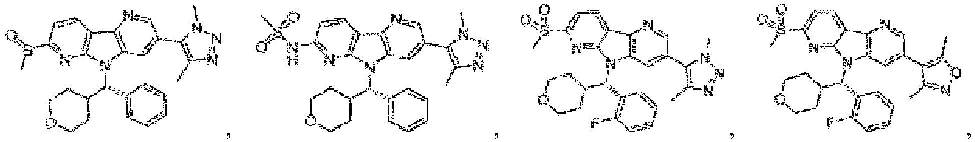

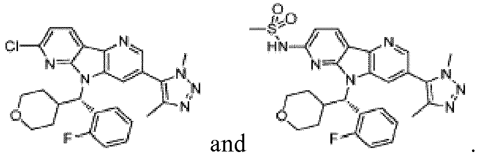

In another aspect, the present application relates to a compound represented by Formula (IV) or a pharmaceutically acceptable salt, solvate, active metabolite, polymorph, ester, isomer or prodrug thereof, 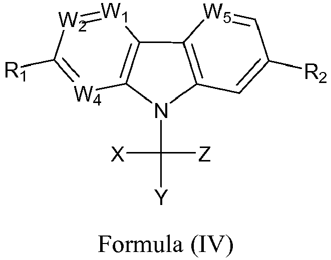 wherein
W₁, W₂ and W₄ are each independently CH or N, and at least one of them is N;
W₅ is N or CR₃;
X and Y are each independently selected from the group consisting of optionally substituted C₁₋₆ alkyl, optionally substituted C₃₋₈ cycloalkyl, optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S;
Z is selected from the group consisting of hydrogen and C₁₋₆ alkyl;
R₁ is selected from the group consisting of halogen, -CN, -OH, -NH₂, -NH-optionally substituted C₁₋₆ alkyl, -C(=O)NH₂, -C(=O)-optionally substituted C₁₋₆ alkyl, - C(=O)N(CH₃)-OCH₃, -C(=O)NH-optionally substituted C₁₋₆ alkyl, -COOH, -C(=O)O-optionally substituted C₁₋₆ alkyl, -OC(=O)NH-optionally substituted C₁₋₆ alkyl, -NHOC(=O)-optionally substituted C₁₋₆ alkyl, -NHC(=O)NH-optionally substituted C₁₋₆ alkyl, -NHSO₂NH-optionally substituted C₁₋₆ alkyl, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₈ cycloalkyl, optionally substituted C₃₋₈ cycloalkyl-CO-, optionally substituted C₃₋₈ cycloalkyl-SO₂-, optionally substituted aryl C₁₋₆ alkoxy, optionally substituted C₃₋₈ cycloalkyl-C₁₋₆ alkoxy, optionally substituted heterocycloalkyl-CO-, optionally substituted heterocycloalkyl, optionally substituted C₁₋₆ alkyl-SO₂-, -NHSO₂-optionally substituted C₁₋₆ alkyl, -N(SO₂-optionally substituted C₁₋₆ alkyl)₂, -NHSO₂-optionally substituted heterocycloalkyl, optionally substituted C₁₋₆ alkyl-NHSO₂- and optionally substituted heterocycloalkyl-NHSO₂-;
R₂ is selected from the group consisting of optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S; and
R₃ is selected from the group consisting of hydrogen, halogen, -CN, -OH, -NH₂, - NH-C₁₋₆ alkyl, -C(=O)NH-C₁₋₆ alkyl, -COOH, -C(=O)O-C₁₋₆ alkyl, C₁₋₆ alkyl, C₁₋₆ alkoxy and C₃₋₈ cycloalkyl.

In some embodiments, W₁ is CH, and one of W₂ and W₄ is N and the other is CH.

In some embodiments, one of W₁, W₂ and W₄ is N, and the other two are CH.

In some embodiments, X and Y are each independently selected from the group consisting of C₁₋₆ alkyl, C₃₋₈ cycloalkyl, 6- to 10-membered aryl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, aryl, heteroaryl and heterocycloalkyl may be optionally substituted by halogen, C₁₋₆ alkyl or C₁₋₆ alkoxy;

preferably, X and Y are each independently selected from the group consisting of C₁₋₆ alkyl, C₃₋₈ cycloalkyl, phenyl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, phenyl, heteroaryl and heterocycloalkyl may be optionally substituted by halogen, C₁₋₆ alkyl or C₁₋₆ alkoxy; and

more preferably, X and Y are each independently selected from the group consisting of C₁₋₆ alkyl, C₃₋₈ cycloalkyl, phenyl, pyridyl, thiazolyl and 3- to 8-membered heterocycloalkyl containing 1-3 heteroatoms independently selected from the group consisting of N, O and S, wherein the alkyl, cycloalkyl, phenyl, pyridyl, thiazolyl and heterocycloalkyl may be optionally substituted by halogen, C₁₋₆ alkyl or C₁₋₆ alkoxy.

In some embodiments, X is selected from the group consisting of C₁₋₆ alkyl, phenyl and pyridyl, and Y is selected from the group consisting of C₁₋₆ alkyl, C₃₋₈ cycloalkyl, phenyl, 5-to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, phenyl, pyridyl, cycloalkyl, heteroaryl and heterocycloalkyl may be optionally substituted by halogen, C₁₋₆ alkyl or C₁₋₆ alkoxy.

In some embodiments, X is selected from the group consisting of phenyl and pyridyl, and Y is selected from the group consisting of C₁₋₆ alkyl, C₃₋₈ cycloalkyl, and 3- to 8-membered heterocycloalkyl containing 1-3 heteroatoms independently selected from the group consisting of N, O and S, wherein the phenyl, pyridyl, alkyl, cycloalkyl and heterocycloalkyl may be optionally substituted by halogen, C₁₋₆ alkyl or C₁₋₆ alkoxy.

In some embodiments, X is selected from the group consisting of 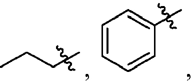 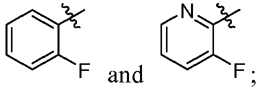 and Y is selected from the group consisting of

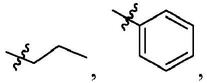

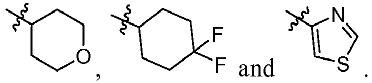

In some embodiments, X is selected from the group consisting of 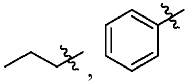 and 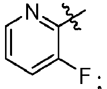 and Y is selected from the group consisting of

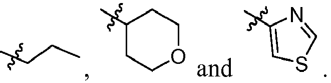

In some embodiments, Z is hydrogen.

In some embodiments, R₁ is selected from the group consisting of halogen, -CN, - OH, -NH₂, -NH-C₁₋₆ alkyl, -C(=O)NH₂, -C(=O)-C₁₋₆ alkyl, -C(=O)N(CH₃)-OCH₃, -C(=O)NH-C₁₋₆ alkyl, -COOH, -C(=O)O-C₁₋₆ alkyl, C₁₋₆ alkyl, C₂₋₆ alkenyl, -C₁₋₆ alkoxy, C₃₋₈ cycloalkyl, 3-to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by F, Cl, Br, -OH or -NH₂;

preferably, R₁ is selected from the group consisting of -OH, -NH₂, -NH-C₁₋₆ alkyl, - C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, - NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or -NH₂;
more preferably, R₁ is selected from the group consisting of -OH, -NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and -N(SO₂-C₁₋₆ alkyl)₂, wherein the alkyl and alkenyl may be optionally substituted by F, Cl, Br, -OH or -NH₂; and
further preferably, R₁ is selected from the group consisting of -F, -OH,

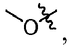

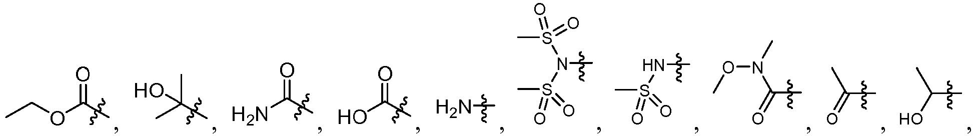

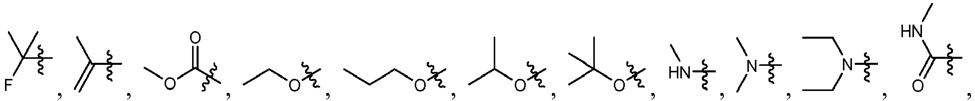

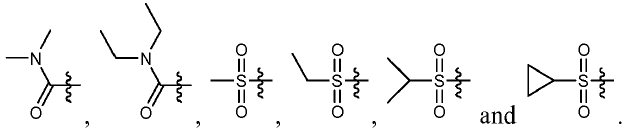

In some embodiments, R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl, C₂₋₆ alkenyl or C₃₋₈ cycloalkyl; and

preferably, R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl.

In some embodiments, R₂ is selected from the group consisting of the following structures: 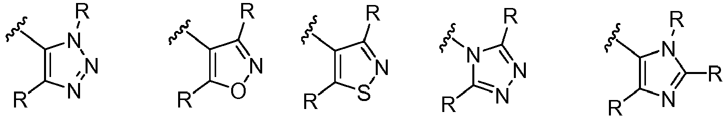 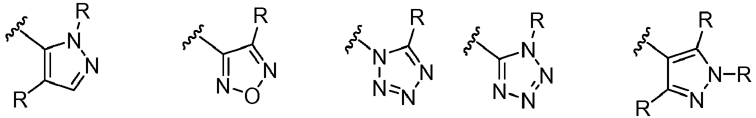 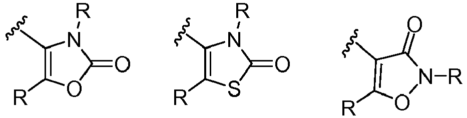
R is selected from the group consisting of hydrogen, optionally substituted C₁₋₆ alkyl, optionally substituted C₃₋₈ cycloalkyl-C₁₋₆ alkyl, optionally substituted aryl-C₁₋₆ alkyl, optionally substituted heteroaryl-C₁₋₆ alkyl, optionally substituted heterocycloalkyl-C₁₋₆ alkyl, optionally substituted C₁₋₆ alkyl-CO-, optionally substituted aryl-CO-, optionally substituted C₃₋₈ cycloalkyl-CO-, optionally substituted heteroaryl, optionally substituted heterocycloalkyl-CO-, optionally substituted aryl-SO₂-, optionally substituted C₁₋₆ alkyl-SO₂-, optionally substituted C₃₋₈ cycloalkyl-SO₂-, optionally substituted heteroaryl-SO₂-, optionally substituted C₁₋₆ alkyl-OCO- and optionally substituted C₃₋₈ cycloalkyl-OCO-;
preferably, R is selected from the group consisting of hydrogen, C₁₋₆ alkyl, C₃₋₈ cycloalkyl, C₁₋₆ alkyl-CO-, aryl-CO-, C₃₋₈ cycloalkyl-CO-, heteroaryl, heterocycloalkyl-CO-, aryl-SO₂-, C₁₋₆ alkyl-SO₂-, C₃₋₈ cycloalkyl-SO₂-, heteroaryl-SO₂-, C₁₋₆ alkyl-OCO- and C₃₋₈ cycloalkyl-OCO-;
more preferably, R is selected from the group consisting of hydrogen, C₁₋₆ alkyl and heteroaryl; and
most preferably, R is selected from the group consisting of hydrogen and C₁₋₆ alkyl.

In some embodiments, R₂ is selected from the group consisting of 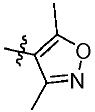 and

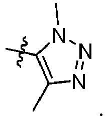

In some embodiments, R₃ is selected from the group consisting of hydrogen, halogen, C₁₋₆ alkyl and C₃₋₈ cycloalkyl; and

preferably, R₃ is selected from the group consisting of hydrogen, halogen and C₁₋₆ alkyl.

In some embodiments, R₃ is hydrogen or F.

In some embodiments, in the compound represented by Formula (IV) or a pharmaceutically acceptable salt, solvate, active metabolite, polymorph, ester, isomer or prodrug thereof,
W₁, W₂ and W₄ are each independently selected from the group consisting of CH and N, and at least one of them is N;
W₅ is N or CR₃;
X and Y are each independently selected from the group consisting of C₁₋₆ alkyl, C₃₋₈ cycloalkyl, 6- to 10-membered aryl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, heteroaryl and heterocycloalkyl may be optionally substituted by halogen, C₁₋₆ alkyl or C₁₋₆ alkoxy;
Z is selected from the group consisting of hydrogen and C₁₋₆ alkyl;
R₁ is selected from the group consisting of halogen, -CN, -OH, -NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, - NHSO₂-C₁₋₆ alkyl and -N(SO₂-C₁₋₆ alkyl)₂, wherein the alkyl, alkenyl, cycloalkyl and heterocycloalkyl may be optionally substituted by -OH or -NH₂;
R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl, C₂₋₆ alkenyl or C₃₋₈ cycloalkyl; and
R₃ is selected from the group consisting of hydrogen, halogen, C₁₋₆ alkyl and C₃₋₈ cycloalkyl.

In some embodiments, in the compound represented by Formula (IV) or a pharmaceutically acceptable salt, solvate, active metabolite, polymorph, ester, isomer or prodrug thereof,
W₁ is CH, and one of W₂ and W₄ is N and the other is CH;
W₅ is N or CR₃, and R₃ is selected from the group consisting of hydrogen, halogen and C₁₋₆ alkyl;
X and Y are each independently selected from the group consisting of C₁₋₆ alkyl, C₃₋₈ cycloalkyl, phenyl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, phenyl, heteroaryl and heterocycloalkyl may be optionally substituted by halogen, C₁₋₆ alkyl or C₁₋₆ alkoxy; and
Z is hydrogen.

In some embodiments, in the compound represented by Formula (IV) or a pharmaceutically acceptable salt, solvate, active metabolite, polymorph, ester, isomer or prodrug thereof,
R₁ is selected from the group consisting of -OH, -NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and -N(SO₂-C₁₋₆ alkyl)₂, wherein the alkyl and alkenyl may be optionally substituted by -OH; and
R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl.

In some embodiments, the compound represented by Formula (IV) according to the present application does not comprise the following compounds or stereoisomers thereof:

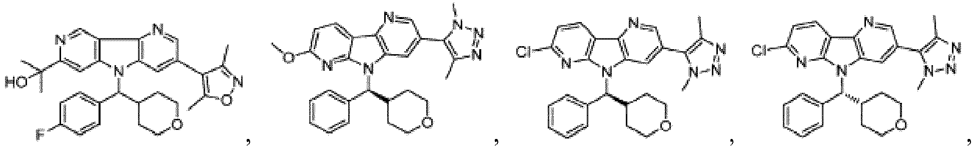

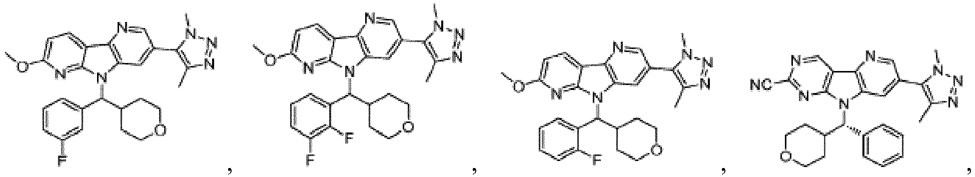

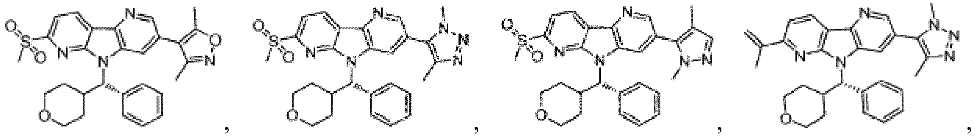

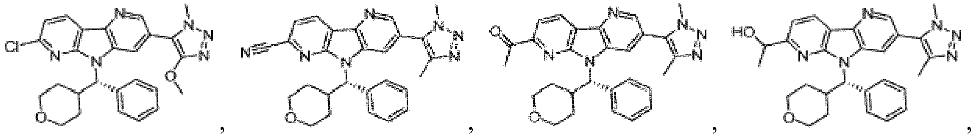

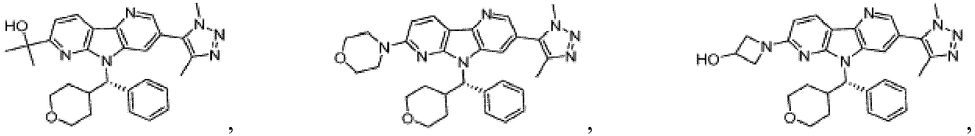

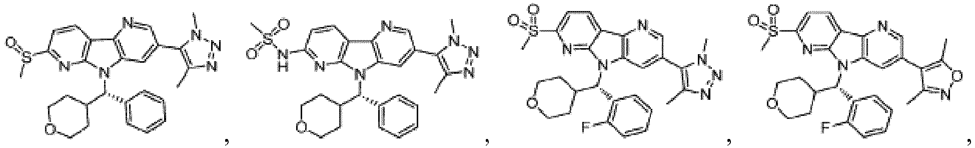

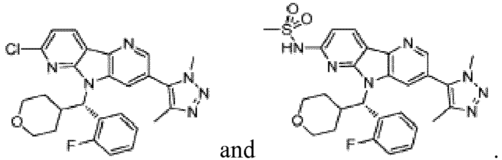

In another aspect, the present application relates to a compound represented by Formula (V) or a pharmaceutically acceptable salt, solvate, active metabolite, polymorph, ester, isomer or prodrug thereof, 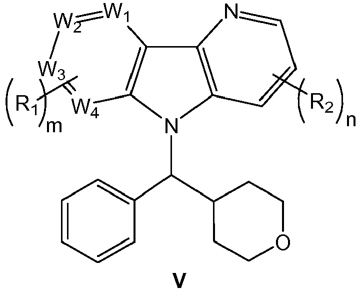 wherein
W₁, W₂, W₃ and W₄ are each independently selected from the group consisting of CH and N, and at least one of them is N;
R₁ is selected from the group consisting of halogen, -CN, -OH, -NH₂, -NH-optionally substituted C₁₋₆ alkyl, -C(=O)NH₂, -C(=O)-optionally substituted C₁₋₆ alkyl, - C(=O)N(CH₃)-OCH₃, -C(=O)NH-optionally substituted C₁₋₆ alkyl, -COOH, -C(=O)O-optionally substituted C₁₋₆ alkyl, -OC(=O)NH-optionally substituted C₁₋₆ alkyl, -NHOC(=O)-optionally substituted C₁₋₆ alkyl, -NHC(=O)NH-optionally substituted C₁₋₆ alkyl, -NHSO₂NH-optionally substituted C₁₋₆ alkyl, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₈ cycloalkyl, optionally substituted C₃₋₈ cycloalkyl-CO-, optionally substituted C₃₋₈ cycloalkyl-SO₂-, optionally substituted aryl C₁₋₆ alkoxy, optionally substituted C₃₋₈ cycloalkyl-C₁₋₆ alkoxy, optionally substituted heterocycloalkyl-CO-, optionally substituted heterocycloalkyl, optionally substituted C₁₋₆ alkyl-SO₂-, -NHSO₂-optionally substituted C₁₋₆ alkyl, -N(SO₂-optionally substituted C₁₋₆ alkyl)₂, -NHSO₂-optionally substituted heterocycloalkyl, optionally substituted C₁₋₆ alkyl-NHSO₂- and optionally substituted heterocycloalkyl-NHSO₂-;
R₂ is selected from the group consisting of optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S;
m is selected from the group consisting of 0, 1, 2 and 3; and
n is selected from the group consisting of 1, 2 and 3.

In some embodiments, one or two of W₁, W₂, W₃ and W₄ are N, and the others are CH; and preferably, one of W₁, W₂, W₃ and W₄ is N, and the others are CH.

In some embodiments, R₁ is selected from the group consisting of halogen, -CN, - OH, -NH₂, -NH-C₁₋₆ alkyl, -C(=O)NH₂, -C(=O)-C₁₋₆ alkyl, -C(=O)N(CH₃)-OCH₃, -C(=O)NH-C₁₋₆ alkyl, -COOH, -C(=O)O-C₁₋₆ alkyl, C₁₋₆ alkyl, C₂₋₆ alkenyl, -C₁₋₆ alkoxy, C₃₋₈ cycloalkyl, 3-to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, - Cl, -Br, -OH or -NH₂.

In some embodiments, preferably, R₁ is selected from the group consisting of -OH, - NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or - NH₂.

In some embodiments, more preferably, R₁ is selected from the group consisting of - OH, -NH₂, -NH-C₁₋₆ alkyl, -C(=O)NH₂, -C(=O)-C₁₋₆ alkyl, -C(=O)NH-C₁₋₆ alkyl, -COOH, - C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and - N(SO₂-C₁₋₆ alkyl)₂, wherein the alkyl and alkenyl may be optionally substituted by -F, -Cl, -Br, - OH or -NH₂.

In some embodiments, further preferably, R₁ is selected from the group consisting of

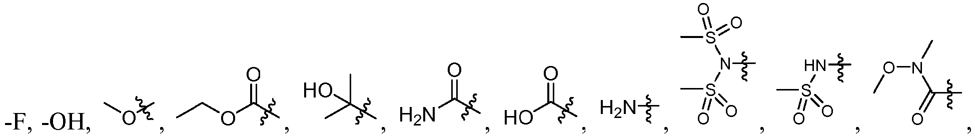

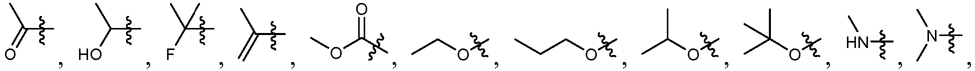

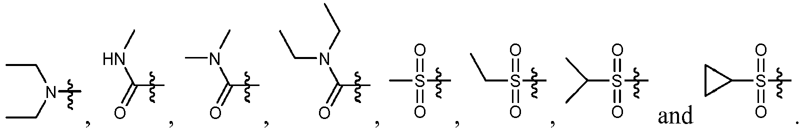

In some embodiments, R₁ is connected to W₁, W₂ or W₃; and preferably, R₁ is connected to W₂ or W₃.

In some embodiments, R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl, C₂₋₆ alkenyl or C₃₋₈ cycloalkyl.

In some embodiments, preferably, R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl.

In some embodiments, R₂ is independently selected from the group consisting of the following structures: 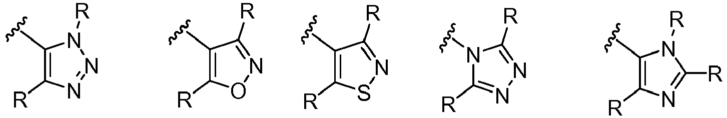 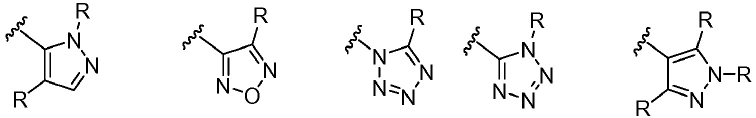 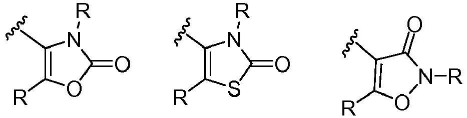 wherein R is selected from the group consisting of hydrogen, optionally substituted C₁₋₆ alkyl, optionally substituted C₃₋₈ cycloalkyl-C₁₋₆ alkyl, optionally substituted aryl-C₁₋₆ alkyl, optionally substituted heteroaryl-C₁₋₆ alkyl, optionally substituted heterocycloalkyl-C₁₋₆ alkyl, optionally substituted C₁₋₆ alkyl-CO-, optionally substituted aryl-CO-, optionally substituted C₃₋₈ cycloalkyl-CO-, optionally substituted heteroaryl, optionally substituted heterocycloalkyl-CO-, optionally substituted aryl-SO₂-, optionally substituted C₁₋₆ alkyl-SO₂-, optionally substituted C₃₋₈ cycloalkyl-SO₂-, optionally substituted heteroaryl-SO₂-, optionally substituted C₁₋₆ alkyl-OCO- and optionally substituted C₃₋₈ cycloalkyl-OCO-.

In some embodiments, preferably, R is selected from the group consisting of hydrogen, C₁₋₆ alkyl, C₃₋₈ cycloalkyl, C₁₋₆ alkyl-CO-, aryl-CO-, C₃₋₈ cycloalkyl-CO-, heteroaryl, heterocycloalkyl-CO-, aryl-SO₂-, C₁₋₆ alkyl-SO₂-, C₃₋₈ cycloalkyl-SO₂-, heteroaryl-SO₂-, C₁₋₆ alkyl-OCO- and C₃₋₈ cycloalkyl-OCO-.

In some embodiments, more preferably, R is selected from the group consisting of hydrogen, C₁₋₆ alkyl and heteroaryl.

In some embodiments, most preferably, R is selected from the group consisting of hydrogen and C₁₋₆ alkyl.

In some embodiments, R₂ is selected from the group consisting of 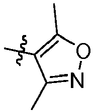 and

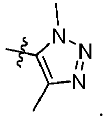

In some embodiments, m is selected from the group consisting of 0, 1 and 2, and n is selected from the group consisting of 1 and 2.

In some embodiments, m and n are 1.

In some embodiments, the compound represented by Formula (V) according to the present application does not comprise the following compounds or stereoisomers thereof:

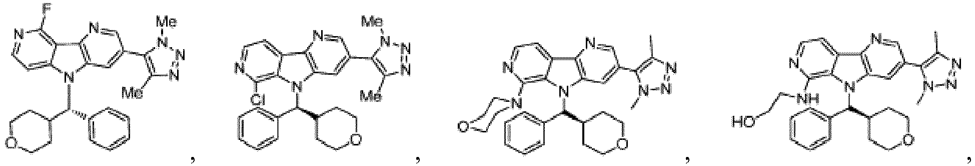

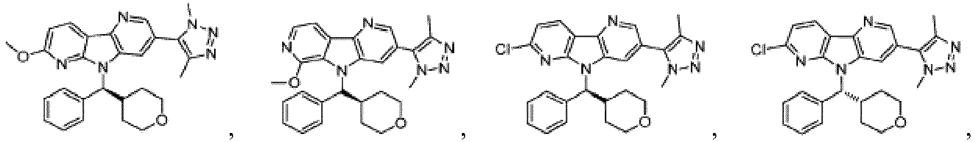

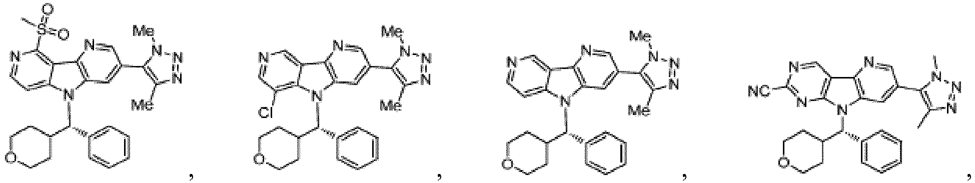

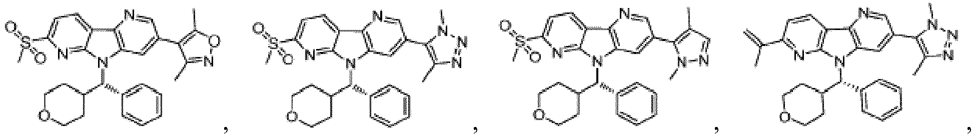

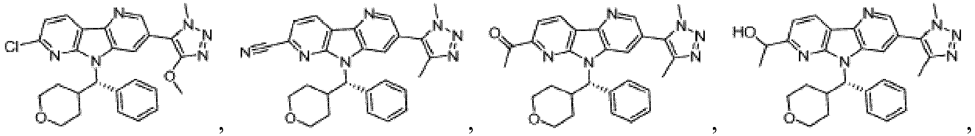

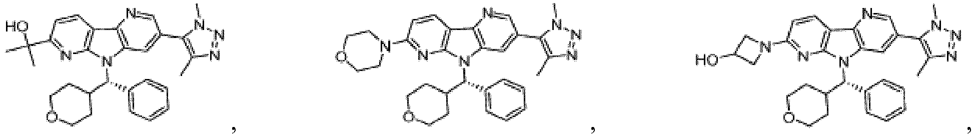

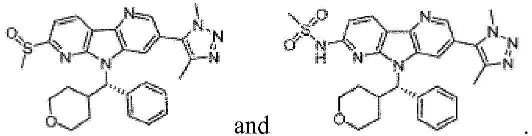

In another aspect, the present application relates to a compound represented by Formula (VI) or a pharmaceutically acceptable salt, solvate, active metabolite, polymorph, ester, isomer or prodrug thereof, 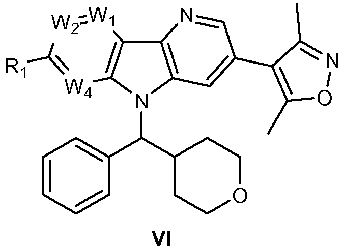 wherein
W₁, W₂ and W₄ are each independently CH or N, and at least one of them is N; and
R₁ is selected from the group consisting of halogen, -CN, -OH, -NH₂, -NH-optionally substituted C₁₋₆ alkyl, -C(=O)NH₂, -C(=O)-optionally substituted C₁₋₆ alkyl, - C(=O)N(CH₃)-OCH₃, -C(=O)NH-optionally substituted C₁₋₆ alkyl, -COOH, -C(=O)O-optionally substituted C₁₋₆ alkyl, -OC(=O)NH-optionally substituted C₁₋₆ alkyl, -NHOC(=O)-optionally substituted C₁₋₆ alkyl, -NHC(=O)NH-optionally substituted C₁₋₆ alkyl, -NHSO₂NH-optionally substituted C₁₋₆ alkyl, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₈ cycloalkyl, optionally substituted C₃₋₈ cycloalkyl-CO-, optionally substituted C₃₋₈ cycloalkyl-SO₂-, optionally substituted aryl C₁₋₆ alkoxy, optionally substituted C₃₋₈ cycloalkyl-C₁₋₆ alkoxy, optionally substituted heterocycloalkyl-CO-, optionally substituted heterocycloalkyl, optionally substituted C₁₋₆ alkyl-SO₂-, -NHSO₂-optionally substituted C₁₋₆ alkyl, -N(SO₂-optionally substituted C₁₋₆ alkyl)₂, -NHSO₂-optionally substituted heterocycloalkyl, optionally substituted C₁₋₆ alkyl-NHSO₂- and optionally substituted heterocycloalkyl-NHSO₂-.

In some embodiments, W₁ is CH, and one of W₂ and W₄ is N and the other is CH.

Preferably, W₂ is N, W₁ and W₄ are CH; or W₄ is N, and W₁ and W₂ are CH; or W₁ is N, and W₂ and W₄ are CH.

In some embodiments, R₁ is selected from the group consisting of halogen, -CN, - OH, -NH₂, -NH-C₁₋₆ alkyl, -C(=O)NH₂, -C(=O)-C₁₋₆ alkyl, -C(=O)N(CH₃)-OCH₃, -C(=O)NH-C₁₋₆ alkyl, -COOH, -C(=O)O-C₁₋₆ alkyl, C₁₋₆ alkyl, C₂₋₆ alkenyl, -C₁₋₆ alkoxy, C₃₋₈ cycloalkyl, 3-to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, - Cl, -Br, -OH or -NH₂.

In some embodiments, preferably, R₁ is selected from the group consisting of -OH, - NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or - NH₂.

In some embodiments, more preferably, R₁ is selected from the group consisting of - OH, -NH₂, -NH-C₁₋₆ alkyl, -C(=O)NH₂, -C(=O)-C₁₋₆ alkyl, -C(=O)NH-C₁₋₆ alkyl, -COOH, - C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and - N(SO₂-C₁₋₆ alkyl)₂, wherein the alkyl and alkenyl may be optionally substituted by -F, -Cl, -Br, - OH or -NH₂.

In some embodiments, further preferably, R₁ is selected from the group consisting of

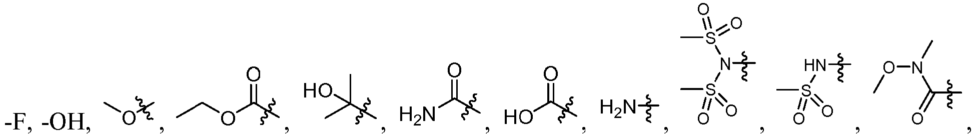

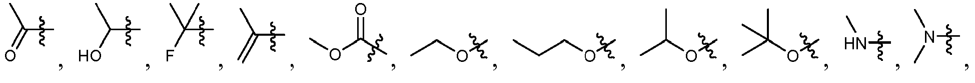

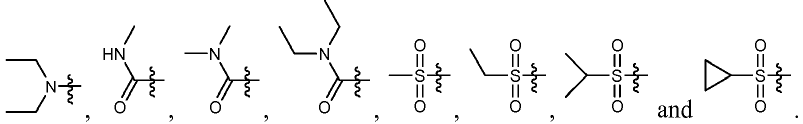

In some embodiments, the compound represented by Formula (VI) according to the present application does not comprise the following compounds or stereoisomers thereof:

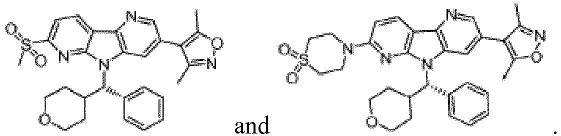

According to some embodiments of the present application, the present application provides the following compounds: 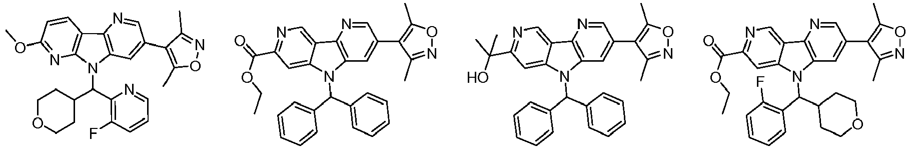 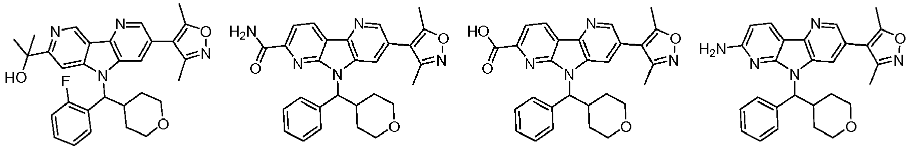 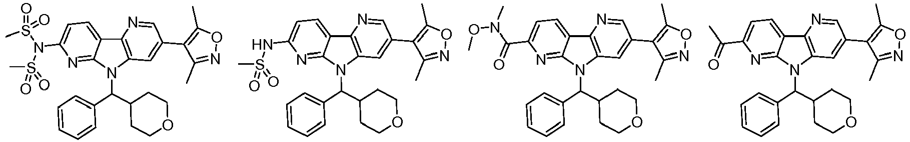 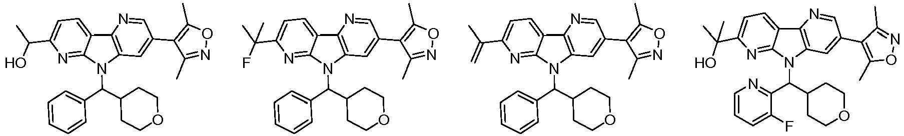 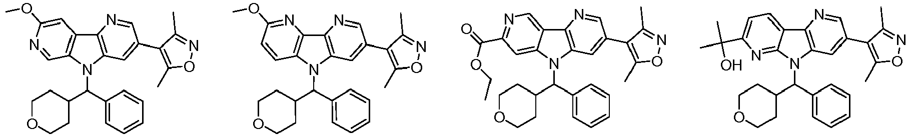 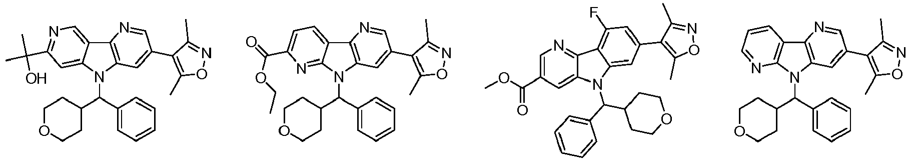 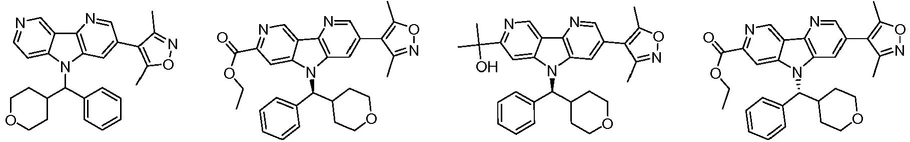 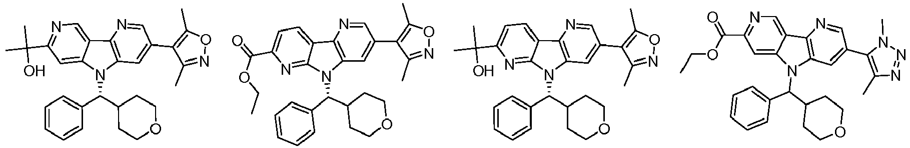 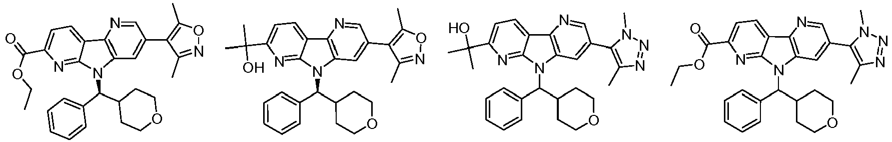 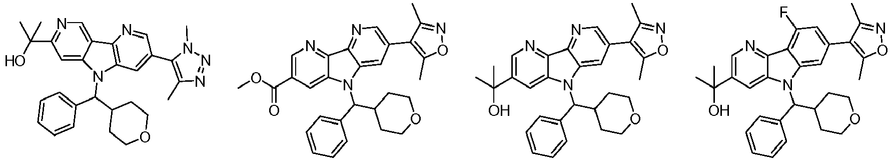 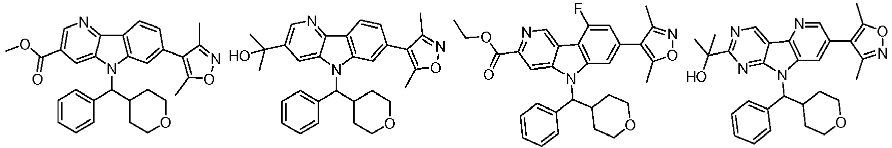 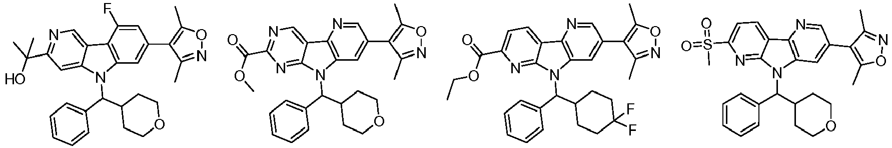 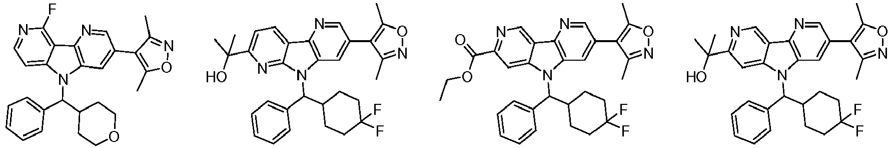 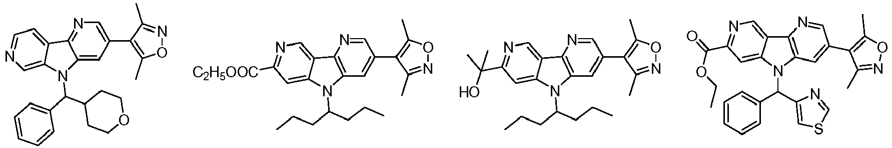 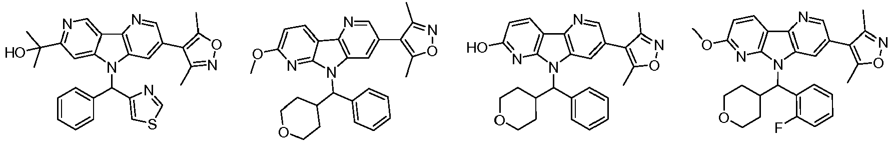 or a pharmaceutically acceptable salt, solvate, active metabolite, polymorph, ester, isomer or prodrug thereof.

In another aspect, the present application provides the compound or a pharmaceutically acceptable salt, solvate, active metabolite, polymorph, ester, isomer or prodrug thereof in the foregoing first aspect for use as a drug.

In another aspect, the present application provides a pharmaceutical composition comprising at least any one of the compound or a pharmaceutically acceptable salt, solvate, active metabolite, polymorph, ester, isomer or prodrug thereof provided herein. In some embodiments, the composition provided herein further comprises a pharmaceutically acceptable diluent, excipient and/or binder.

In another aspect, the present application provides use of the compound or a pharmaceutically acceptable salt, solvate, active metabolite, polymorph, ester, isomer or prodrug thereof or the pharmaceutical composition provided herein in the preparation of a medicament for treating a disease associated with BET proteins.

In another aspect, the present application provides a method for treating a disease associated with BET proteins, comprising administering to a patient in need thereof a therapeutically effective amount of at least any one of the compound or a pharmaceutically acceptable salt, solvate, active metabolite, polymorph, ester, isomer or prodrug thereof or the pharmaceutical composition provided herein.

In another aspect, the present application provides a method for inhibiting BET protein activities, comprising in vitro or in vivo test.

In another aspect, the present application provides methods for preparing the compounds represented by Formulae (III) and (IV) or pharmaceutically acceptable salts, solvates, polymorphs, tautomers or prodrugs thereof.

### DETAILED DESCRIPTION OF THE INVENTION

The new features of the present invention are specifically set forth in the claims of the present application. Illustrative embodiments using the principles of the present invention are set forth in the detailed description of the present invention hereinafter. The features and advantages of the present invention can be better understood by referring to the following contents of the present invention.

Although preferred embodiments of the present application are described herein, these embodiments are provided as examples only. It should be understood that variations of the embodiments of the present invention provided herein may also be used to implement the present invention. Those of ordinary skill in the art should understand that many variations, alterations and replacements may occur without departing from the scope of the present invention. It should be understood that the scopes of protection of the various aspects of the present invention are determined by the claims, and that the methods and structures within the scopes of these claims and their equivalent methods and structures are all within the scopes of the claims of the present application.

The section headings used herein are for the purpose of organizing text only and should not be construed as limiting the subject matters described. All documents or portions of documents cited in the present application include, but are not limited to, patents, patent applications, articles, books, manuals, and treatises, which are incorporated herein by reference in their entirety.

### Certain Chemical Terminology

Unless defined otherwise, all technical and scientific terms used herein have the same meaning as is commonly understood by one of skill in the art to which the claimed subject matters belong. All patents, patent applications, published materials referred to throughout the entire disclosure herein, unless noted otherwise, are incorporated herein by reference in their entirety. In the event that there is a plurality of definitions for terms herein, those in this section prevail. Where reference is made to a URL or other such identifier or address, it is understood that such identifiers can change and exchange with particular information on the internet, but equivalent information can be found by searching the internet or other appropriate reference sources. Reference thereto evidences the availability and public dissemination of such information.

It should be understood that the foregoing summary and the following detailed description are exemplary and explanatory only and are not restrictive of any subject matters claimed. In this application, the use of the singular includes the plural unless specifically stated otherwise. It must be noted that, as used in the specification and the claims, the singular forms include plural referents unless the context clearly dictates otherwise. It should also be noted that use of "or" means "and/or" unless stated otherwise. Furthermore, use of the term "comprise" as well as other forms, such as "comprises", "comprising", and "include" is not limiting.

Definition of standard chemistry terms may be found in reference works, including Carey and Sundberg "ADVANCED ORGANIC CHEMISTRY 4TH ED" Vols. A (2000) and B (2001), Plenum Press, New York. Unless otherwise indicated, conventional methods of mass spectroscopy, NMR, HPLC, protein chemistry, biochemistry, recombinant DNA techniques and pharmacology, within the skill of the art are employed. Unless specific definitions are provided, the nomenclature employed in connection with, and the laboratory procedures and techniques of, analytical chemistry, synthetic organic chemistry, and medicinal and pharmaceutical chemistry described herein are those known in the art. Standard techniques can be used for chemical syntheses, chemical analyses, pharmaceutical preparation, formulation, and delivery, and treatment of patients. Standard techniques can be used for recombinant DNA, oligonucleotide synthesis, and tissue culture and transformation (e.g., electroporation, and lipid transfection). For example, reactions and purification techniques can be performed using kits with specifications provided by manufacturers, or in accordance with methods well known in the art, or in accordance with the methods described in the present application. The foregoing techniques and procedures can be generally performed by conventional methods well known in the art and as described in various general and more specific references that are cited and discussed throughout the present application.

Where substituents are specified by their conventional chemical formulae, written from left to right, they equally encompass the chemically identical substituents that would result from writing the structure from right to left. As a non-limiting example, -CH₂O- is equivalent to -OCH₂-.

Unless otherwise noted, the use of general chemical terms, for example but not limited to "alkyl", "amine", "aryl" and the like are equivalent to their optionally substituted forms. For example, "alkyl" as used herein, includes optionally substituted alkyl.

The compounds of the present application may possess one or more stereoisomeric centers, each of which may exist in R or S configuration or a combination thereof. Likewise, the compounds of the present application may possess one or more double bonds, each of which may exist in *E* (trans) or Z (cis) configuration or a combination thereof. A specific stereoisomer, regioisomer, diastereomer, enantiomer or epimer should be understood to include all possible stereoisomers, regioisomers, diastereomers, enantiomers or epimers and mixtures thereof. Therefore, the compounds of the present application include all stereoisomers, regioisomers, diastereomers, enantiomers or epimers of different configurations as well as the corresponding mixtures thereof. The technologies for transforming a specific stereoisomer or leaving a specific stereoisomer unchanged, and for resolving mixtures of stereoisomers are well known in the art. A person skilled in the art can choose a suitable method for a specific situation. See, for example, Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; and Heller, Acc. Chem. Res. 1990, 23, 128.

It should be understood that the methods and compositions described herein are not limited to the specific methods, protocols, cell lines, constructs and reagents described herein, and may vary in this regard. It may also be understood that the terms used herein are for the purpose of describing particular embodiments only, and are not intended to limit the scopes of the methods and compositions described herein, which are only limited by the appended claims.

All publications and patents mentioned in the present application are incorporated herein by reference in their entirety, for the purpose of description and disclosure. For example, the constructs and methods described in the publications may be used in combination with the methods, compositions and compounds described in the present application. The publications discussed herein are provided solely for their disclosure prior to the filing date of the present application. Nothing herein is to be construed as an admission that the inventors described herein are not entitled to antedate such disclosure by virtue of prior invention or for any other reason.

The terms "moiety", "structural moiety", "chemical moiety", "group" and "chemical group" used herein refer to a specific segment or functional group of a molecule. Chemical moieties are usually considered as chemical entities embedded in or attached to a molecule.

The term "bond" or "single bond" refers to a chemical bond that connects two atoms or two moieties through a bond to obtain a larger structural moiety.

The term "optional" or "optionally" means that the subsequently described event or circumstance may or may not occur, and that the description includes instances where said event or circumstance occurs and instances in which it does not. For example, "optionally substituted alkyl" means either "unsubstituted alkyl" (alkyl which is not substituted by a substituent) or "substituted alkyl" (alkyl which is substituted by a substituent), as defined below. Further, an optionally substituted group may be unsubstituted (e.g., -CH₂CH₃), fully substituted (e.g., - CF₂CF₃), monosubstituted (e.g., -CH₂CH₂F) or substituted at a level anywhere between fully substituted and monosubstituted (e.g., -CH₂CHF₂, -CF₂CH₃, -CFHCHF₂, etc). It will be understood by those skilled in the art with respect to any groups containing one or more substituents that such groups are not intended to introduce any substitutions or substitution patterns that are sterically impractical and/or synthetically non-feasible (e.g., substituted alkyl includes optionally substituted cycloalkyl, which in turn is defined as including optionally substituted alkyl, and so forth). Therefore, the substituents described should generally be understood as having a maximum molecular weight of about 1,000 daltons, and more typically, up to about 500 daltons (except for those instances where macromolecular substituents are clearly intended, e.g., polypeptides, polysaccharides, polyethylene glycols, DNA, RNA and the like).

The C₁₋ₓ used herein includes C₁₋₂, C₁₋₃,... and C_{1-x.}

The term "alkyl" has the common meaning in the art.

The term "heteroalkyl" refers to optionally substituted alkyl, in which one or more of the skeletal chain carbon atoms (and any connected hydrogen atoms, as appropriate) are each independently replaced with a heteroatom (i.e. an atom other than carbon, for example but not limited to, oxygen, nitrogen, sulfur, silicon, phosphorous, tin or combinations thereof).

The term "lower heteroalkyl" refers to a heteroalkyl having one to eight carbon atoms, preferably having one to six, or one to five, or one to four, or one to three, or one to two carbon atoms.

The alkyl of the compound described in the present application may be specified as "C₁₋₄ alkyl" or the like. By way of example only, "C₁₋₄ alkyl" indicates that there are one to four carbon atoms in the alky chain, i.e., the alkyl chain is selected from methyl, ethyl, propyl, isopropyl, n-butyl, isobutyl, sec-butyl and t-butyl. Thus, C₁₋₄ alkyl includes C₁₋₂ alkyl and C₁₋₃ alkyl. Alkyl may be substituted or unsubstituted. Examples include, but are not limited to, methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, sec-butyl, t-butyl, n-pentyl, isopentyl, neopentyl, tert-amyl and hexyl, and longer alkyl groups, such as heptyl, octyl and the like. Where a numerical range appears in a group defined herein (such as "alkyl"), such as "C₁-C₆ alkyl" or "C₁₋₆ alkyl", means that the alkyl group may consist of 1 carbon atom, 2 carbon atoms, 3 carbon atoms, 4 carbon atoms, 5 carbon atoms or 6 carbon atoms, and the present definition also covers the occurrence of the term "alkyl" wherein no numerical range is designated. The alkyl may also be a "lower alkyl" having 1 to 6 carbon atoms, i.e., C₁₋₆ alkyl.

The term "alkenyl" refers to a type of alkyl in which the first two atoms of the alky form a double bond that is not part of an aromatic group. That is, an alkenyl group begins with the atoms -C(R)=C(R)-R, wherein R refers to the remaining portions of the alkenyl group, and each R may be the same or different. Alkenyl may have 2 to 10 carbon atoms. The alkenyl may also be a "lower alkenyl" having 2 to 6 carbon atoms.

The term "alkynyl" refers to a type of alkyl in which the first two atoms of the alkyl form a triple bond. That is, an alkynyl group begins with the atoms -C=C-R, wherein R refers to the remaining portions of the alkynyl group, and each R may be the same or different. The "R" portion of the alkynyl moiety may be branched, straight chain or cyclic. Alkynyl may have 2 to 10 carbon atoms. The alkynyl may also be a "lower alkynyl" having 2 to 6 carbon atoms.

The term "alkoxy" refers to the group (alkyl)-O-, wherein the alkyl is as defined herein.

The term "amide" is a chemical moiety with the formula -C(O)NHR or -NHC(O)R, wherein R is selected from alkyl, cycloalkyl, aryl, heteroaryl and heteroalicyclic. An amide moiety may form a linkage between an amino acid or a peptide molecule and a compound described herein, thereby forming a prodrug.

The term "ester" refers to a chemical moiety with the formula -COOR, wherein R is selected from alkyl, cycloalkyl, aryl, heteroaryl and heteroalicyclic. Any hydroxyl or carboxyl side chain on the compounds described herein can be esterified.

The term "ring" refers to any covalently closed structure. The ring includes, for example, carbocycles, heterocycles, aromatics and non-aromatics. The ring may be optionally substituted. The ring may be monocyclic or polycyclic.

The term "membered ring" can comprise any cyclic structures. The term "membered" denotes the number of skeletal atoms that constitute the ring. Thus, for example, pyridine and thiopyran are 6-membered rings and cyclophentyl and pyrrole are 5-membered rings.

The terms "carbocyclic" and "carbocycle" refer to a ring wherein each of the atoms forming the ring is a carbon atom. Carbocycle includes aryl and cycloalkyl. The term thus distinguishes carbocycle from heterocycle in which the ring backbone contains at least one atom which is different from carbon. Heterocycle includes heteroaryl and heterocycloalkyl. Carbocycles and heterocycles may be optionally substituted.

The term "aromatic" refers to a planar ring having a delocalized π-electron system containing 4n+ π electrons, wherein n is an integer. Aromatic rings can be formed from five, six, seven, eight, nine, or more than nine atoms. Aromatics may be optionally substituted. The term "aromatic" includes carbocyclic aryl and heterocyclic aryl groups. The term includes monocyclic or fused-ring polycyclic groups.

The term "aryl" refers to an aromatic ring wherein each of the atoms forming the ring is a carbon atom. Aryl ring can be formed by five, six, seven, eight, nine, or more than nine carbon atoms. Aryl groups may be optionally substituted. Examples of aryl include, but are not limited to, phenyl, naphthyl, phenanthryl, anthranyl, fluorenyl and indenyl. Depending on the structure, an aryl group may be a monovalent group or a divalent group, i.e., arylene.

The term "cycloalkyl" refers to a monocyclic or polycyclic group that contains only carbon and hydrogen, and may be saturated, partially unsaturated or fully unsaturated. Cycloalkyl includes groups having from 3 to 10 ring atoms. Illustrative examples of cycloalkyl include the following moieties: 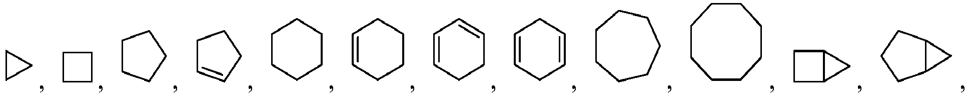 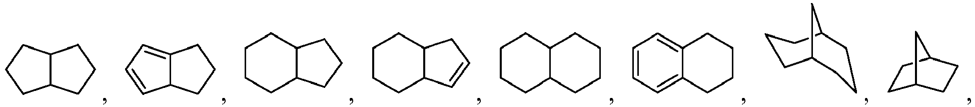 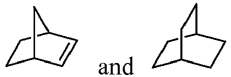 and so on. Depending on the structure, a cycloalkyl group can be a monovalent group or a divalent group. The cycloalkyl group may also be a "lower cycloalkyl" having 3 to 8 carbon atoms.

The terms "heterocycle" refers to heteroaryl and heteroalicyclic groups containing one to four heteroatoms each selected from O, S and N, wherein each heterocyclic group has from 4 to 10 atoms in its ring system, with the proviso that the ring of said group does not contain two adjacent O or S atoms. Herein, whenever the number of carbon atoms in a heterocycle is indicated, at least one other heteroatom must be present in the ring. Expressions such as "C₁₋₆ heterocycle" refer only to the number of carbon atoms in the ring and do not refer to the total number of atoms in the ring. It is understood that the heterocyclic ring can have additional heteroatoms in the ring. Expressions such as "4-6 membered heterocycle" refer to the total number of atoms that are contained in the ring. In heterocycles that have two or more heteroatoms, those two or more heteroatoms may be the same or different from one another. Heterocycles may be optionally substituted. Binding to a heterocycle may be at a heteroatom or via a carbon atom. Non-aromatic heterocyclic groups include groups having only 4 atoms in their ring system, but aromatic heterocyclic groups must have at least 5 atoms in their sing system. The heterocyclic groups include benzo-fused ring systems. An example of a 4-membered heterocyclic group is azetidinyl (derived from azetidine). A non-limiting example of a 5-membered heterocyclic group is thiazolyl. A non-limiting example of a 6-membered heterocyclic group is pyridyl. A non-limiting example of a 10-membered heterocyclic group is quinolinyl. Non-limiting examples of non-aromatic heterocyclic groups are pyrrolidinyl, tetrahydrofuranyl, dihydropyranyl, thioxanyl, piperazinyl, and quinolizinyl. Non-limiting examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, benzofurazanyl, benzothiophenyl, and furopyridinyl.

The term "heteroaryl" refers to optionally substituted monovalent aromatic group containing from about five to about twenty skeletal ring atoms, wherein one or more of the ring atoms is a heteroatom independently selected from, but not limited to, oxygen, nitrogen, sulfur, phosphorous, silicon, selenium and tin; with the proviso that the ring of said group does not contain two adjacent O or S atoms. In embodiments in which two or more heteroatoms are present in the ring, the two or more heteroatoms may be the same as each another, or some or all of the two or more heteroatoms may each be different from the others. The term heteroaryl includes optionally substituted monovalent fused or non-fused heteroaryl having at least one heteroatom. In addition, the term heteroaryl also includes fused or non-fused heteroaryl having from 5 to about 12 skeletal ring atoms, as well as those having from 5 to about 10 skeletal ring atoms. Bonding to a heteroaryl group may be via a carbon atom or a heteroatom. Thus, as an example, imidazole may be attached to a parent molecule via any of its carbon atoms (imidazol-2-yl, imidazol-4-yl or imidazol-5-yl) or its nitrogen atoms (imidazol-1-yl or imidazol-3-yl). Likewise, a heteroaryl group may be further substituted via any or all of its carbon atoms, and/or any or all of its heteroatoms. A fused heteroaryl may contain a fused ring in which 2 to 4 heteroaromatic rings are fused and the other individual rings may be alicyclic ring, heterocycle, aromatic ring, heteroaromatic ring or any combinations thereof. A non-limiting example of single-ring heteroaryl groups includes pyridyl; non-limiting examples of fused-ring heteroaryl groups include benzimidazolyl, quinolinyl, and acridinyl; and a non-limiting example of non-fused bi-heteroaryl groups includes bipyridinyl. Further examples of heteroaryls include, but are not limited to, furanyl, thienyl, oxazolyl, acridinyl, phenazinyl, isoxazolyl, isothiazolyl, pyrazolyl, purinyl, quinolinyl, triazolyl, thiazolyl, triazinyl, thiadiazolyl and the like, and their oxides, such as pyridyl-N-oxide.

The term "non-aromatic heterocycle", "heterocycloalkyl" or "heteroalicyclic" refers to a non-aromatic ring wherein one or more atoms forming the ring are a heteroatom. A "non-aromatic heterocycle" or "heterocycloalkyl" refers to a cycloalkyl group that includes at least one heteroatom selected from nitrogen, oxygen and sulfur. The groups may be fused with an aryl or heteroaryl. Heterocycloalkyl can be formed by three, four, five, six, seven, eight, nine, or more than nine atoms. Heterocycloalkyl rings may be optionally substituted. In certain embodiments, non-aromatic heterocycles contain one or more carbonyl or thiocarbonyl groups such as oxo- and thio-containing groups. Examples of heterocycloalkyl include, but are not limited to, lactams, lactones, tetrahydrothiopyran, 4H-pyran, tetrahydropyran, piperidine, piperazine, tetrahydrothiophene, tetrahydrofuran, pyrroline, pyrrolidine, pyrrolidone, pyrrolidione, thiazoline and thiazolidine. Illustrative examples of heterocycloalkyl, also referred to as non-aromatic heterocycles, include: 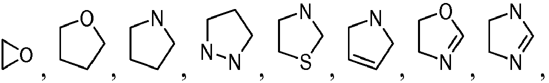 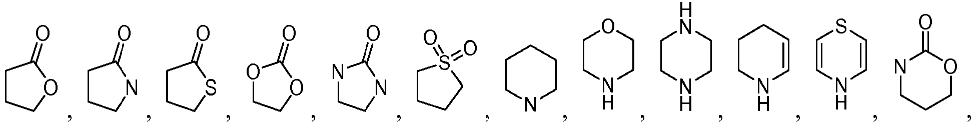 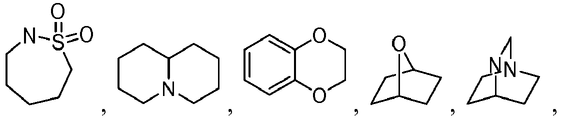 and the like.

The term "halogen", "halo" or "halide" refers to fluoro, chloro, bromo and iodo.

The term "sulfonyl" refers to a divalent group of -S(=O)₂-R.

The terms "sulfonamide", "sulfonamido" and "sulfonamidyl" refer to the groups of - S(O)₂NH- and -NHS(=O)₂-.

The term "cyano" refers to the group -CN.

### Certain Pharmaceutical Terminology

### Bromodomain

A bromodomain is a protein domain that recognizes acetylated lysine residues such as those on the N-terminal tails or other sites of histones. This recognition process is often a prerequisite for protein-histone association and chromatin remodeling. The domain itself adopts an all-α protein fold form, and is formed by a bundle of four α-helices.

Specifically, the bromodomain is a highly evolutionary-conserved protein functional domain having approximately 110 amino acids, which can specifically recognize acetylated lysine sites at the terminals of histones, and is involved in signal-dependent and non-basic gene transcription regulation through chromatin assembly and acetylation; the bromodomain can also be extensively involved in the processes, such as cell cycle regulation, cell differentiation and signal transduction, through acetylation modification of non-histone proteins such as transcription factors; the change of bromodomain proteins is involved in the occurrence of leukemia and other malignancies through the deregulation of histone acetylation; and the research on its relationship with tumor will provide a new strategy for oncotherapy.

The terms "preventing", "prevention" and "prevent" include reducing the likelihood of a patient incurring or developing autoimmune disease, heteroimmune disease, inflammatory disease, thromboembolic disorder or cancer (such as, diffuse large β-cell lymphoma, chronic lymphocytic lymphoma, β-cell prolymphocytic leukemia and the like).

The term "subject", "patient" or "individual" refers to individuals suffering from a disease, a disorder, a condition, and the like, and comprises mammals and non-mammals. Examples of mammals include, but are not limited to, any member of the mammalian class: humans, non-human primates such as chimpanzees, and other apes and monkeys; farm animals such as cattle, horses, sheep, goats, and swine; domestic animals such as rabbits, dogs and cats; laboratory animals including rodents, such as rats, mice and guinea pigs, and the like. Examples of non-human mammals include, but are not limited to, birds, fish, and the like. In one embodiment of the methods and compositions provided herein, the mammal is a human.

The terms "treat", "treating" and "treatment" and other similar synonyms include alleviating, abating or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving symptoms caused by the disease or condition, or stopping the symptoms of the disease or condition, and the terms are intended to include prophylaxis. The terms further include achieving a therapeutic effect and/or a prophylactic effect. The therapeutic effect refers to eradication or amelioration of the underlying disorder being treated. In addition, a therapeutic effect also includes the eradication or amelioration of one or more of the physiological symptoms associated with the underlying disorder, for example, an improvement is observed in the patient, notwithstanding that the patient may still be affected with the underlying disorder. For prophylactic effect, the composition may be administered to a patient at risk of developing a particular disease, or to a patient having one or more of the physiological symptoms of a disease, even though a diagnosis of this disease may not have been made.

The terms "effective amount", "therapeutically effective amount" and "pharmaceutically effective amount" refer to a sufficient amount of at least one agent or compound being administered which will relieve to some extent one or more of the symptoms of the disease or condition being treated. The result can be reduction and/or alleviation of the signs, symptoms or causes of a disease, or any other desired alteration of a biological system. For example, an "effective amount" for therapeutic uses is the amount of the composition comprising a compound as disclosed herein required to provide a clinically significant alleviation of a disease. An appropriate "effective" amount in any individual case may be determined by using techniques, such as a dose escalation study.

The terms "administer", "administering", "administration", and the like, refer to the methods that may be used to enable delivery of a compound or a composition to the desired site of biological action. These methods include, but are not limited to, oral route, intraduodenal route, parenteral injection (including intravenous, subcutaneous, intraperitoneal, intramuscular, and intra-arterial rejection or infusion), topical and rectal administrations. Those of skill in the art are familiar with administration techniques that can be employed for the compounds and methods described herein, e.g., as discussed in Goodman and Gilman, The Pharmacological Basis of Therapeutics, current ed.; Pergamon; and Remington's Pharmaceutical Sciences (current edition), Mack Publishing Co., Easton, Pa. In preferred embodiments, the compounds and compositions described herein are administered orally.

The term "acceptable" means having no persistent detrimental effect on the general health of the subject being treated.

The term "pharmaceutically acceptable" refers to a material, such as a carrier or diluent, which does not abrogate the biological activity or properties of the compounds described herein, and is relatively nontoxic, i.e., the material may be administered to an individual without causing undesirable biological effects or interacting in a deleterious manner with any of the components of the composition in which it is contained.

The term "composition" or "pharmaceutical composition" refers to a biologically active compound, optionally mixed with at least one pharmaceutically acceptable chemical component, including but not limited to, carriers, stabilizers, diluents, dispersing agents, suspending agents, thickening agents and/or excipients.

The term "carrier" refers to relatively nontoxic chemical compounds or agents that facilitate the incorporation of a compound into cells or tissues.

The term "modulate" means to interact with a target directly or indirectly so as to alter the activity of the target, including, by way of example only, to enhance the activity of the target, to inhibit the activity of the target, to limit the activity of the target, or to extend the activity of the target.

The term "pharmaceutically acceptable salt" refers to salts that retain the biological effectiveness of the free acids and bases of the specified compound and that are not biologically or otherwise undesirable. Compounds described herein may possess acidic or basic groups and therefore may react with any of a number of inorganic or organic bases, and inorganic and organic acids, to form a pharmaceutically acceptable salt. These salts can be prepared in situ during the final isolation and purification of the compounds of the present application, or by separately reacting a compound of the present application in its free base form with a suitable organic or inorganic acid, and isolating the salt thus formed. Examples of pharmaceutically acceptable salts include those salts prepared by the reactions of the compounds described herein with an inorganic or organic acid or an inorganic or organic base. These salts include acetate, acrylate, adipate, alginate, aspartate, benzoate, benzenesulfonate, bisulfate, bisulfite, bromide, butyrate, butyn-1,4-dioate, camphorate, camphorsulfonate, caprylate, chlorobenzoate, chloride, citrate, cyclopentanepropionate, decanoate, gluconate, dihydrogenphosphate, dinitrobenzoate, dodecylsulfate, ethanesulfonate, formate, fumarate, glucoheptanoate, glycerophosphate, glycolate, hemisulfate, heptanoate, hexyne-1,6-dioate, hydroxybenzoate, y-hydroxybutyrate, hydrochloride, hydrobromide, hydroiodide, 2-hydroxyethanesulfonate, iodide, isobutyrate, lactate, maleate, malonate, methanesulfonate, mandelate, metaphosphate, methoxybenzoate, methylbenzoate, monohydrogenphosphate, 1-napthalenesulfonate, 2-napthalenesulfonate, nicotinate, nitrate, palmoate, pectinate, persulfate, 3-phenylpropionate, phosphate, picrate, pivalate, propionate, pyrosulfate, pyrophosphate, propiolate, phthalate, phenylacetate, phenylbutyrate, propanesulfonate, salicylate, succinate, sulfate, sulfite, suberate, sebacate, sulfonate, tartrate, thiocyanate, tosylate, undeconate and xylenesulfonate. Other acids, such as oxalic, while not pharmaceutically acceptable in themselves, may be employed in the preparation of salts useful as intermediates in obtaining the compounds of the present application and their pharmaceutically acceptable acid addition salts (See the examples in Berge et al., J. Pharm. Sci. 1977, 66, 1-19). Furthermore, those compounds described herein which may comprise a free acid group may react with a suitable base, such as the hydroxide, carbonate or bicarbonate of a pharmaceutically acceptable metal cation, with ammonia, or with a pharmaceutically acceptable organic primary, secondary or tertiary amine. Representative alkali-metal or alkaline-earth-metal salts include lithium, sodium, potassium, calcium, magnesium and aluminum salts and the like. Illustrative examples of bases include sodium hydroxide, potassium hydroxide, choline hydroxide, sodium carbonate, IV' (C₁₋₄ alkyl)₄, and the like. Representative organic amines useful for the formation of base addition salts include ethylamine, diethylamine, ethylenediamine, ethanolamine, diethanolamine, piperazine and the like. It should be understood that the compounds described herein also include the quaternization of any basic nitrogen-containing groups they may contain. Water or oil-soluble or dispersible products may be obtained by such quaternization. See, for example, the above reference of Berge et al.

The term "solvate" refers to a combination of a compound of the present application with a solvent molecule formed by solvation. In some embodiments, the solvate refers to a hydrate, e.g., the solvent molecule is a water molecule, and the combination of a compound of the present application and water forms monohydrate.

The term "polymorph" or "polymorphism" refers to a compound of the present application present in different crystal lattice forms.

The term "ester" refers to a derivative of a compound of the present application derived from compounds containing carboxyl or hydroxyl, any one of which can be the compound of the present application.

The term "tautomer" refers to an isomer that can be readily interconverted from a compound of the present application to other compounds by the migration of a hydrogen atom or proton.

The term "pharmaceutically acceptable derivative" or "prodrug" refers to any pharmaceutically acceptable salt, ester, or salt of an ester of a compound of the present application, or a pharmaceutically active metabolite or residue thereof which is capable of providing therapeutic effect for a patient directly or indirectly. Particularly favored pharmaceutically acceptable derivatives or prodrugs are those that increase the pharmaceutical activity of the compounds of the present application when such compounds are administered to a patient (e.g., by allowing the compound to be orally administered so as to be more readily absorbed into blood) or which improve the metabolic absorption of the compound *in vivo* (e.g., the brain or lymphatic system).

The terms "enhance" and "improve" and other similar wordings mean to increase or prolong either in potency or duration of a desired effect. Thus, in regard to enhancing the effect of a therapeutic agent, the term "enhancing" refers to the ability to increase or prolong, either in potency or duration, the effect of another therapeutic agent on a system.

The terms "pharmaceutical combination", "administering an additional therapy", "administering an additional therapeutic agent", and the like refer to a pharmaceutical therapy resulting from mixing or combining more than one active ingredient and includes fixed and non-fixed combinations of the active ingredients. The term "fixed combination" means that at least one of the compounds described herein and at least one co-agent are both administered to a patient simultaneously in the form of a single entity or dosage. The term "non-fixed combination" means that at least one of the compounds described herein and at least one co-agent are administered to a patient as separate entities simultaneously, concurrently or sequentially with variable intervening time limits, wherein such administration provides effective levels of the two or more compounds in the body of the patient. These are also applied to cocktail therapies, e.g. the administration of three or more active ingredients.

The term "metabolite" refers to a derivative of a compound which is formed when the compound is metabolized.

The term "active metabolite" refers to an active derivative of a compound that is formed when the compound is metabolized.

The term "IC₅₀" refers to an amount, concentration or dosage of a particular test compound that achieves a 50% inhibition of a maximal response, such as inhibition of BET proteins, in an assay that measures such response.

The disease associated with BET proteins herein includes a plurality of tumors/cancers, for example, but is not limited to, bone cancer, liver cancer, lung cancer, oral epithelial carcinoma, nasopharyngeal cancer, thyroid cancer, esophageal cancer, lymphoma, chest cancer, digestive tract cancer, pancreatic cancer, intestinal cancer, breast cancer, ovarian cancer, metrocarcinoma, renal cancer, gallbladder cancer, cholangiocarcinoma, central nervous system cancer, testicular cancer, bladder cancer, prostate cancer, skin cancer, melanoma, sarcoma, brain cancer, leukemia, cervical cancer, glioma, gastric cancer and ascites tumor; and preferably acute myelogenous leukemia (AML), acute lymphoblastic leukemia (ALL), small cell lung cancer, non-small cell lung cancer, colorectal cancer, multiple myeloma, breast cancer, glioma, prostate cancer and lymphoma.

### SPECIFIC EMBODIMENTS

The compounds of the present application, as well as their preparation methods and uses, are illustratively described below in connection with the examples.

### Synthetic Methods

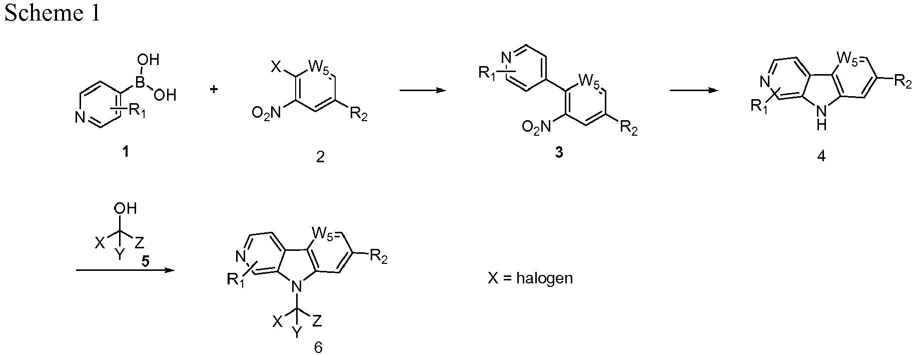

X is halogen. 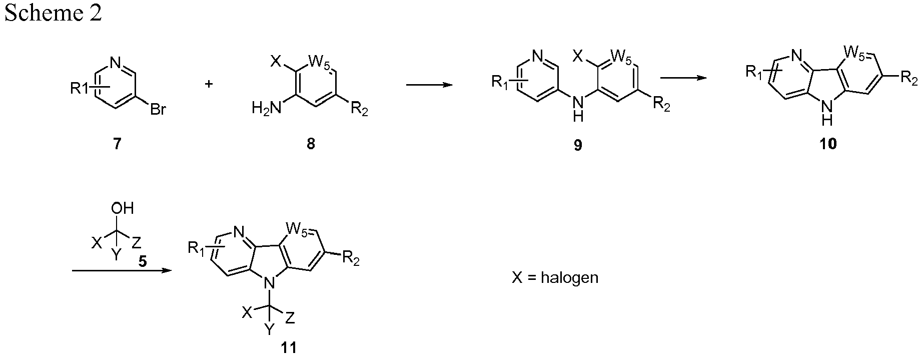 X is halogen.

The compounds of the present application can be prepared according to the route shown in Scheme 1 or Scheme 2. Each product obtained in the reactions of Scheme 1 or Scheme 2 may be obtained through conventional separation techniques, including but not limited to filtration, distillation, crystallization, chromatographic separation, and the like. Starting materials may be synthesized or purchased from commercial institutions (for example but not limited to Adrich or Sigma). These starting materials may be characterized by conventional methods, such as physical constants and spectroscopic data. A single isomer or a mixture of isomers of the compound of the present application may be obtained using a synthetic method.

In Scheme 1, an intermediate 3 is obtained through Suzuki reaction between a starting material 1 and a starting material 2 in the presence of an appropriate palladium catalyst (e.g., PdCl₂ (dppf)). An azacarbazole intermediate 4 is obtained through the reductive cyclization (Cadogan reaction) of the intermediate 3 in the presence of 1,2-bis(diphenylphosphino)ethane. A target compound 6 is obtained through Mitsunobu reaction between the intermediate 4 and a starting material 5.

In Scheme 2, an intermediate 9 is obtained through Buchwald N-arylation reaction between a starting material 7 and a starting material 8. An azacarbazole intermediate 10 is obtained through Pd-catalyzed cyclization of the intermediate 9 under various conditions. A target compound 11 is obtained through Mitsunobu reaction between the intermediate 10 and a starting material 5.

### Examples

The following non-limiting examples are illustrative only, and are not intended to limit the present invention in any way.

Unless otherwise specified, the temperatures are in degrees Celsius. Reagents are purchased from commercial suppliers, such as Sinopharm Chemical Reagent Beijing Co., Ltd, Alfa Aesar, or Beijing J&K Scientific Ltd., and these reagents can be used directly without further purification, unless otherwise specified.

Unless otherwise specified, the following reactions are carried out in anhydrous solvents under a positive pressure of nitrogen or argon, or in drying tubes; the reaction bottle is equipped with a rubber diaphragm so as to add substrates and reagents through syringes; and the glassware is oven dried and/or dried by heating.

Unless otherwise specified, column chromatography purification is performed using 200-300 mesh silica gel from Qingdao Haiyang Chemical Co., Ltd; preparative thin layer chromatography separation is performed using thin layer chromatography silica gel prefabricated plate (HSGF254) produced by Yantai Chemical Industry Research Institute; MS is determined by Thermo LCQ Fleet-type (ESI) liquid chromatography-mass spectrometry; optical rotation is determined using SGW-3 automatic polarimeter from Shanghai Shenguang Instrument Co., Ltd.

Nuclear magnetic resonance data (¹H NMR) are obtained using a Varian device running at 400 MHz. The nuclear magnetic resonance data are obtained using a solvent of CDCl₃, CD₃OD, D₂O, DMSO-d6 or the like, based on tetramethylsilane (0.00 ppm) or residual solvents (CDCl₃: 7.26ppm; CD₃OD: 3.31ppm; D₂O: 4.79ppm; d6-DMSO: 2.50ppm). When peak shape diversity is indicated, the following abbreviations represent different peak shapes: s (singlet peak), d (doublet peak), t (triplet peak), q (quartet peak), m (multiplet peak), br (broad peak), dd (double doublet peak), and dt (double triplet peak). If a coupling constant is given, then Hertz (Hz) is the unit.

### Abbreviations:

- AcOH: Acetic acid
- N₂: Nitrogen
- O₂: Oxygen
- Pd₂(dba)₃: Tris(dibenzylideneacetone)dipalladium
- xant-Phos: 4,5-bis(diphenylphosphino)-9,9-dimethylxanthene
- MeOH: Methanol
- DCM: Dichloromethane
- PdCl₂(dppf): [1,1'-Bis(diphenylphosphino)ferrocene]palladium dichloride
- DIAD: Diisopropyl azodiformate
- DIEA: *N,N-*diisopropylethylamine
- DMAP: 4-*N,N*-dimethylaminopyridine
- DMF: *N,N-*dimethylformamide
- DMSO: Dimethyl sulfoxide
- EtOAc: Ethyl acetate
- EtOH: Ethanol
- Et₂O: Diethyl ether
- Et₃N: Triethylamine
- HATU: *O*-(7-azabenzotriazol-1-yl)-*N,N,N',N'*-tetramethyluronium tetrafluorophosphate
- HPLC: High performance liquid chromatography
- n-BuLi: n-Butyllithium
- *i*-PrOH: Isopropanol
- MS: Mass spectrometry
- PE: Petroleum ether
- *p*-*TSA*: p-Toluenesulfonic acid
- TBAF: Tetrabutylammonium fluoride
- K₃PO4: Anhydrous potassium phosphate
- TFA: Trifluoroacetic acid
- THF: Tetrahydrofuran
- TLC: Thin layer chromatography
- PTLC: Preparative thin layer chromatography

### Example 1

### 4-{8-Methoxy-5-[phenyl(tetrahydro-2H-pyran-4-yl)methyl]-5H-pyrrolo[3,2-b:4,5-b']dipyridm -3-yl}-3,5-dimethylisoxazole

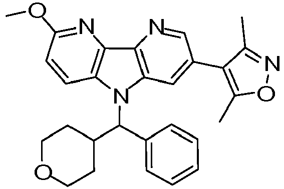

### Step A: 2-Chloro-5-(3,5-dimethylisoxazol-4-yl)pyridin-3-amine

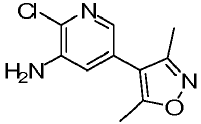

Under the protection of N₂, PdCl₂(dppf) (3.50 g, 0.048 mol) was added to a tetrahydrofuran/water (4:1=200 mL) solution of 5-bromo-2-chloropyridin-3-amine (10.00g, 0.048 mol), (3,5-dimethylisoxazol-4-yl)boric acid (6.80 g, 0.048 mol) and anhydrous potassium phosphate (11.20 g, 0.053 mol), and the resulting mixture was heated to 80°C to react for 4h. After cooling and concentration under reduced pressure, water and ethyl acetate were added to the residue, stirred for 15 min, filtered through Celite filler, and the Celite filler was rinsed with ethyl acetate. After the filtrate was layered, the aqueous phase was extracted with ethyl acetate once. The ethyl acetate phases were combined, washed with a saturated sodium chloride solution, dried with anhydrous sodium sulfate, and concentrated to obtain a brown residue. The residue was purified by silica gel column chromatography eluting with ethyl acetate/petroleum ether (6:1) to obtain a yellow solid product (5.60 g, 52%).
¹H NMR (400 MHz, CDCl₃) δ 7.70 (d, 1H), 6.91 (d, 1H), 4.20 (br, 2H), 2.41 (s, 3H), 2.25 (s, 3H).

### Step B: 2-Chloro-5-(3,5-dimethylisoxazol-4-yl)-N-(6-methoxypyridin-3-yl)pyridin-3-amine

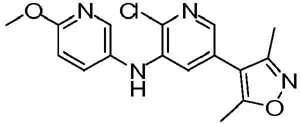

Under the protection of O₂, 4Å powder molecular sieve (3.5 g) was added to a chloroform (50 mL) solution of 2-chloro-5-(3,5-dimethylisoxazol-4-yl)pyridin-3-amine (1.0 g, 0.0045 mol), (6-methoxypyridin-3-yl)boric acid (1.38g, 0.0090 mol), anhydrous copper acetate (1.22g, 0.0068 mol) and pyridine (0.709g, 0.0090 mol), and the resulting mixture reacted at 25°C overnight. After concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a brownish black residue. The residue was purified by silica gel column chromatography eluting with ethyl acetate/petroleum ether (4:1) to obtain a brown solid product (0.69 g, 47%).
¹H NMR (400 MHz, CDCl₃) δ 8.12 (d, *J*=4.0 Hz, 1H), 7.74 (d, *J*=4.0 Hz, 1H), 7.48 (dd, 1H), 6.90 (d, 1H), 6.82 (dd, 1H), 6.01 (s, 1H), 3.96 (s, 3H), 2.36 (s, 3H), 2.20 (s, 3H).

### Step C: C-1: 4-(8-Methoxy-5H-pyrrolo[3,2-b:4,5-b']dipyridin-3-yl)-3,5-dimethylisoxazole

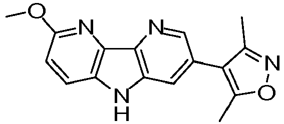

### C-2: 4-(8-Methoxy-5H-pyrrolo[3,2-b:5,4-c']dipyridin-3-yl)-3,5-dimethylisoxazole

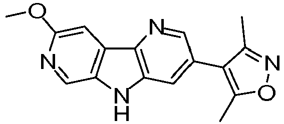

Under the protection of N₂, PdCl₂(dppf) (0.25 g, 0.34 mmol) was added to an *N,N-*dimethylacetamide (5 mL) solution of 2-chloro-5-(3,5-dimethylisoxazol-4-yl)-*N*- (6-methoxypyridin-3-yl)pyridin-3-amine (1.00 g, 3.02 mmol) and sodium acetate trihydrate (1.00 g, 9.06 mmol). The system was heated to 180°C and reacted under stirring for 1h. After cooling to room temperature, water and ethyl acetate were added, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was purified by silica gel column chromatography eluting with dichloromethane/methanol (50:1) to obtain brown solid products C-1 (0.240 g, 27%) and C-2 (0.109 g, 12%).
C-1: ¹H NMR (400 MHz, CDCl₃) δ 9.43 (s, 1H), 8.56 (d, 1H), 7.80 (d, 1H), 7.67 (d, 1H), 6.95 (d, 1H), 4.08 (s, 3H), 2.43 (s, 3H), 2.29 (s, 3H).
C-2: ¹H NMR (400 MHz, CDCl₃) δ 9.24 (s, 1H), 8.51 (s, 1H), 8.47 (d, 1H), 7.63 (s, 1H), 7.61 (d, 1H), 4.03 (s, 3H), 2.46 (s, 3H), 2.31 (s, 3H).

### Step D: Phenyl(tetrahydro-2H-pyran-4-yl)methanone

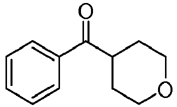

Sulfoxide chloride (50 mL) and DMF (0.1 mL) were successively added dropwise to tetrahydropyran-4-carboxylic acid (10.00 g, 0.077 mol) at 0°C. After the addition was completed, the resulting mixture was slowly heated to 70°C to react for 5h. After the reaction was completed, a corresponding acyl chloride (11g) was obtained by concentration. Benzene (100 mL) was added to the acyl chloride at 0°C, and then anhydrous AlCl₃ (30 g, 0.22 mol) was added in batches. After the addition was completed, the system was stirred for additional 15 min, and heated to reflux for 5h. After the reaction was completed, the resulting mixture was cooled, ice water and a saturated ammonium chloride aqueous solution were successively added, and the resulting mixture was extracted with ethyl acetate. The ethyl acetate phases were combined, washed with a saturated sodium chloride aqueous solution, dried with anhydrous sodium sulfate, and concentrated to obtain a black residue. The residue was purified by silica gel column chromatography (petroleum ether:ethyl acetate=6:1) to obtain a white solid product (8.5 g, 60%).
¹H NMR (400 MHz, CDCl₃) δ 7.96-7.99 (m, 2H), 7.59-7.62 (m, 1H), 7.49-7.53 (m, 2H), 4.06-4.11 (m, 2H), 3.55-3.62 (m, 2H), 3.49-3.54 (m, 1H), 1.86-1.97 (m, 2H), 1.80-1.85 (m, 2H).

### Step E: Phenyl(tetrahydro-2H-pyran-4-yl)methanol

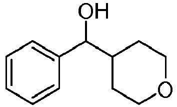

Sodium borohydride (2.00 g, 0.053 mol) was added in batches to a methanol (50 mL) and water (10 mL) solution containing phenyl(tetrahydro-2*H*-pyran-4-yl)methanone (8.5 g, 0.045 mol) at 0°C. After the addition was completed, the resulting mixture was slowly heated to 50°C to react for 1h. After the reaction was completed, the resulting mixture was cooled, concentrated under reduced pressure, and extracted with ethyl acetate. The ethyl acetate phases were combined, washed with a saturated sodium chloride aqueous solution, dried with anhydrous sodium sulfate, and concentrated. The residue was purified by silica gel column chromatography (petroleum ether:ethyl acetate=4:1) to obtain the target product (8.00 g, 93%).
¹H NMR (400 MHz, CDCl₃) δ 7.28-7.36 (m, 5H), 4.32-4.35 (m, 1H), 3.97-4.01 (m, 1H), 3.85-3.89 (m, 1H), 3.23-3.38 (m, 2H), 2.16 (d, 1H), 1.87-1.92 (m, 2H), 1.39-1.49 (m, 1H), 1.24-1.35 (m, 1H), 1.10-1.17 (m, 1H).

### Step F: 4- {8-Methoxy-5-[phenyl(tetrahydro-2H-pyran-4-yl)methyl]-5H-pyrrolo[3,2-b:4,5-b'] dipyridm-3-yl}-3,5-dimethylisoxazole

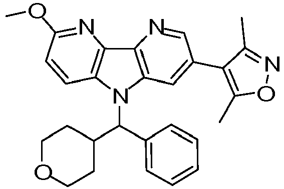

Diisopropyl azodiformate (57 mg, 0.28 mmol) was added dropwise to an anhydrous tetrahydrofuran (2 mL) solution containing 4-(8-methoxy-5H-pyrrolo[3,2-b:4,5-b']dipyridin-3-yl) -3,5-dimethylisoxazole (40 mg, 0.14 mmol), phenyl(tetrahydro-2H-pyran-4-yl) methanol (52 mg, 0.28 mmol) and triphenylphosphine (73 mg, 0.28 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol=50:1 as the developing solvent, to obtain the target product (8 mg, 13%).
¹H NMR (400 MHz, CDCl₃) δ 8.53 (d, 1H), 7.91 (d, 1H), 7.63 (d, 1H), 7.26-7.43 (m, 5H), 6.98 (d, 1H), 5.40 (d, 1H), 4.13 (s, 3H), 4.00-4.08 (m, 1H), 3.82-3.90 (m, 1H), 3.49-3.55 (m, 1H), 3.32-3.38 (m, 1H), 3.03-3.06 (m, 1H), 2.41 (s, 3H), 2.25 (s, 3H), 1.94-2.00 (m, 1H), 1.53-1.58 (m, 1H), 1.24-1.38 (m, 1H), 1.10-1.13 (m, 1H).

### Example 2

### 4-{8-Methoxy-5-[phenyl(tetrahydro-2H-pyran-4-yl)methyl]-5H-pyrrolo[3,2-b:4,5-c']dipyridin-3-yl}-3,5-dimethylisoxazole

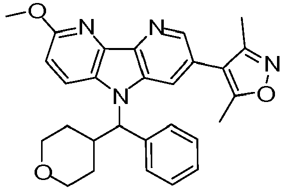

Diisopropyl azodiformate (29 mg, 0.14 mmol) was added dropwise to an anhydrous tetrahydrofuran (1 mL) solution containing 4-(8-methoxy-5H-pyrrolo[3,2-b:5,4-c']dipyridin-3-yl) -3,5-dimethylisoxazole (20 mg, 0.068 mmol), phenyl(tetrahydro-2H-pyran-4-yl)methanol (26 mg, 0.14 mmol) and triphenylphosphine (37 mg, 0.14 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol=40:1 as the developing solvent, to obtain the target product (16 mg, 50%).
¹H NMR (400 MHz, CDCl₃) δ 8.74 (s, 1H), 8.44 (d, 1H), 7.64 (s, 1H), 7.60 (d, 1H), 7.43-7.45 (m, 2H), 7.27-7.36 (m, 3H), 5.38 (d, 1H), 4.00-4.03 (m, 4H), 3.83-3.89 (m, 1H), 3.51-3.58 (m, 1H), 3.36-3.41 (m, 1H), 3.05-3.16 (m, 1H), 2.44 (s, 3H), 2.28 (s, 3H), 1.91-1.96 (m, 1H), 1.48- 1.60 (m, 1H), 1.37-1.43 (m, 1H), 1.10-1.13 (m, 1H).

### Example 3

### Ethyl 3-(3,5-dimethylisoxazol-4-yl)-5-[phenyl(tetrahydro-2H-pyran-4-yl)methyl]-5H-pyrrolo [2,3-b:4,5-c']dipyridine-7-carboxylate

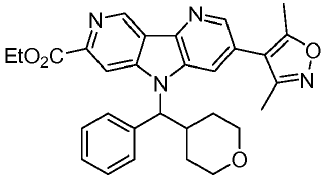

### Step A: Ethyl 5-(4,4,5,5-tetramethyl-1,3,2-dioxaborolan-2-yl)picolinate

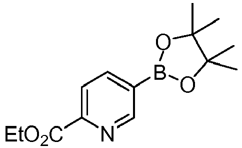

Under the protection of N₂, PdCl₂(dppf) (1.80 g, 0.0025 mol) was added to an anhydrous 1,4-dioxane (200 mL) solution of ethyl 5-bromo-2-picolinate (10.00 g, 0.043 mol), bis(pinacolato)diboron (12.20 g, 0.048 mol) and anhydrous potassium acetate (13.00 g, 0.13 mol), and the resulting mixture was heated to 100 °C to react overnight. After cooling and concentration under reduced pressure, water and ethyl acetate were added to the residue, stirred for 15 min, and filtered through Celite, and the Celite was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue, which was separated through a silica gel column (ethyl acetate/petroleum ether=1:10-1:2) to obtain a white solid product (9.03 g, 75%).
¹H NMR (400 MHz, CDCl₃,) δ 9.06 (d, J=1.6 Hz, 1H), 8.21 (dd, J=7.6 Hz, J=1.6 Hz, 1H), 8.10 (d, J=7.6 Hz, 1H), 4.48 (q, J=7.2 Hz, 2H), 1.45 (t, J=7.2 Hz, 3H), 1.37 (s, 12H).

### Step B: Ethyl 5-bromo-3-nitro-[2,3'-bipyridine]-6'-carboxylate

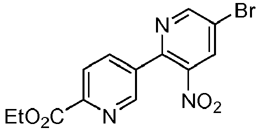

Under the protection of N₂ at room temperature, PdCl₂(dppf) (1.60 g, 0.0022 mol) was added to a THF (200 mL) and water (20 mL) solution of ethyl 5-(4,4,5,5-tetramethyl-1,3,2-dioxaborolan-2-yl)picolinate (9.00 g, 0.032 mol), 2,5-dibromo-3-nitropyridine (13.72g, 0.048 mol) and anhydrous potassium phosphate (13.58g, 0.064 mol), and the resulting mixture was heated to 70 °C to react overnight. After cooling and concentration under reduced pressure, water and ethyl acetate were added to the residue, stirred for 15 min, filtered through Celite, and the Celite was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue, which was separated through a silica gel column (ethyl acetate/petroleum ether=1:10-1:3) to obtain a brown solid product (7.64 g, 67%).
¹H NMR (400 MHz, CDCl₃) δ 9.00 (d, 1H), 8.90 (d, 1H), 8.46 (d, 1H), 8.24 (d, 1H), 8.01 (dd, 1H), 4.53 (q, 2H), 1.48 (t, 3H).

### Step C: Ethyl 5-(3,5-dimethylisoxazol-4-yl)-3-nitro-[2,3'-bipyridine]-6'-carboxylate

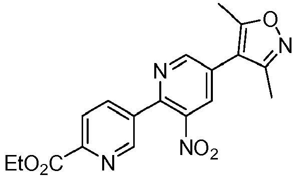

Under the protection of N₂ at room temperature, PdCl₂(dppf) (0.12 g, 0.16 mmol) was added to a THF (20 mL) and water (2 mL) solution of ethyl 5-bromo-3-nitro-[2,3'-bipyridine] -6'-carboxylate (1.00g, 0.0028 mol), (3,5-dimethylisoxazol -4-yl)boric acid (0.80 g, 0.0056 mol) and anhydrous potassium phosphate (1.20 g, 0.0056 mol), and the resulting mixture was heated to 70 °C to react overnight. After cooling and concentration under reduced pressure, water and ethyl acetate were added to the residue, stirred for 15 min, filtered through Celite, and the Celite was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated. The residue was separated through a silica gel column (ethyl acetate/petroleum ether=1:10-1:2) to obtain ethyl 5-(3,5-dimethylisoxazol-4-yl)-3-nitro-[2,3'-bipyridine]-6'-carboxylate (0.80 g, 76%).
¹H NMR (400 MHz, CDCl₃) δ 8.98 (d, 1H), 8.86 (d, 1H), 8.27 (d, 1H), 8.20 (d, 1H), 8.06 (dd, 1H), 4.53 (q, 2H), 2.55 (s, 3H), 2.39 (s, 3H), 1.48 (t, 3H).

### Step D: Ethyl 3-(3,5-dimethylisoxazol-4-yl)-5H-pyrrolo[2,3-b:4,5-b']dipyridine-7-carboxylate (D-1)

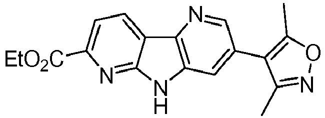

### Ethyl 3-(3,5-dimethylisoxazol-4-yl)-5H-pyrrolo[2,3-b:4,5-c']dipyridine-7-carboxylate (D-2)

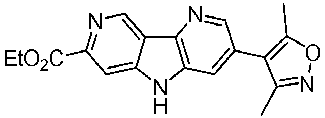

Under the protection of N₂ at room temperature, an o-dichlorobenzene (8 mL) solution of ethyl 5-(3,5-dimethylisoxazol-4-yl)-3-nitro-[2,3'-dipyridine]-6'-carboxylate (0.80 g, 2.17 mmol) and 1,2-bis(diphenylphosphino)ethane (1.73g, 4.34 mmol) was heated to 180 °C and reacted under stirring for 1h. After cooling to room temperature, dichloromethane (70 mL) was added, and the resulting mixture was stirred for 30 min and filtered. Solid was collected to obtain ethyl 3-(3,5-dimethylisoxazol-4-yl)-5H-pyrrolo[2,3-b:4,5-b']dipyridine-7-carboxylate (D-1, 210 mg, 29%).
¹H NMR (400 MHz, DMSO-d₆) δ 12.46 (s, 1H), 8.73 (d, 1H), 8.58 (d, 1H), 8.03 (d, 1H), 7.95 (d, 1H), 4.38 (q, 2H), 2.49 (s, 3H), 2.29 (s, 3H), 1.37 (t, 3H).

The filtrate was separated through a silica gel column (ethyl acetate/dichloromethane=1:10-1:1) to obtain ethyl 3-(3,5-dimethylisoxazol-4-yl)-5H-pyrrolo [2,3-b:4,5-c']dipyridine-7-carboxylate (D-2, 140 mg, 19%).
¹H NMR (400 MHz, DMSO-d₆) δ 12.23 (s, 1H), 9.47 (s, 1H), 8.63 (d, 1H), 8.29 (s, 1H), 8.10 (d, 1H), 4.38 (q, 2H), 2.49 (s, 3H), 2.29 (s, 3H), 1.37 (t, 3H).

### Step E: Ethyl 3-(3,5-dimethylisoxazol-4-yl)-5-[phenyl(tetrahydro-2H-pyran-4-yl)methyl]-5H-pyrrolo[2,3-b:4,5-c']dipyridine-7-carboxylate

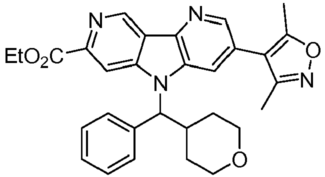

Diisopropyl azodiformate (85 mg, 0.42 mmol) was added dropwise to an anhydrous tetrahydrofuran (2 mL) solution containing ethyl 3-(3,5-dimethylisoxazol-4-yl)-5H-pyrrolo [2,3-b:4,5-c']dipyridine-7-carboxylate (70 mg, 0.21 mmol), phenyl(tetrahydro-2H-pyran-4-yl) methanol (80 mg, 0.42 mmol) and triphenylphosphine (110 mg, 0.42 mmol) at 0 °C. After the addition was completed, the resulting mixture was slowly warmed to 30°C to react for 2h. After the reaction was completed, the reaction mixture was directly separated with a preparative thin layer chromatography plate, using dichloromethane/methanol/ammonia water=500:25:1 as the developing solvent, to obtain the target product (25 mg, 24%).
¹H NMR (400 MHz, CD₃OD) δ 9.54 (s, 1H), 8.78 (s, 1H), 8.60 (d, 1H), 8.31 (d, 1H), 7.63-7.66 (m, 2H), 7.36-7.40 (m, 2H), 7.28-7.31 (m, 1H), 5.94 (d, 1H), 4.53 (q, 2H), 3.97-4.00 (m, 1H), 3.79-3.83 (m, 1H), 3.58-3.64 (m, 1H), 3.33-3.42 (m, 2H), 2.45 (s, 3H), 2.28 (s, 3H), 1.94-2.00 (m, 1H), 1.61-1.68 (m, 1H), 1.48 (t, 3H), 1.38-1.43 (m, 1H), 1.00-1.04 (m, 1H).

### Example 4

### 2-{3-(3,5-Dimethylisoxazol-4-yl)-5-[phenyl(tetrahydro-2H-pyran-4-yl)methyl]-5H-pyrrolo [3,2-b:4,5-c']dipyridin-7-yl}propan-2-ol

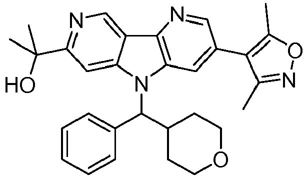

A 3 mol/L diethyl ether solution of methylmagnesium bromide (0.24 mL, 0.72 mmol) was added dropwise slowly to a tetrahydrofuran (2 mL) solution of ethyl 3-(3,5-dimethylisoxazol-4 -yl)-5-[phenyl(tetrahydro-2H-pyran-4-yl)methyl]-5H-pyrrolo[2,3-b:4,5-c']dipyridine-7-carboxylate (25 mg, 0.049 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane/methanol/ammonia water=500:25:1 as the developing solvent, to obtain the target product (15 mg, 62%).
¹H NMR (400 MHz, CD₃OD) δ 9.40 (s, 1H), 8.48 (d, 1H), 8.23 (s, 1H), 8.20 (d, 1H), 7.62-7.64 (m, 2H), 7.36-7.40 (m, 2H), 7.26-7.30 (m, 1H), 5.77 (d, 1H), 3.97-4.00 (m, 1H), 3.79-3.83 (m, 1H), 3.55-3.62 (m, 1H), 3.33-3.42 (m, 2H), 2.43 (s, 3H), 2.26 (s, 3H), 1.94-2.00 (m, 1H), 1.61-1.69 (m, 7H), 1.38-1.43 (m, 1H), 1.05-1.09 (m, 1H).

### Example 5

### Ethyl 3-(3,5-dimethylisoxazol-4-yl)-5-[phenyl(tetrahydro-2H-pyran-4-yl)methyl]-5H-pyrrolo [2,3-b:4,5-b']dipyridine-7-carboxylate

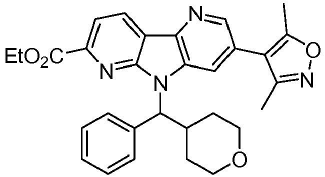

Diisopropyl azodiformate (85 mg, 0.42 mmol) was added dropwise to an anhydrous tetrahydrofuran (2 mL) solution containing ethyl 3-(3,5-dimethylisoxazol-4-yl)-5H-pyrrolo [2,3-b:4,5-b']dipyridine-7-carboxylate (70 mg, 0.21 mmol), phenyl(tetrahydro-2H-pyran-4-yl) methanol (80 mg, 0.42 mmol) and triphenylphosphine (110 mg, 0.42 mmol) at 0°C. After the addition was completed, the resulting mixture was slowly warmed to room temperature to react for 2h. After the reaction was completed, the reaction mixture was separated by PTLC, using dichloromethane/methanol/ammonia water=500:25:1 as the developing solvent, to obtain the target product (52 mg, 50%).
¹H NMR (400 MHz, CD₃Cl) δ 8.71 (d, 1H), 8.49 (d, 1H), 8.17 (d, 1H), 7.69 (d, 1H), 7.65-7.66 (m, 2H), 7.29-7.33 (m, 2H), 7.23-7.26 (m, 1H), 5.86 (d, 1H), 4.55 (q, 2H), 3.96-4.05 (m, 1H), 3.82-3.903 (m, 1H), 3.55-3.62 (m, 1H), 3.48-3.57 (m, 1H), 3.36-3.42 (m, 1H), 2.43 (s, 3H), 2.28 (s, 3H), 1.75-1.81 (m, 1H), 1.56-1.60 (m, 4H), 1.38-1.42 (m, 1H), 1.19-1.22 (m, 1H).

### Example 6

### 2-{3-(3,5-Dimethylisoxazol-4-yl)-5-[phenyl(tetrahydro-2H-pyran-4-yl)methyl]-5H-pyrrolo [2,3-b:4,5-b']dipyridin-7-yl}propan-2-ol

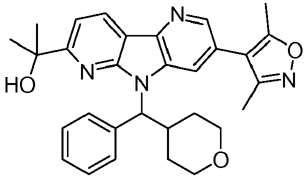

A 3 mol/L diethyl ether solution of methylmagnesium bromide (0.24 mL, 0.72 mmol) was added dropwise slowly to a tetrahydrofuran (2 mL) solution of ethyl 3-(3,5-dimethylisoxazol-4- yl)-5-[phenyl(tetrahydro-2H-pyran-4-yl)methyl]-5H-pyrrolo[2,3-b:4,5-b']dipyridine-7-carboxylate (26 mg, 0.051 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane/methanol/ammonia water=500:25:1 as the developing solvent, to obtain the target product (13 mg, 51%).
¹H NMR (400 MHz, CD₃OD) δ 8.62 (d, 1H), 8.41 (d, 1H), 8.21 (d, 1H), 7.72-7.77 (m, 3H), 7.27-7.31 (m, 2H), 7.20-7.23 (m, 1H), 5.85 (d, 1H), 3.97-4.00 (m, 1H), 3.72-3.85 (m, 2H), 3.48-3.55 (m, 1H), 3.36-3.42 (m, 1H), 2.45 (s, 3H), 2.29 (s, 3H), 1.76 (s, 6H), 1.62-1.68 (m, 1H), 1.50-1.59 (m, 1H), 1.36-1.44 (m, 1H), 1.23-1.26 (m, 1H).

### Example 7

### 3,5-Dimethyl-4-{5-[phenyl(tetrahydro-2H-pyran-4-yl)methyl]-5H-pyrrolo[3,2-b:4,5-c']dipyridin -3-yl}isoxazole

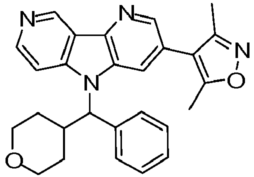

### Step A: 4-(6-Chloro-5-nitropyridin-3-yl)-3,5-dimethylisoxazole

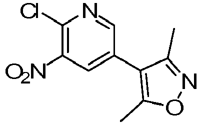

Under the protection of N₂, PdCl₂(dppf) (0.774 g, 1.06 mmol) was added to a tetrahydrofuran/water (4:1=120 mL) solution of 5-bromo-2-chloro-3-nitropyridine (5.00g, 0.021 mol), (3,5-dimethylisoxazol-4-yl)boric acid (2.98 g, 0.021 mol) and anhydrous potassium phosphate (4.90 g, 0.023 mol), and the resulting mixture was heated to 70°C to react for 4h. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a brown residue. The residue was purified by silica gel column chromatography eluting with ethyl acetate/petroleum ether (10:1) to obtain a yellow solid product (1.6 g, 30%).
¹H NMR (400 MHz, CDCl₃) δ 8.55 (d, 1H), 8.12 (d, 1H), 2.49 (s, 3H), 2.33 (s, 3H).

### Step B: 3,5-Dimethyl-4-(3-nitro-[2,3'-bipyridine]-5-yl)isoxazole

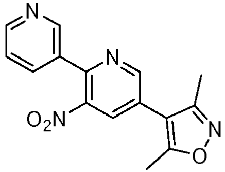

Under the protection of N₂, PdCl₂(dppf) (0.046 g, 0.063 mmol) was added to a tetrahydrofuran/water (4:1=20 mL) solution of 4-(6-chloro-5-nitropyridin-3-yl)-3,5-dimethylisoxazole (0.32 g, 1.26 mmol), (pyridin-3-yl)boric acid (0.17 g, 1.38 mmol) and anhydrous potassium phosphate (0.29 g, 1.37 mmol), and the resulting mixture was heated to 70°C to react for 4h. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a brown residue. The residue was purified by silica gel column chromatography eluting with ethyl acetate/petroleum ether (4:1) to obtain a yellow solid product (0.30 g, 80%).
¹H NMR (400 MHz, CDCl₃) δ 8.83-8.87 (m, 1H), 8.84 (d, 1H), 8.72-8.75 (m, 1H), 8.15 (d, 1H), 7.91-7.94 (m, 1H), 7.43-7.47(m, 1H), 2.54 (s, 3H), 2.38 (s, 3H).

### Step C: 3,5-Dimethyl-4-(5H-pyrrolo[3,2-b:4,5-b']dipyridin-3-yl)isoxazole (C-1)

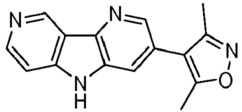

### 3,5-Dimethyl-4-(5H-pyrrolo[2,3-b:4,5-b']dipyridin-3-yl)isoxazole (C-2)

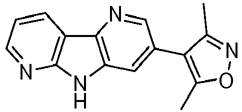

Under the protection of N₂, an o-dichlorobenzene (5 mL) solution of 3,5-dimethyl-4-(3-nitro-[2,3'-bipyridine]-5-yl)isoxazole (0.30 g, 1.01 mmol) and 1,2-bis(diphenylphosphino)ethane (0.80 g, 2.02 mmol) was heated to 180°C and reacted under stirring for 1h. After cooling to room temperature, dichloromethane (70 mL) was added, and the resulting mixture was stirred for 30 min and filtered. Solid was collected, and purified by silica gel column chromatography eluting with ethyl acetate/petroleum ether (2:1) to obtain 3,5-dimethyl-4-(5H-pyrrolo[3,2-b:4,5-b']dipyridin-3-yl)isoxazole (C-1, 123 mg, 46%) and 3,5-dimethyl-4-(5H-pyrrolo[2,3-b:4,5-b']dipyridin-3-yl)isoxazole (C-2, 80 mg, 30%).
C-1: ¹H NMR (400 MHz, CDCl₃) δ 9.61 (s, 1H), 8.58 (d, 1H), 8.51 (s, 1H), 7.73 (s, 1H), 7.51 (d, 1H), 7.40 (s, 1H), 2.42 (s, 3H), 2.27 (s, 3H).
C-2: ¹H NMR (400 MHz, CDCl₃) δ 10.39 (br, 1H), 8.71 (dd, 1H), 8.66 (dd, 1H), 8.53 (d, 1H), 7.76 (d, 1H), 7.37 (dd, 1H), 2.50 (s, 3H), 2.35 (s, 3H).

### Step D: 3,5-Dimethyl-4- {5-[phenyl(tetrahydro-2H-pyran-4-yl)methyl]-5H-pyrrolo[3,2-b:4,5-c'] dipyridin-3-yl}isoxazole

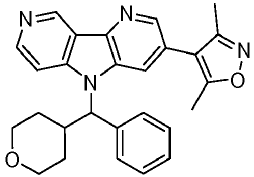

Diisopropyl azodiformate (57 mg, 0.28 mmol) was added dropwise to an anhydrous tetrahydrofuran (2 mL) solution containing 3,5-dimethyl-4-(5H-pyrrolo[3,2-b:4,5-b']dipyridin-3-yl) isoxazole (37 mg, 0.14 mmol), phenyl(tetrahydro-2H-pyran-4-yl)methanol (52 mg, 0.28 mmol) and triphenylphosphine (73 mg, 0.28 mmol) at 0°C. After the addition was completed, the resulting mixture was slowly warmed to 30°C to react for 2h. After the reaction was completed, the reaction mixture was directly separated with a preparative thin layer chromatography plate, using dichloromethane: methanol=50:1 as the developing solvent, to obtain the target product (8 mg, 13%).
¹H NMR (400 MHz, CDCl₃) δ 9.64 (s, 1H), 8.89 (s, 1H), 8.53 (s, 1H), 7.34-7.54 (m, 7H), 5.48 (d, 1H), 4.01-4.14 (m, 1H), 3.81-3.92 (m, 1H), 3.48-3.60 (m, 1H), 3.31-3.40 (m, 1H), 3.00-3.16 (m, 1H), 2.39 (s, 3H), 2.22 (s, 3H), 1.98-2.10 (m, 1H), 1.44-1.61 (m, 1H), 1.24-1.40 (m, 1H), 1.01-1.12 (m, 1H).

### Example 8

### 3,5-Dimethyl-4-(5-phenyl(tetrahydro-2H-pyran-4-yl)methyl-5H-pyrrolo[2,3-b:4,5-b']dipyridin-3-yl)isoxazole

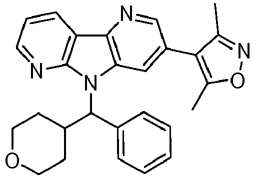

Diisopropyl azodiformate (61 mg, 0.302 mmol) was added dropwise to an anhydrous tetrahydrofuran (1 mL) solution containing 3,5-dimethyl-4-(5H-pyrrolo[2,3-b:4,5-b']dipyridin-3-yl) isoxazole (40 mg, 0.151 mmol), phenyl(tetrahydro-2H-pyran-4-yl)methanol (29 mg, 0.151 mmol) and triphenylphosphine (79 mg, 0.302 mmol) at 0°C. After the addition was completed, the resulting mixture was slowly warmed to 30°C to react for 2h. After the reaction was completed, the reaction mixture was directly separated with a preparative thin layer chromatography plate, using dichloromethane: methanol=40:1 as the developing solvent, to obtain the target product (30 mg, 45%).
¹H NMR (400 MHz, CDCl₃) δ 8.71 (dd, 1H), 8.62 (dd, 1H), 8.43 (d, 1H), 7.64 (d, 1H), 7.60 (d, 2H), 7.25-7.38 (m, 4H), 5.99 (d, 1H), 4.00-4.03 (m, 1H), 3.80-3.89 (m, 1H), 3.48-3.58 (m, 1H), 3.34-3.41 (m, 2H), 2.40 (s, 3H), 2.24 (s, 3H), 1.78-1.84 (m, 1H), 1.66-1.77 (m, 1H), 1.37-1.42 (m, 1H), 1.14-1.20 (m, 1H).

### Example 9

### Ethyl (S)-3-(3,5-dimethylisoxazol-4-yl)-5-[phenyl(tetrahydro-2H-pyran-4-yl)methyl]-5H-pyrrolo [3,2-b:4,5-c']dipyridine-7-carboxylate

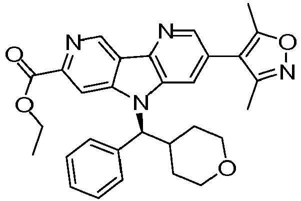

### Step A: (2S)-1-p-tosyl-pyrrolidine-2-carboxylic acid

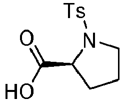

p-Toluenesulfonyl chloride (47.2 g, 0.244 mol) was added to a mixture of (S)-pyrrolidine-2-carboxylic acid (14.0g, 0.122 mol) and a sodium hydroxide aqueous solution (240 mL, 2 mol/L), and the resulting mixture was heated to 70°C to react for 2h. After cooling and suction filtration under reduced pressure, the aqueous phase was adjusted with 1N hydrochloric acid to pH 3, and the resulting mixture was extracted with ethyl acetate (3X100 mL). The ethyl acetate phases were combined, washed with a saturated sodium chloride aqueous solution, dried with anhydrous sodium sulfate, and concentrated to obtain a white solid product (32.4 g, 99%).
¹H NMR (400 MHz, CDCl₃,) δ 7.77 (d, 2H), 7.35 (d, 2H), 4.27 (q, 1H), 3.48-3.60 (m, 1H), 3.23-3.29 (m, 1H), 2.45 (s, 3H), 2.13-2.21 (m, 1H), 1.88-2.01 (m, 2H), 1.68-1.80 (m, 1H).

### Step B: [(S)-Phenyl(tetrahydro-2H-pyran-4-yl)]methyl (2S)-1-p-tosylpyrrolidine-2-carboxylate (B-1)

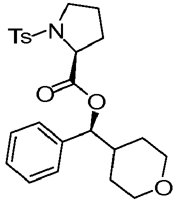

### [(R)-Phenyl(tetrahydro-2H-pyran-4-yl)]methyl (2S)-1-p-tosylpyrrolidine-2-carboxylate (B-2)

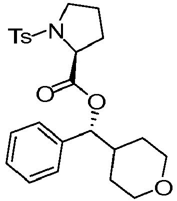

Diisopropyl azodiformate (4.10 g, 0.02 mol) was added dropwise to anhydrous tetrahydrofuran (60.00 mL) containing (2S)-1-p-tosylpyrrolidine-2-carboxylic acid (4.20 g, 0.015 mol), phenyl(tetrahydro-2H-pyran-4-yl)methanol (3.00 g, 0.015 mol) and triphenylphosphine (5.30 g, 0.02 mol) at 0 °C. After the addition was completed, the resulting mixture was slowly warmed to 30°C to react for 2h. After the reaction was completed, the residue was purified by silica gel column chromatography eluting with ethyl acetate/petroleum ether=1:4 to obtain the target product [(S)-phenyl(tetrahydro-2H-pyran-4-yl)]methyl (2S)-1-p-tosylpyrrolidine-2-carboxylate (B-1, 2.5 g, 36%) and [(R)-phenyl(tetrahydro-2H-pyran-4-yl)]methyl (2S)-1-p-tosylpyrrolidine-2-carboxylate (B-2, 1.6 g, 23%).
B-1: ¹H NMR (400 MHz, CDCl₃) δ 7.76 (d, 2H), 7.23-7.39 (m, 7H), 5.51 (d, 1H), 4.38-4.42 (m, 1H), 3.96-4.06 (m, 1H), 3.84-3.94 (m, 1H), 3.24-3.45 (m, 4H), 2.42 (s, 3H), 1.68-2.10 (m, 5H), 1.20-1.53 (m, 4H).
B-2: ¹H NMR (400 MHz, CDCl₃) δ 7.58 (d, 2H), 7.28-7.39 (m, 5H), 7.21 (d, 2H), 5.41 (d, 1H), 4.21-4.35 (m, 1H), 3.88-3.94 (m, 1H), 3.82-3.86 (m, 1H), 3.40-3.46 (m, 1H), 3.18-3.35 (m, 3H), 2.33 (s, 3H), 1.80-1.94 (m, 4H), 1.62-1.73 (m, 1H), 1.16-1.42 (m, 4H).

### Step C: (R)-Phenyl(tetrahydro-2H-pyran-4-yl)methanol

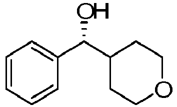

Under the protection of N₂, an aqueous solution (1 mol/L) of lithium hydroxide (0.20 mL, 0.2 mmol) was added to a methanol (1.0 mL) solution of [(R)-phenyl(tetrahydro-2H-pyran-4-yl)]methyl (2S)-1-p-tosylpyrrolidine-2-carboxylate (83 mg, 0.19 mmol), and the resulting mixture reacted at room temperature overnight. After adjusting to pH 3, water and ethyl acetate were added to the reaction flask, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a luminous yellow residue. The residue was purified by silica gel column chromatography eluting with ethyl acetate/petroleum ether (4:1) to obtain a colorless oily product (30 mg, 84%).
¹H NMR (400 MHz, CDCl₃) δ 7.25-7.39 (m, 5H), 4.34 (d, 1H), 3.95-4.10 (m, 1H), 3.84-3.92 (m, 1H), 3.22-3.40 (m, 2H), 1.79-92 (m, 2H), 1.38-1.52 (m, 1H), 1.23-1.37 (m, 1H), 1.11-1.18 (m, 1H).
[α]^{D}₂₅=+40.50° (C=0.09 CHCl₃).
HPLC RT=12.02 min (column: Chiralcel OJ-H column 4.6*250mm, 5µm; column temperature: 25°C; flow rate: 1.0 mL/min; wavelength: 210 nm; mobile phase: n-hexane-ethanol=85:15).

### Step D: Ethyl (S)-3-(3,5-dimethylisoxazol-4-yl)-5-[phenyl(tetrahydro-2H-pyran-4-yl)methyl]-5H-pyrrolo[3,2-b:4,5-c']dipyridine-7-carboxylate

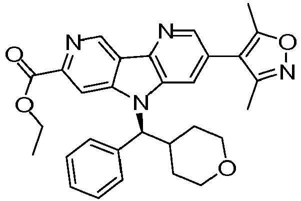

The target product was obtained using the method described in Example 3 with ethyl 3-(3,5-dimethylisoxazol-4-yl)-5H-pyrrolo[3,2-b:4,5-c']dipyridine-7-carboxylate and (R)-phenyl(tetrahydro-2H-pyran-4-yl)methanol as starting materials.
¹H NMR (400 MHz, CD₃OD) δ 9.54 (s, 1H), 8.78 (s, 1H), 8.60 (d, 1H), 8.31 (d, 1H), 7.63-7.66 (m, 2H), 7.36-7.40 (m, 2H), 7.28-7.31 (m, 1H), 5.94 (d, 1H), 4.53 (q, 2H), 3.97-4.00 (m, 1H), 3.79-3.83 (m, 1H), 3.58-3.64 (m, 1H), 3.33-3.42 (m, 2H), 2.45 (s, 3H), 2.28 (s, 3H), 1.94-2.00 (m, 1H), 1.61-1.68 (m, 1H), 1.48 (t, 3H), 1.38-1.43 (m, 1H), 1.00-1.04 (m, 1H).

### Example 10

### (S)-2-{3-(3,5-dimethylisoxazol-4-yl)-5-[phenyl(tetrahydro-2H-pyran-4-yl)methyl]-5H-pyrrolo [3,2-b:4,5-c']dipyridin-7-yl}propan-2-ol

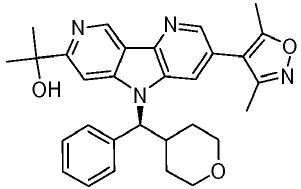

The target product was obtained using the method described in Example 4 with ethyl (S)-3-(3,5-dimethylisoxazol-4-yl)-5-[phenyl(tetrahydro-2H-pyran-4-yl)methyl]-5H-pyrrolo [3,2-b:4,5-c']dipyridine-7-carboxylate as a starting material.
¹H NMR (400 MHz, CD₃OD) δ 9.40 (s, 1H), 8.48 (d, 1H), 8.23 (s, 1H), 8.20 (d, 1H), 7.62-7.64 (m, 2H), 7.36-7.40 (m, 2H), 7.26-7.30 (m, 1H), 5.77 (d, 1H), 3.97-4.00 (m, 1H), 3.79-3.83 (m, 1H), 3.55-3.62 (m, 1H), 3.33-3.42 (m, 2H), 2.43 (s, 3H), 2.26 (s, 3H), 1.94-2.00 (m, 1H), 1.61-1.69 (m, 7H), 1.38-1.43 (m, 1H), 1.05-1.09 (m, 1H).
HPLC RT=88.74 min (column: Chiralcel IC column 4.6*250mm, 5µm; column temperature: 40°C; flow rate: 1.0 mL/min; wavelength: 230 nm; mobile phase: n-hexane:isopropanol=80:20).

### Example 11

### Ethyl (R)-3-(3,5-dimethylisoxazol-4-yl)-5-[phenyl(tetrahydro-2H-pyran-4-yl)methyl]-5H-pyrrolo [3,2-b:4,5-c']dipyridine-7-carboxylate

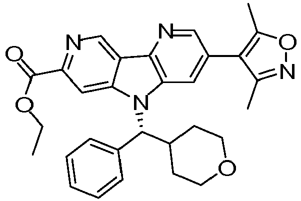

### Step A: (S)-Phenyl(tetrahydro-2H-pyran-4-yl)methanol

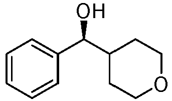

Under the protection of N₂, an aqueous solution (1 mol/L) of lithium hydroxide (0.3 mL, 0.3 mmol) was added to a methanol (1.5 mL) solution of [(S)-phenyl(tetrahydro-2H-pyran-4-yl)]methyl (2S)-1-p-tosylpyrrolidine-2-carboxylate (120 mg, 0.27 mmol, Step B in Example 9), and the resulting mixture reacted at room temperature overnight. After adjusting to pH 3, water and ethyl acetate were added to the reaction flask, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a luminous yellow residue. The residue was purified by silica gel column chromatography eluting with ethyl acetate/petroleum ether (3:1) to obtain a light yellow oily product (44 mg, 85%).
¹H NMR (400 MHz, CDCl₃) δ 7.25-7.39 (m, 5H), 4.34 (d, 1H), 3.95-4.10 (m, 1H), 3.84-3.92 (m, 1H), 3.22-3.40 (m, 2H), 2.07 (s, 1H), 1.79-92 (m, 2H), 1.38-1.52 (m, 1H), 1.23-1.37 (m, 1H), 1.11-1.18 (m, 1H).
[α]^{D}₂₅=-41.45° (C=0.088 CHCl₃).
HPLC RT=7.20 min (column: Chiralcel OJ-H column 4.6*250mm, 5µm; column temperature: 25°C; flow rate: 1.0 mL/min; wavelength: 210 nm; mobile phase: n-hexane-ethanol=85:15).

### Step B: Ethyl (R)-3-(3,5-dimethylisoxazol-4-yl)-5-[phenyl(tetrahydro-2H-pyran-4-yl)methyl] - 5H-pyrrolo[3,2-b:4,5-c']dipyridine-7-carboxylate

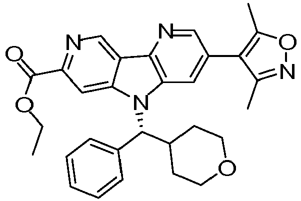

The target product was obtained using the method described in Example 3 with ethyl 3-(3,5-dimethylisoxazol-4-yl)-5H-pyrrolo[3,2-b:4,5-c']dipyridine-7-carboxylate and (S)-phenyl(tetrahydro-2H-pyran-4-yl)methanol as starting materials.
¹H NMR (400 MHz, CD₃OD) δ 9.54 (s, 1H), 8.78 (s, 1H), 8.60 (d, 1H), 8.31 (d, 1H), 7.63-7.66 (m, 2H), 7.36-7.40 (m, 2H), 7.28-7.31 (m, 1H), 5.94 (d, 1H), 4.53 (q, 2H), 3.97-4.00 (m, 1H), 3.79-3.83 (m, 1H), 3.58-3.64 (m, 1H), 3.33-3.42 (m, 2H), 2.45 (s, 3H), 2.28 (s, 3H), 1.94-2.00(m, 1H), 1.61-1.68 (m, 1H), 1.48 (t, 3H), 1.38-1.43 (m, 1H), 1.00-1.04 (m, 1H).

### Example 12

### (R)-2-{3-(3,5-dimethylisoxazol-4-yl)-5-[phenyl(tetrahydro-2H-pyran-4-yl)methyl]-5H-pyrrolo [3,2-b:4,5-c']dipyridin-7-yl}propan-2-ol

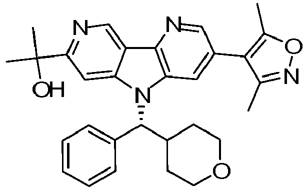

The target product was obtained using the method described in Example 4 with ethyl (R)-3-(3,5-dimethylisoxazol-4-yl)-5-[phenyl(tetrahydro-2H-pyran-4-yl)methyl]-5H-pyrrolo [3,2-b:4,5-c']dipyridine-7-carboxylate as a starting material.
¹H NMR (400 MHz, CD₃OD) δ 9.40 (s, 1H), 8.48 (d, 1H), 8.23 (s, 1H), 8.20 (d, 1H), 7.62-7.64 (m, 2H), 7.36-7.40 (m, 2H), 7.26-7.30 (m, 1H), 5.77 (d, 1H), 3.97-4.00 (m, 1H), 3.79-3.83 (m, 1H), 3.55-3.62 (m, 1H), 3.33-3.42 (m, 2H), 2.43 (s, 3H), 2.26 (s, 3H), 1.94-2.00 (m, 1H), 1.61-1.69 (m, 7H), 1.38-1.43 (m, 1H), 1.05-1.09 (m, 1H).
HPLC RT=95.74 min (column: Chiralcel IC column 4.6*250mm, 5µm; column temperature: 40°C; flow rate: 1.0 mL/min; wavelength: 230 nm; mobile phase: n-hexane:isopropanol=80:20).

### Example 13

### Ethyl (R)-3-(3,5-dimethylisoxazol-4-yl)-5-[phenyl(tetrahydro-2H-pyran-4-yl)methyl]-5H-pyrrolo [2,3-b:4,5-b']dipyridine-7-carboxylate

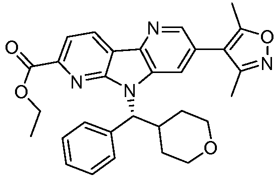

The target product was obtained using the method described in Example 5 with ethyl 3-(3,5-dimethylisoxazol-4-yl)-5H-pyrrolo[2,3-b:4,5-b']dipyridine-7-carboxylate and (S)-phenyl(tetrahydro-2H-pyran-4-yl)methanol as starting materials.
¹H NMR (400 MHz, CD₃Cl) δ 8.71 (d, 1H), 8.49 (d, 1H), 8.17 (d, 1H), 7.69 (d, 1H), 7.65-7.66 (m, 2H), 7.29-7.33 (m, 2H), 7.23-7.26 (m, 1H), 5.86 (d, 1H) 4.55 (q, 2H), 3.96-4.05 (m, 1H), 3.82-3.903 (m, 1H), 3.55-3.62 (m, 1H), 3.48-3.57 (m, 1H), 3.36-3.42 (m, 1H), 2.43 (s, 3H), 2.28 (s, 3H), 1.75-1.81 (m, 1H),1.56-1.60 (m, 4H), 1.38-1.42 (m, 1H), 1.19-1.22 (m, 1H).

### Example 14

### (R)-2-{3-(3,5-dimethylisoxazol-4-yl)-5-[phenyl(tetrahydro-2H-pyran-4-yl)methyl]-5H-pyrrolo [2,3-b:4,5-b']dipyridin-7-yl}propan-2-ol

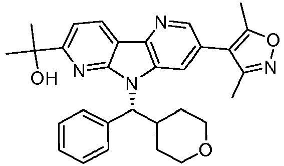

The target product was obtained using the method described in Example 6 with ethyl (R)-3-(3,5-dimethylisoxazol-4-yl)-5-[phenyl(tetrahydro-2H-pyran-4-yl)methyl]-5H-pyrrolo [2,3-b:4,5-b']dipyridine-7-carboxylate as a starting material.
¹H NMR (400 MHz, CDCl₃) δ 8.62 (d, 1H), 8.46 (s, 1H), 7.63 (s, 1H), 7.51-7.58 (m, 2H), 7.46 (d, 1H), 7.26-7.36 (m, 3H), 5.89 (d, 1H), 4.59 (s, 1H), 3.99-4.06 (m, 1H), 3.82-3.91 (m, 1H), 3.45-3.57 (m, 1H), 3.31-3.40 (m, 2H), 2.41 (s, 3H), 2.25 (s, 3H), 1.81-1.88 (m, 1H), 1.73 (m, 6H), 1.48-1.59 (m, 1H), 1.33-1.42 (m, 1H), 1.20-1.24 (m, 1H).
HPLC RT=46.64 min (column: Chiralcel IC column 4.6*250mm, 5µm; column temperature: 40°C; flow rate: 1.0 mL/min; wavelength: 230 nm; mobile phase: n-hexane:isopropanol=80:20).

### Example 15

### Ethyl (S)-3-(3,5-dimethylisoxazol-4-yl)-5-[phenyl(tetrahydro-2H-pyran-4-yl)methyl]-5H-pyrrolo [2,3-b:4,5-b']dipyridine-7-carboxylate

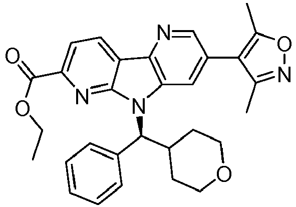

The target product was obtained using the method described in Example 5 with ethyl 3-(3,5-dimethylisoxazol-4-yl)-5H-pyrrolo[2,3-b:4,5-b']dipyridine-7-carboxylate and (R)-phenyl(tetrahydro-2H-pyran-4-yl)methanol as starting materials.
¹H NMR (400 MHz, CD₃Cl) δ 8.71 (d, 1H), 8.49 (d, 1H), 8.17 (d, 1H), 7.69 (d, 1H), 7.65-7.66 (m, 2H), 7.29-7.33 (m, 2H), 7.23-7.26 (m, 1H), 5.86 (d, 1H), 4.55 (q, 2H), 3.96-4.05 (m, 1H), 3.82-3.903 (m, 1H), 3.55-3.62 (m, 1H), 3.48-3.57 (m, 1H), 3.36-3.42 (m, 1H), 2.43 (s, 3H), 2.28 (s, 3H), 1.75-1.81 (m, 1H),1.56-1.60 (m, 4H), 1.38-1.42 (m, 1H), 1.19-1.22 (m, 1H).

### Example 16

### (S)-2-{3-(3,5-dimethylisoxazol-4-yl)-5-[phenyl(tetrahydro-2H-pyran-4-yl)methyl]-5H-pyrrolo [2,3-b:4,5-b']dipyridin-7-yl}propan-2-ol

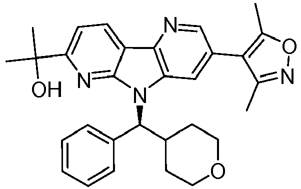

The target product was obtained using the method described in Example 6 with ethyl (S)-3-(3,5-dimethylisoxazol-4-yl)-5-[phenyl(tetrahydro-2H-pyran-4-yl)methyl]-5H-pyrrolo [2,3-b:4,5-b']dipyridine-7-carboxylate as a starting material.
¹H NMR (400 MHz, CDCl₃) δ 8.62 (d, 1H), 8.46 (s, 1H), 7.63 (s, 1H), 7.51-7.58 (m, 2H), 7.46 (d, 1H), 7.26-7.36 (m, 3H), 5.89 (d, 1H), 4.59 (s, 1H), 3.99-4.06 (m, 1H), 3.82-3.91 (m, 1H), 3.45-3.57 (m, 1H), 3.31-3.40 (m, 2H), 2.41 (s, 3H), 2.25 (s, 3H), 1.81-1.88 (m, 1H), 1.73 (m, 6H), 1.48-1.59 (m, 1H), 1.33-1.42 (m, 1H), 1.20-1.24 (m, 1H).
HPLC RT=60.32 min (column: Chiralcel IC column 4.6*250mm, 5µm; column temperature: 40°C; flow rate: 1.0 mL/min; wavelength: 230 nm; mobile phase: n-hexane-isopropanol=80:20).

### Example 17

### Ethyl 7-(1,4-dimethyl-1H-1,2,3-triazol-5-yl)-5-[phenyl(tetrahydro-2H-pyran-4-yl)methyl]-5H-pyrrolo[3,2-b:4,5-b']dipyridine-3-carboxylate

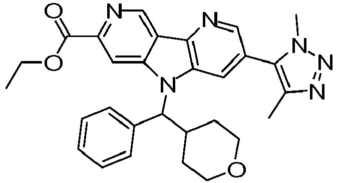

### Step A: 1,4-Dimethyl-5-(tributylstannyl)-1H-1,2,3-triazole

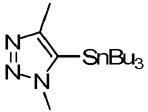

Under the protection of N₂, a tetrahydrofuran (5.0 mL) solution of 1,4-dimethyl-1,2,3-triazole (0.56 g, 5.77 mol) was added dropwise to a tetrahydrofuran (5.0 mL) solution of butyllithium (2.77mL, 6.27 mol, 2.4 mol/L n-hexane solution) at -78°C, and the resulting mixture was kept at -70°C and reacted for 1h. Then chlorotributyltin (1.71 mL, 6.34 mol) was added. The system became clear, and was gradually warmed to room temperature. Water and ethyl acetate were added to the reaction flask, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was purified by silica gel column chromatography eluting with ethyl acetate/petroleum ether (5:1) to obtain a luminous yellow oily product (1.40g, 63%).
¹H NMR (400 MHz, CDCl₃) δ 4.03 (s, 3H), 2.35 (s, 3H), 1.45-1.54 (m, 6H), 1.28-1.40 (m, 6H), 1.16-1.21 (m, 6H), 0.91 (t, 9H).

### Step B: Ethyl 5-(1,4-dimethyl-1H-1,2,3-triazol-5-yl)-3-nitro-[2,3'-bipyridine]-6'-carboxylate

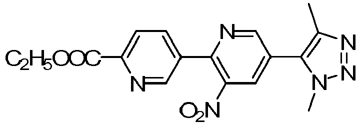

Under the protection of N₂, Pd(PPh₃)₄ (0.35g, 0.30 mmol) was added to an anhydrous dioxane (20 mL) solution of ethyl 5-bromo-3-nitro-[2,3'-bipyridine]-6'-carboxylate (1.00 g, 2.8 mmol), 1,4-dimethyl-5-(tributylstannyl)-1H-1,2,3-triazole (2.2g, 5.6 mmol) and cuprous iodide (0.40 g, 1.12 mmol), and the resulting mixture was heated to 125°C to react for 9h. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was purified by silica gel column chromatography eluting with ethyl acetate/petroleum ether (2:1) to obtain an offwhite solid product (0.64 g, 61%).
¹H NMR (400 MHz, CDCl₃) δ 8.99 (dd, 1H), 8.94 (d, 1H), 8.31 (d, 1H), 8.26 (dd, 1H), 8.10 (dd, 1H), 4.53 (q, J=7.2 Hz, 2H), 4.13 (s, 3H), 2.44 (s, 3H), 1.49 (t, J=7.2 Hz, 3H).

### Step C: Ethyl 7-(1,4-dimethyl-1H-1,2,3-triazol-5-yl)-5H-pyrrolo[3,2-b:4,5-b']dipyridine-3-carboxylate (C-1)

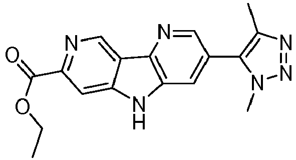

### Ethyl 3-(1,4-dimethyl-1H-1,2,3-triazol-5-yl)-5H-pyrrolo[2,3-b:4,5-b']dipyridine-7-carboxylate (C-2)

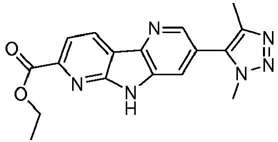

Under the protection of N₂, an o-dichlorobenzene (2 mL) solution of ethyl 5-(1,4-dimethyl-1H-1,2,3-triazol-5-yl)-3-nitro-[2,3'-bipyridine]-6'-carboxylate (104 mg, 0.283 mmol) and 1,2-bis(diphenylphosphino)ethane (0.226g, 0.566 mmol) was heated to 180°C and reacted under stirring for 4h. After cooling to room temperature, dichloromethane (70 mL) was added, stirred for 30 min, and filtered. Solid was collected to obtain the product ethyl 7-(1,4-dimethyl-1H-1,2,3-triazol-5-yl)-5H-pyrrolo[3,2-b:4,5-b']dipyridin-3-carboxylate (C-1, 40 mg, 42%).
¹H NMR (400 MHz, CDCl₃) δ 11.23 (br, 1H), 9.77 (s, 1H), 8.65 (d, 1H), 8.49 (s, 1H), 7.97 (d, 1H), 4.54 (q, 2H), 4.04 (s, 3H), 2.28 (s, 3H), 1.37 (t, 3H).

After the filtrate was concentrated, the residue was purified by silica gel column chromatography eluting with ethyl acetate/dichloromethane (3:1) to obtain ethyl 3-(1,4-dimethyl-1H-1,2,3-triazol-5-yl)-5H-pyrrolo[2,3-b:4,5-b']dipyridin-7-carboxylate (C-2, 30 mg, 32%).
¹H NMR (400 MHz, CDCl₃) δ 12.58 (br, 1H), 9.79 (d, 1H), 8.65 (d, 1H), 8.09 (d, 1H), 8.05 (d, 1H), 4.40 (q, 2H), 3.99 (s, 3H), 2.29 (s, 3H), 1.37 (m, 3H).

### Step D: Ethyl 7-(1,4-dimethyl-1H-1,2,3-triazol-5-yl)-5-[phenyl(tetrahydro-2H-pyran-4-yl)methyl] -5H-pyrrolo[3,2-b:4,5-b']dipyridine-3-carboxylate

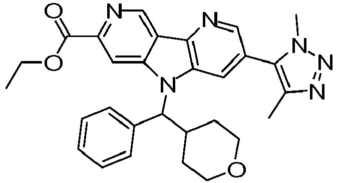

Diisopropyl azodiformate (31 mg, 0.15 mmol) was added dropwise to an anhydrous tetrahydrofuran (2 mL) solution containing ethyl 7-(1,4-dimethyl-1H-1,2,3-triazol-5-yl)-5H-pyrrolo [3,2-b:4,5-b']dipyridin-3-carboxylate (26 mg, 0.077 mmol), phenyl(tetrahydro-2H-pyran-4-yl) methanol (30 mg, 0.15 mmol) and triphenylphosphine (41 mg, 0.15 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (20 mg, 50%).
¹H NMR (400 MHz, CDCl₃) δ 9.74 (d, 1H), 8.58-8.60 (m, 2H), 7.70 (s, 1H), 7.40-7.42 (m, 2H), 7.31-7.40 (m, 3H), 5.59 (d, 1H), 4.60 (q, 2H), 4.00-4.08 (m, 1H), 3.80-3.92 (m, 4H), 3.44-3.59 (m, 1H), 3.30-3.36 (m, 1H), 3.05-3.15 (m, 1H), 2.29 (s, 3H), 1.98-2.06 (m, 1H), 1.42-1.64 (m, 4H), 1.36-1.40 (m, 1H), 1.00-1.07 (m, 1H).

### Example 18

### 2-(7-(1,4-Dimethyl-1H-1,2,3-triazol-5-yl)-5-[phenyl(tetrahydro-2H-pyran-4-yl)methyl]-5H-pyrrolo [3,2-b:4,5-b']dipyridin-3-yl)propan-2-ol

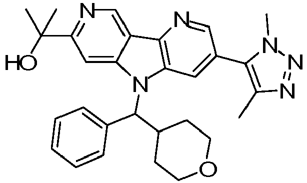

A 3 mol/L diethyl ether solution of methylmagnesium bromide (0.24 mL, 0.72 mmol) was added dropwise slowly to a tetrahydrofuran (2 mL) solution of ethyl 7-(1,4-dimethyl-1H- 1,2,3-triazol-5-yl)-5-[phenyl(tetrahydro-2H-pyran-4-yl)methyl]-5H-pyrrolo[3,2-b:4,5-b']dipyridin-3-carboxylate (20mg, 0.039 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol=25:1 as the developing solvent, to obtain the target product (12mg, 62%).
¹H NMR (400 MHz, CDCl₃) δ 9.53 (s, 1H), 8.59 (d, 1H), 7.74 (s, 1H), 7.59 (d, 1H), 7.29-7.42 (m, 5H), 5.52 (d, 1H), 4.00-4.11 (m, 1H), 3.80-3.92 (m, 4H), 3.48-3.60 (m, 1H), 3.31-3.39 (m, 1H), 3.00-3.14 (m, 1H), 2.29 (s, 3H), 1.94-2.05 (m, 2H), 1.71 (d, 6H), 1.52-1.64 (m, 1H), 1.36-1.42 (m, 1H), 1.08-1.14 (m, 1H).

### Example 19

### Ethyl 3-(1,4-dimethyl-1H-1,2,3-triazol-5-yl)-5-[phenyl(tetrahydro-2H-pyran-4-yl)methyl]-5H-pyrrolo[2,3-b:4,5-b']dipyridine-7-carboxylate

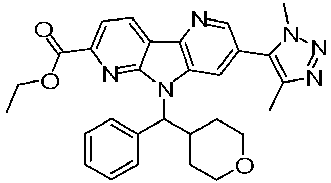

Diisopropyl azodiformate (48 mg, 0.24 mmol) was added dropwise to an anhydrous tetrahydrofuran (2 mL) solution containing ethyl 3-(1,4-dimethyl-1H-1,2,3-triazol-5-yl)-5H-pyrrolo[2,3-b:4,5-b']dipyridine-7-carboxylate (40 mg, 0.12 mmol), phenyl(tetrahydro-2H-pyran - 4-yl)methanol (46 mg, 0.24 mmol) and triphenylphosphine (63 mg, 0.24 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (40 mg, 66%).
¹H NMR (400 MHz, CDCl₃) δ 8.75 (d, 1H), 8.52 (d, 1H), 8.22 (d, 1H), 7.78 (d, 1H), 7.64 (d, 2H), 7.26-7.38 (m, 3H), 5.92 (d, 1H), 4.56 (q, 2H), 4.00-4.06 (m, 1H), 3.94 (s, 3H), 3.82-3.91 (m, 1H), 3.48-3.63 (m, 2H), 3.37-3.42 (m, 1H), 2.34 (s, 3H), 1.73-1.81 (m, 1H), 1.38-1.56 (m, 5H), 1.19-1.26 (m, 1H).

### Example 20

### 2-(3-(1,4-Dimethyl-1H-1,2,3-triazol-5-yl)-5-[phenyl(tetrahydro-2H-pyran-4-yl)methyl]-5H-pyrrolo [2,3-b:4,5-b']dipyridin-7-yl)propan-2-ol

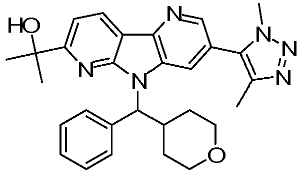

A 3 mol/L diethyl ether solution of methylmagnesium bromide (0.36 mL, 1.18 mmol) was added dropwise slowly to a tetrahydrofuran (2 mL) solution of the compound ethyl 3-(1,4-dimethyl-1H-1,2,3-triazol-5-yl)-5-[phenyl(tetrahydro-2H-pyran-4-yl)methyl]-5H-pyrrolo [2,3-b:4,5-b']dipyridin-7-carboxylate (40mg, 0.078 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=50:25:1 as the developing solvent, to obtain the target product (20 mg, 51%).
¹H NMR (400 MHz, CDCl₃) δ 8.65 (d, 1H), 8.48 (d, 1H), 7.70 (d, 1H), 7.52-7.58 (m, 3H), 7.25-7.36 (m, 3H), 5.93 (d, 1H), 4.50 (br, 1H), 4.00-4.08 (m, 1H), 3.94 (s, 3H), 3.83-3.91 (m, 1H), 3.48-3.56 (m, 1H), 3.33-3.42 (m, 2H), 2.33 (s, 3H), 1.80-1.88 (m, 1H), 1.74 (d, 6H), 1.38-1.61 (m, 2H), 1.18-1.28 (m, 1H).

### Example 21

### Methyl 7-(3,5-dimethylisoxazol-4-yl)-5-(phenyl(tetrahydro-2H-pyran-4-yl)methyl)-5H-pyrrolo [3,2-b:4,5-b']dipyridine-3-carboxylate

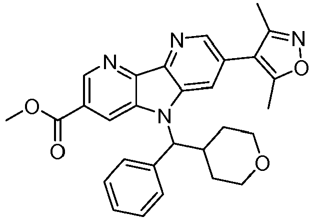

### Step A: Methyl 5-(2-chloro-5-(3,5-dimethylisoxazol-4-yl)pyridin-3-ylamino)nicotinate

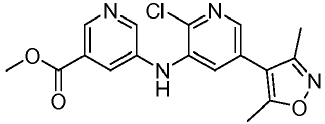

Methyl 5-bromonicotinate (773 mg, 3.6 mmol), 2-chloro-5-(3,5-dimethylisoxazol-4-yl) pyridin-3-ylamine (400 mg, 1.8 mmol, step A in Example 1), cesium carbonate (1.46 g, 4.48 mmol), Pd₂(dba)₃ (165 mg, 0.18 mmol) and xant-Phos (208 mg, 0.36 mmol) in methylbenzene (15 mL) reacted at 80°C for 8h under the protection of nitrogen. The solvent was removed under vacuum to obtain a crude product. The crude product was purified by column chromatography to obtain a white solid (334 mg, 52%).
¹H NMR (400 MHz, CDCl₃): δ 8.94 (d, J=1.6 Hz, 1H), 8.67 (d, J=2.4 Hz, 1H), 8.15-8.16 (m, 1H), 7.91 (d, J=2.0 Hz, 1H), 7.38 (d, J=2.0 Hz, 1H), 6.36 (br, 1H), 3.96 (s, 3H), 2.44 (s, 3H), 2.28 (s, 3H).

### Step B: Methyl 7-(3,5-dimethylisoxazol-4-yl)-5H-pyrrolo[3,2-b:4,5-b']dipyridine-3-carboxylate

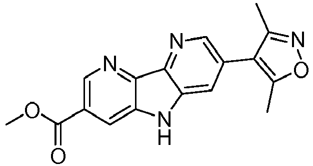

Methyl 5-(2-chloro-5-(3,5-dimethylisoxazol-4-yl)pyridin-3-ylamino)nicotinate (40 mg, 0.11 mmol), PdCl₂(dppf) (7 mg, 0.01 mmol) and sodium acetate trihydrate (29 mg, 0.22 mmol) were added to N,N-dimethylacetamide (1 mL), and the resulting mixture reacted at 180°C for 1h under the protection of nitrogen. The reaction mixture was poured into water, and extracted with ethyl acetate. The organic phase was separated, washed with a saturated saline solution, and dried. The solvent was removed under vacuum to obtain a crude product. The crude product was purified by column chromatography to obtain a white solid (8 mg, 22%).
¹H NMR (400 MHz, DMSO): δ 11.96 (s, 1H), 9.13 (d, J=1.2 Hz, 1H), 8.67 (d, J=1.2 Hz, 1H), 8.52 (d, J=2.0 Hz, 1H), 8.11 (d, J=2.0 Hz, 1H), 3.96 (s, 3H), 2.50 (s, 3H), 2.33 (s, 3H). Step C: Methyl 7-(3,5-dimethylisoxazol-4-yl)-5-(phenyl(tetrahydro-2H-pyran-4-yl)methyl)-5H-pyrrolo[3,2-b:4,5-b']dipyridin-3-carboxylate

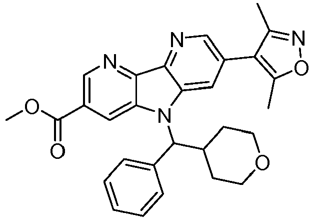

Methyl 7-(3,5-dimethylisoxazol-4-yl)-5H-pyrrolo[3,2-b:4,5-b']dipyridin-3-carboxylate (33 mg, 0.10 mmol) and phenyl(tetrahydro-2H-pyran-4-yl)methanol (38 mg, 0.20 mmol) were added to dry tetrahydrofuran (2 mL). Triphenylphosphine (52.5 mg, 0.20 mmol) and DIAD (40.4 mg, 0.20 mmol) were added, and the resulting mixture was stirred at 30°C for 1h. The solvent was removed under vacuum to obtain a crude product. The crude product was purified by column chromatography to obtain a white solid (10 mg, 20%).
¹H NMR (400 MHz, CD₃OD): δ 9.2 (br, 1H), 8.97 (s, 1H), 8.61 (br, 1H), 8.32 (s, 1H), 7.63-7.65 (m, 2H), 7.26-7.38 (m, 3H), 5.90 (d, J=11.2 Hz, 1H), 4.05 (s, 3H), 3.97-4.00 (m, 1H), 3.83-3.97 (m, 1H), 3.57-3.64 (m, 1H), 3.33-3.43 (m, 2H), 2.46 (s, 3H), 2.30 (s, 3H), 1.93-1.96 (m, 1H), 1.60-1.70 (m, 1H), 1.38-1.45 (m, 1H), 1.03-1.06 (m, 1H).

### Example 22

### 2-(7-(3,5-Dimethylisoxazol-4-yl)-5-(phenyl(tetrahydro-2H-pyran-4-yl)methyl]-5H-pyrrolo [3,2-b:4,5-b']dipyridin-3-yl)propan-2-ol

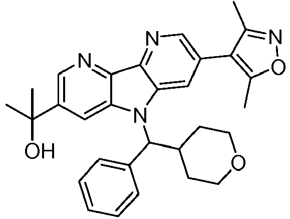

Methyl 7-(3,5-dimethylisoxazol-4-yl)-5-[phenyl(tetrahydro-2H-pyran-4-yl)methyl] - 5H-pyrrolo[3,2-b:4,5-b']dipyridine-3-carboxylate (10 mg, 0.02 mmol) was dissolved in dry tetrahydrofuran (1 mL), and cooled in an ice water bath, and then a 3 mol/L diethyl ether solution of methylmagnesium bromide (0.1 mL, 0.3 mmol) was added. The resulting mixture was stirred at 30°C for 2h. A saturated ammonium chloride aqueous solution was added to quench the reaction. The resulting mixture was extracted with ethyl acetate. The organic phase was separated, washed with a saturated saline solution, and dried. The solvent was removed under vacuum to obtain a crude product. The crude product was purified by column chromatography to obtain a white solid (2 mg, 20%).
¹H NMR (400 MHz, CD₃OD): δ 8.79 (br, 1H), 8.54 (br, 1H), 8.50 (s, 1H), 8.24 (s, 1H), 7.65-7.63 (m, 2H), 7.40-7.28 (m, 3H), 5.81 (d, J=10.8 Hz, 1H), 3.99-4.03 (m, 1H), 3.82-3.86 (m, 1H), 3.59-3.66 (m, 1H), 3.40-3.46 (m, 2H), 2.48 (s, 3H), 2.31 (s, 3H), 1.94-1.98 (m, 1H), 1.73 (s, 3H), 1.72 (s, 3H), 1.61-1.67 (m, 1H), 1.43-1.46 (m, 1H), 1.09-1.12 (m, 1H).

### Example 23

### Methyl 7-(3,5-dimethylisoxazol-4-yl)-9-fluoro-5-[phenyl(tetrahydro-2H-pyran-4-yl)methyl)-5H-pyridino[3,2-b]indole-3-carboxylate

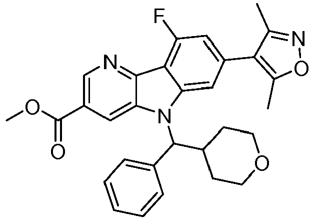

### Step A: 4-(4-Bromo-3-fluorophenyl)-3,5-dimethylisoxazole

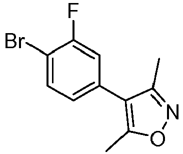

1,4-Dibromo-2-fluobenzene (1.6 g, 5.9 mmol), (3,5-dimethylisoxazol-4-yl)boric acid (830 mg, 5.9 mmol), cesium carbonate (3.9 g, 12 mmol) and PdCl₂(dppf) (219 mg, 0.3 mmol) were added to a mixed solution of dioxane (20 mL) and water (5 mL). The resulting mixture reacted at 90°C for 6h under the protection of nitrogen. The reaction mixture was poured into water, and extracted with ethyl acetate. The organic phase was separated, washed with a saturated saline solution, and dried with anhydrous sodium sulfate. The solvent was removed under vacuum to obtain a crude product. The crude product was purified by column chromatography to obtain a white solid (420 mg, 26%).
¹H NMR (400 MHz, CDCl₃): δ 7.64 (t, 1H), 7.04 (dd, 1H), 6.95 (dd, 1H), 2.43 (s, 3H), 2.29 (s, 3H).

### Step B: [4-(3,5-Dimethylisoxazol-4-yl)-2-fluorophenyl]boric acid

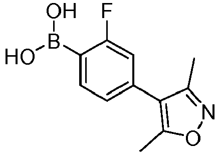

4-(4-Bromo-3-fluorophenyl)-3,5-dimethylisoxazole (216 mg, 0.8 mmol) was dissolved in freshly dried tetrahydrofuran (2 mL). After substituting the air with nitrogen, the resulting mixture was cooled to -78°C. A 2.4 mol/L n-hexane solution of n-butyllithium (0.4 mL, 0.96 mmol) was added dropwise. The reaction mixture was kept at -78°C for 1h, and isopropyl borate (300 mg, 1.6 mmol) was added. The reaction mixture was slowly warmed to room temperature to react for 3h. IN sodium hydroxide aqueous solution (5 mL) was added, and the aqueous phase was separated, acidified with IN diluted hydrochloric acid to pH 3, and extracted with ethyl acetate. The extract was washed with a saturated saline solution, and dried. The solvent was removed under vacuum to obtain a white solid (168 mg, 90%).
¹H NMR (400 MHz, CDCl₃): δ 7.92 (t, 1H), 7.12 (dd, 1H), 6.97 (dd, 1H), 5.40 (br, 2H), 2.43 (s, 3H), 2.29 (s, 3H).

### Step C: Methyl 6-[4-(3,5-dimethylisoxazol-4-yl)-2-fluorophenyl]-5-nitronicotinate

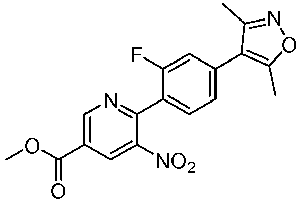

[4-(3,5-Dimethylisoxazol-4-yl)-2-fluorophenyl]boric acid (100 mg, 0.43 mmol), methyl 6-chloro-5-nitronicotinate (100 mg, 0.46 mmol), potassium phosphate (200 mg, 0.94 mmol) and PdCl₂(dppf) (29 mg, 0.04 mmol) were added to dry tetrahydrofuran (2 mL). The resulting mixture reacted at 70°C for 4h under the protection of nitrogen. The reaction mixture was poured into water, and extracted with ethyl acetate. The organic phase was separated, washed with a saturated saline solution, and dried with anhydrous sodium sulfate. The solvent was removed under vacuum to obtain a crude product. The crude product was purified by column chromatography to obtain a white solid (73 mg, 46%).
¹H NMR (400 MHz, CDCl₃): δ 9.47 (d, J=1.6 Hz, 1H), 8.91 (d, J=2.0 Hz, 1H), 7.85 (t, 1H), 7.27 (dd, 1H), 7.04 (dd, 1H), 4.05 (s, 3H), 2.48 (s, 3H), 2.33 (s, 3H).

### Step D: Methyl 7-(3,5-dimethylisoxazol-4-yl)-9-fluoro-5H-pyridino[3,2-b]indole-3-carboxylate

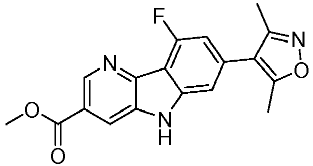

Methyl 6-[4-(3,5-dimethylisoxazol-4-yl)-2-fluorophenyl]-5-nitronicotinate (73 mg, 0.20 mmol) and 1,2-bis(diphenylphosphino)ethane (160 mg, 0.40 mmol) were added to o-dichlorobenzene (2 mL). The resulting mixture reacted at 180°C for 2h under the protection of nitrogen. The reaction mixture was purified by column chromatography to obtain a white solid (34 mg, 50%).
¹H NMR (400 MHz, DMSO-d6): δ 12.10 (brs, 1H), 9.06 (d, J=2.0 Hz, 1H), 8.44 (d, J=2.0 Hz, 1H), 7.48 (d, J=1.2 Hz, 1H), 7.15 (dd, J=11.2 Hz, J=1.2 Hz, 1H), 3.95 (s, 3H), 2.49 (s, 3H), 2.32 (s, 3H).

### Step E: Methyl 7-(3,5-dimethylisoxazol-4-yl)-9-fluoro-5-[phenyl(tetrahydro-2H-pyran-4-yl)methyl] -5H-pyridino[3,2-b]indole-3-carboxylate

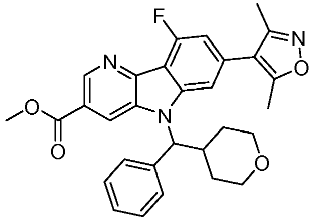

Methyl 7-(3,5-dimethylisoxazol-4-yl)-9-fluoro-5H-pyridino[3,2-b]indole-3-carboxylate (33 mg, 0.10 mmol) and phenyl(tetrahydro-2H-pyran-4-yl)methanol (38 mg, 0.20 mmol) were added to dry tetrahydrofuran (2 mL). Triphenylphosphine (52 mg, 0.20 mmol) and DIAD (40 mg, 0.20 mmol) were added, and the resulting mixture was stirred at 30°C for 1h. The solvent was removed under vacuum to obtain a crude product. The crude product was purified by column chromatography to obtain a white solid (21 mg, 42%).
¹H NMR (400 MHz, CD₃OD): δ 9.11 (br, 1H), 8.87 (br, 1H), 7.62-7.67 (m, 3H), 7.27-7.39 (m, 3H), 7.06 (dd, 1H), 5.89 (d, 1H), 4.04 (s, 3H), 3.96-4.01 (m, 1H), 3.79-3.83 (m, 1H), 3.62-3.64 (m, 1H), 3.37-3.43 (m, 2H), 2.45 (s, 3H), 2.30 (s, 3H), 1.98-2.01 (m, 1H), 1.62-1.72 (m, 1H), 1.35-1.43 (m, 1H), 1.01-1.05 (m, 1H).

### Example 24

### 2-{7-(3,5-dimethylisoxazol-4-yl)-9-fluoro-5-[phenyl(tetrahydro-2H-pyran-4-yl)methyl]-5H-pyridino[3,2-b]indol-3-yl}propan-2-ol

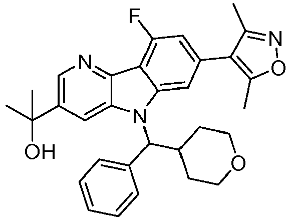

Methyl 7-(3,5-dimethylisoxazol-4-yl)-9-fluoro-5-[phenyl(tetrahydro-2H-pyran-4-yl) methyl]-5H-pyrrolo[3,2-b]indole-3-carboxylate (20 mg, 0.04 mmol) was dissolved in dry tetrahydrofuran (1 mL), and cooled in an ice water bath, and then a 3 mol/L diethyl ether solution of methylmagnesium bromide (0.2 mL, 0.6 mmol) was added. The resulting mixture was stirred at 30°C for 2h. A saturated ammonium chloride aqueous solution was added to quench the reaction. The reaction mixture was extracted with ethyl acetate. The organic phase was separated, washed with a saturated saline solution, and dried. The solvent was removed under vacuum to obtain a crude product. The crude product was purified by column chromatography to obtain a white solid (8 mg, 40%).
¹H NMR (400 MHz, CD₃OD): δ 8.64 (br, 1H), 8.39 (br, 1H), 7.56-7.60 (m, 3H), 7.25-7.37 (m, 3H), 6.98 (dd, 1H), 5.78 (d, 1H), 3.96-4.00 (m, 1H), 3.79-3.82 (m, 1H), 3.57-3.63 (m, 1H), 3.34-3.42 (m, 2H), 2.43 (s, 3H), 2.28 (s, 3H), 1.95-1.99 (m, 1H), 1.69 (s, 3H), 1.68 (s, 3H), 1.60-1.66 (m, 1H), 1.33-1.43 (m, 1H), 1.02-1.05 (m, 1H).

### Example 25

### Methyl 7-(3,5-dimethylpyridin-4-yl)-5-[phenyl(tetrahydro-2H-pyran-4-yl)methyl)-5H-pyridino [3,2-b]indole-3-carboxylate

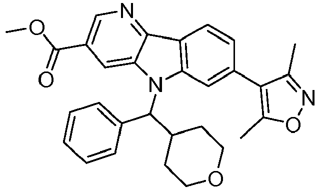

### Step A: 4-(4-Bromophenyl)-3,5-dimethylisoxazole

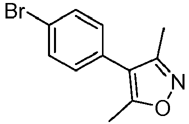

Under the protection of N₂, PdCl₂(dppf) (1.53 g, 0.0021 mol) was added to a tetrahydrofuran (126 mL) and water (32 mL) solution of 1,4-dibromobenzene (5.00g, 0.021 mol), (3,5-dimethylisoxazol-4-yl)boric acid (2.37 g, 0.0168 mol) and K₃PO₄ (13.35 g, 0.063 mol), and the resulting mixture was heated to 80°C to react for 3h. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated through a silica gel column (ethyl acetate:petroleum ether=1:5) to obtain a white solid product (2.29 g, 54%).
¹H NMR (400 MHz, CDCl₃) δ 7.58 (d, 2H), 7.14 (d, 2H), 2.40 (s, 3H), 2.27 (s, 3H).

### Step B: 4-(3,5-Dimethylisoxazol-4-yl)phenylboric acid

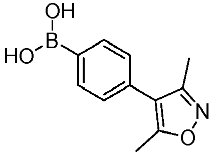

Under the protection of N₂, a 2.4 mol/L n-hexane solution of n-BuLi (3.0 mL, 0.0073 mol) was added to an anhydrous THF (10 mL) solution of 4-(4-bromophenyl)-3,5-dimethylisoxazole (1.67 g, 0.0066 mol) at -78°C to react for 30min. Isopropyl borate (1.48 g, 0.0079 mol) was added, and the resulting mixture was slowly warmed to room temperature. After the reaction was completed, a saturated ammonium chloride solution was added. The resulting mixture was extracted with ethyl acetate, washed with a saturated sodium chloride aqueous solution, dried with anhydrous sodium sulfate, and concentrated. The residue was separated through a silica gel column (ethyl acetate:petroleum ether=1:10) to obtain a white solid product (1.1 g, 76%).
¹H NMR (400 MHz, CDCl₃) δ 8.34 (d, 2H), 7.44 (d, 2H), 2.49 (s, 3H), 2.35 (s, 3H).

### Step C: Methyl 6-[4-(3,5-dimethylisoxazol-4-yl)phenyl]-5-nitronicotinate

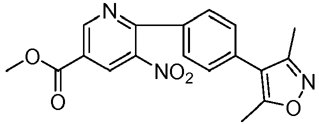

Under the protection of N₂, PdCl₂(dppf) (0.069g, 0.094 mmol) was added to an anhydrous tetrahydrofuran (10 mL) solution of 4-(3,5-dimethylisoxazol-4-yl)phenylboric acid (0.205 g, 0.94 mmol), methyl 6-chloro-5-nitronicotinate (0.244 g, 1.1 mmol) and anhydrous potassium phosphate (0.399g, 1.9 mmol), and the resulting mixture was heated to 70°C to react overnight. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated through a silica gel column (ethyl acetate:petroleum ether=1:5) to obtain a white product (0.108 g, 33%).
¹H NMR (400 MHz, CDCl₃) δ 9.45 (d, 1H), 8.76 (d, 1H), 7.73 (d, 2H), 7.42 (d, 2H), 4.08 (s, 3H), 2.49 (s, 3H), 2.35 (s, 3H).

### Step D: Methyl 7-(3,5-dimethylpyridin-4-yl)-5H-pyridino[3,2-b]indole-3-carboxylate

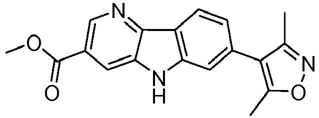

Under the protection of N₂, an o-dichlorobenzene (4 mL) solution of methyl 6-[4-(3,5-dimethylisoxazol-4-yl)phenyl]-5-nitronicotinate (0.16 g, 0.45 mmol) and 1,2-bis(diphenylphosphino)ethane (0.36 g, 0.90 mmol) was heated to 180°C and reacted under stirring for 2h. After cooling to room temperature, dichloromethane (70 mL) was added, and the resulting mixture was stirred for 30 min, and filtered. Solid was collected to obtain methyl 7-(3,5-dimethylpyridin-4-yl)-5H-pyridino[3,2-b]indole-3-carboxylate (60 mg, 41%).
¹H NMR (400 MHz, DMSO-d6) δ 11.78 (s, 1H), 9.02 (d, 1H), 8.40 (d, 1H), 8.32 (d, 1H), 7.63 (s, 1H), 7.30 (m, 1H), 3.94 (s, 3H), 2.48 (s, 3H), 2.30 (s, 3H).

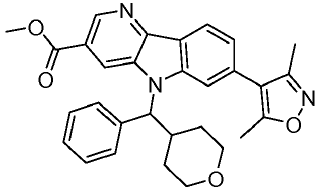

### Step E: Methyl 7-(3,5-dimethylpyridin-4-yl)-5-[phenyl(tetrahydro-2H-pyran-4-yl)methyl)-5H-pyridino[3,2-b]indole-3-carboxylate

Diisopropyl azodiformate (226 mg, 1.12 mmol) was added dropwise to an anhydrous tetrahydrofuran (2 mL) solution containing methyl 7-(3,5-dimethylpyridin-4-yl)-5H-pyridino [3,2-b]indole-3-carboxylate (90 mg, 0.28 mmol), phenyl(tetrahydro-2H-pyran-4-yl)methanol (56 mg, 0.56 mmol) and triphenylphosphine (293 mg, 1.12 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (113 mg, 81%).
¹H NMR (400 MHz, CD₃OD) δ 9.08 (s, 1H), 8.84 (s, 1H), 8.42 (s, 1H), 7.81 (s, 1H), 7.61 (d, 2H), 7.27-7.37 (m, 4H), 5.84 (d, 1H), 4.03 (s, 3H), 3.77-3.81 (m, 1H), 3.57-3.64 (m, 1H), 3.26-3.42 (m, 1H), 2.43(s, 3H), 2.28 (s, 3H), 1.94-2.00 (m, 2H), 1.58-1.68 (m, 2H), 1.29 (s, 1H), 0.89 (s, 1H).

### Example 26

### 2-{7-(3,5-Dimethylpyridin-4-yl)-5-[phenyl(tetrahydro-2H-pyran-4-yl)methyl]-5H-pyridino[3,2-b] indol-3-yl}propan-2-ol

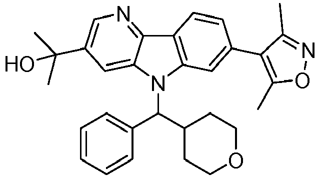

A 3 mol/L diethyl ether solution of methylmagnesium bromide (0.55 mL, 1.65 mmol) was added dropwise slowly to a tetrahydrofuran (3 mL) solution of methyl 7-(3,5-dimethylpyridin-4-yl)-5-(phenyl(tetrahydro-2H-pyran-4-yl)methyl)-5H-pyridino[3,2-b]indole-3-carboxylate (55 mg, 0.11 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=500:25:1 as the developing solvent, to obtain the target product (27 mg, 49%).
¹H NMR (400 MHz, CD₃OD) δ 8.60 (d, 1H), 8.34-7.36 (m, 2H), 7.28 (d, 1H), 7.59-7.61 (m, 2H), 7.32-7.36 (m, 2H), 7.23-7.28 (m, 2H), 5.77 (d, 1H), 3.96-4.04 (m, 1H), 3.79-3.85 (m, 1H), 3.59-3.66 (m, 1H), 3.37-3.46 (m, 2H), 2.43 (s, 3H), 2.28 (s, 3H), 1.98-2.06 (m, 1H), 1.70 (s, 3H), 1.69 (s, 3H), 1.58-1.65 (m, 1H), 1.36-1.43 (m, 1H), 1.04-1.08 (m, 1H).

### Example 27

### Ethyl 7-(3,5-dimethylisoxazol-4-yl)-9-fluoro-5-(phenyl(tetrahydro-2H-pyran-4-yl)methyl)-5H-pyridino[4,3-b]indole-3-carboxylate

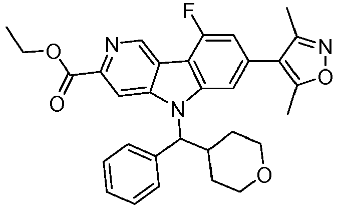

### Step A: 5-Bromo-2-(ethoxycarbonyl)pyridine1-oxide

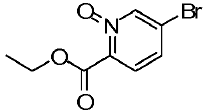

Under the protection of N₂, m-chloroperoxybenzoic acid (36.00g, 0.208 mol) was added to an ethyl acetate (126 mL) solution of ethyl 5-bromopyridine-2-carboxylate (15.00g, 0.065 mol), and the resulting mixture was heated to 70°C to react overnight. After cooling, water was added. The resulting mixture was extracted with ethyl acetate, washed with a saturated sodium sulfite aqueous solution, washed with a saturated sodium carbonate aqueous solution, dried with anhydrous sodium sulfate, and concentrated. The residue was separated through a silica gel column (ethyl acetate:petroleum ether=1:5) to obtain a yellow product (6.60 g, 41%).
¹H NMR (400 MHz, CDCl₃,) δ 8.42 (d, 1H), 7.53 (d, 1H), 7.41-7.43 (m, 1H), 4.44-4.50 (q, 2H), 1.42 (t, 3H).

### Step B: 5-Bromo-2-(ethoxycarbonyl)-4-nitropyridinel-oxide

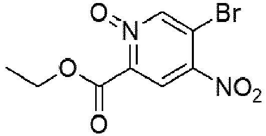

At 0 °C, fuming sulfuric acid (20 mL) was added to fuming nitric acid (20 mL), and then 5-bromo-2-(ethoxycarbonyl) pyridine1-oxide (3 g, 12.2 mmol) was added thereto. The resulting mixture was heated to 70 °C, and stirred for 20h. After the reaction was completed, the reaction system was added in ice water, and extracted with ethyl acetate. The organic phase was washed with a saturated sodium bicarbonate aqueous solution, dried with anhydrous sodium sulfate, and concentrated. The residue was separated through a silica gel column (ethyl acetate:petroleum ether=1 :5) to obtain a yellow solid product (200 mg, 5.7%).
¹H NMR (400 MHz, CDCl₃) δ 8.53 (s, 1H), 8.41 (s, 1H), 4.50 (q, 2H), 1.44 (t, 3H). Step C: 5-(4-(3,5-Dimethylisoxazol-4-yl)-2-fluorophenyl)-2-(ethoxycarbonyl)-4-nitropyridine1-oxide

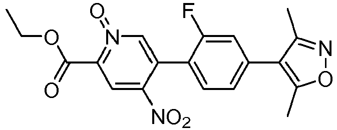

Under the protection of N₂, PdCl₂(dppf) (0.069g, 0.094 mmol) was added to an anhydrous tetrahydrofuran (10 mL) solution of (4-(3,5-dimethylisoxazol-4-yl)-2-fluorophenyl)boric acid (0.145 g, 0.618 mmol), 5-bromo-2-(ethoxycarbonyl)-4-nitropyridine1-oxide (0.36 g, 1.237 mmol) and anhydrous potassium phosphate (0.399 g, 1.9 mmol), and the resulting mixture was heated to 70°C to react overnight. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated through a silica gel column (ethyl acetate:petroleum ether=1:10-1:3) to obtain a white product (0.12 g, 48%).
¹H NMR (400 MHz, CD₃OD) δ 8.47 (s, 1H), 8.25 (s, 1H), 7.41 (s, 1H), 7.21-7.23 (m, 1H), 7.09-7.12 (m, 1H), 4.50-4.56 (m, 2H), 2.49 (s, 3H), 2.34 (s, 3H), 1.46 (t, 3H).

### Step D: Ethyl 7-(3,5-dimethylisoxazol-4-yl)-9-fluoro-5H-pyridino[4,3-b]indole-3-carboxylate

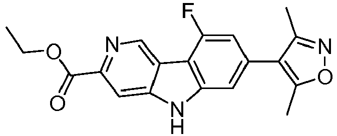

Under the protection of N₂, an o-dichlorobenzene (4 mL) solution of 5-(4-(3,5-dimethylisoxazol-4-yl)-2-fluorophenyl)-2-(ethoxycarbonyl)-4-nitropyridine1-oxide (0.10 g, 0.25 mmol) and 1,2-bis(diphenylphosphino)ethane (0.20g, 0.50 mmol) was heated to 180°C and reacted under stirring for 2h. After cooling to room temperature, dichloromethane (70 mL) was added, and the resulting mixture was stirred for 30 min, and filtered. Solid was collected to obtain ethyl 7-(3,5-dimethylisoxazol-4-yl)-9-fluoro-5H-pyridino[4,3-b]indol-3-carboxylate (50 mg, 57%).
¹H NMR (400 MHz, CD₃OD) δ 9.50 (s, 1H), 9.37 (brs, 1H), 8.34 (s, 1H), 7.24 (s, 1H), 6.96 (d, 1H), 4.52-4.57 (m, 2H), 2.48 (s, 3H), 2.33 (s, 3H), 1.49 (t, 3H).

### Step E: Ethyl 7-(3,5-dimethylisoxazol-4-yl)-9-fluoro-5-(phenyl(tetrahydro-2H-pyran-4-yl)methyl) -5H-pyridino[4,3-b]indole-3-carboxylate

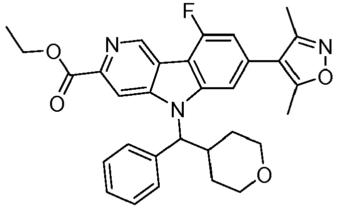

Diisopropyl azodiformate (202 mg, 1.0 mmol) was added dropwise to an anhydrous tetrahydrofuran (2 mL) solution containing ethyl 7-(3,5-dimethylisoxazol-4-yl)-9-fluoro-5H-pyridino[4,3-b]indole-3-carboxylate (40 mg, 0.11 mmol), phenyl(tetrahydro-2H-pyran-4-yl) methanol (70 mg, 0.36 mmol) and triphenylphosphine (262 mg, 1.0 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (10 mg, 16.7 %).
¹H NMR (400 MHz, CD₃Cl) δ 9.50 (s, 1H), 8.50 (s, 1H), 7.44-7.46 (m, 2H), 7.30-7.39 (m, 3H), 7.17 (s, 1H), 6.92 (d, 1H), 5.54 (d, 1H), 4.58 (q, 2H), 4.05-4.08 (m, 1H), 3.83-3.87 (m, 1H), 3.52-3.58 (m, 1H), 3.33-3.39 (m, 1H), 3.14-3.16 (m, 1H), 2.38 (s, 3H), 2.24 (s, 3H), 1.99-2.05 (m, 1H), 1.58-1.63 (m, 1H), 1.53 (t, 3H), 1.31-1.38 (m, 1H), 1.00-1.03 (m, 1H).

### Example 28

### 2-(7-(3,5-Dimethylisoxazol-4-yl)-9-fluoro-5-(phenyl(tetrahydro-2H-pyran-4-yl)methyl]-5H-pyridino[4,3-b]indole-3-yl)propan-2-ol

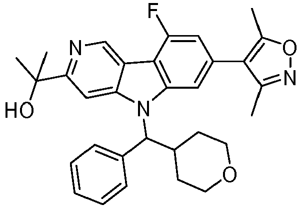

A 3 mol/L diethyl ether solution of methylmagnesium bromide (0.1 mL, 0.30 mmol) was added dropwise slowly to a tetrahydrofuran (2 mL) solution of ethyl 7-(3,5-dimethylisoxazol-4 -yl)-9-fluoro-5-(phenyl(tetrahydro-2H-pyran-4-yl)methyl)-5H-pyridino[4,3-b]indole-3-carboxylate (9 mg, 0.017 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=500:25:1 as the developing solvent, to obtain the target product (5 mg, 57%).
¹H NMR (400 MHz, CDCl₃) δ 9.29 (s, 1H), 7.56 (s, 1H), 7.25-7.44 (m, 5H), 7.09 (s, 1H), 6.87 (d, 1H), 5.47 (d, 1H), 4.01-4.06 (m, 1H), 3.82-3.92 (m, 1H), 3.49-3.60 (m, 1H), 3.31-3.40 (m, 1H), 3.01-3.17 (m, 1H), 2.36 (s, 3H), 2.22 (s, 3H), 1.94-2.00 (m, 2H), 1.52-1.72 (m, 7H), 1.41-1.45 (m, 1H),1.02-1.09 (m, 1H).

### Example 29

### Methyl 7-(3,5-dimethylisoxazol-4-yl)-9-[phenyl(tetrahydro-2H-pyran-4-yl)methyl]-9H-pyridino [2',3':4,5]pyrrolo[2,3-d]pyrimidine-2-carboxylate

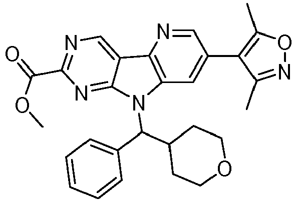

### Step A: Methyl 5-(4,4,5,5-tetramethyl-1,3,2-dioxaborolan-2-yl)pyrimidine-2-carboxylate

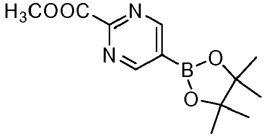

Under the protection of N₂, PdCl₂(dppf) (0.337 g, 0.461 mmol) was added to an anhydrous 1,4-dioxane (200 mL) solution of methyl 5-bromo-2-pyrimidinecarboxylate (2.00g, 9.22 mmol), bis(pinacolato)diboron (2.80 g, 11.1 mol) and anhydrous potassium acetate (2.70 g, 27.7 mol), and the resulting mixture was heated to 100°C to react overnight. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a brownish black residue. The residue was purified by silica gel column chromatography eluting with ethyl acetate/petroleum ether (5:1) to obtain a white solid product (1.47 g, 60%).
¹H NMR (400 MHz, CDCl₃) δ 9.19 (s, 2H), 4.10 (s, 3H), 1.39 (s, 12H).

### Step B: Methyl 5-[5-(3,5-dimethylisoxazol-4-yl)-3-nitropyridin-2-yl]pyrimidine-2-carboxylate

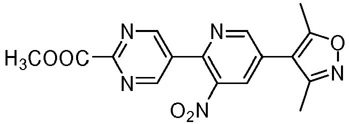

Under the protection of N₂, PdCl₂(dppf) (0.103g, 0.14 mmol) was added to a tetrahydrofuran/water (3:1=30 mL) solution of 2-chloro-5-(3,5-dimethylisoxazol-4-yl) -3-nitropyridine (0.716 g, 2.80 mmol), methyl 5-(4,4,5,5-tetramethyl-1,3,2-dioxaborolan-2-yl) pyrimidine-2-carboxylate (1.00 g, 3.79 mmol) and anhydrous potassium phosphate (0.78 g, 3.67 mmol), and the resulting mixture was heated to 70°C to react overnight. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was purified by silica gel column chromatography eluting with ethyl acetate/petroleum ether (3:1) to obtain methyl 5 - [5 -(3,5 -dimethylisoxazol-4-yl)-3 -nitropyridin-2-yl]pyrimidine-2-carboxylate (0.32 g, 32%).
¹H NMR (400 MHz, CDCl₃) δ 9.18 (s, 2H), 8.93 (d, 1H), 8.32 (d, 1H), 4.13 (s, 3H), 2.56 (s, 3H), 2.40 (s, 3H).

### Step C: Methyl 7-(3,5-dimethylisoxazol-4-yl)-9H-pyridino[2',3':4,5]pyrrolo[2,3-d]pyrimidine - 2-carboxylate

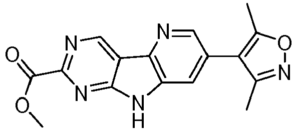

Under the protection of N₂, an o-dichlorobenzene (4 mL) solution of methyl 5-[5-(3,5-dimethylisoxazol-4-yl)-3-nitropyridin-2-yl]pyridinecarboxylate (0.32 g, 0.90 mmol) and 1,2-bis(diphenylphosphino)ethane (0.73 g, 1.80 mmol) was heated to 180°C and reacted under stirring for 2h. After cooling to room temperature, dichloromethane (70 mL) was added, stirred for 30min, and filtered to obtain the product (140 mg, 48%).
¹H NMR (400 MHz, DMSO-d6) δ 12.8 (br, 1H), 9.61 (s, 1H), 8.66 (d, 1H), 8.05 (d, 1H), 3.93 (s, 3H), 2.48 (s, 3H), 2.29 (s, 3H).

### Step D: Methyl 7-(3,5-dimethylisoxazol-4-yl)-9-[phenyl(tetrahydro-2H-pyran-4-yl)methyl]-9H-pyridino[2'3':4,5]pyrrolo[2,3-d]pyrimidine-2-carboxylate

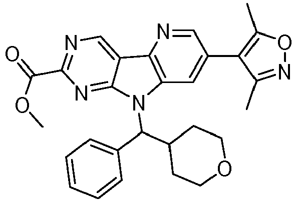

Diisopropyl azodiformate (85 mg, 0.42 mmol) was added dropwise to an anhydrous tetrahydrofuran (2 mL) solution containing methyl 7-(3,5-dimethylisoxazol-4-yl)-9H-pyridino[2',3':4,5]pyrrolo[2,3-d]pyrimidine-2-carboxylate (70 mg, 0.21 mmol), phenyl(tetrahydro -2H-pyran-4-yl)methanol (80 mg, 0.42 mmol) and triphenylphosphine (110 mg, 0.42 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (25 mg, 24%).
¹H NMR (400 MHz, CDCl₃) δ 9.72 (s, 1H), 8.58 (d, 1H), 7.73 (d, 1H), 7.43-7.51 (m, 2H), 7.25-7.37 (m, 3H), 5.91 (d, 1H), 4.18 (s, 3H), 3.97-4.08 (m, 1H), 3.79-3.96 (m, 1H), 3.46-3.55 (m, 1H), 3.35-3.41 (m, 1H), 3.17-3.30 (m, 1H), 2.40 (s, 3H), 2.24 (s, 3H), 1.74-1.85 (m, 1H), 1.46-1.62 (m, 1H), 1.32-1.40 (m, 1H), 1.08-1.21 (m, 1H).

### Example 30

### 2-{7-(3,5-Dimethylisoxazol-4-yl)-9-[phenyl(tetrahydro-2H-pyran-4-yl)methyl]-9H-pyridino [2',3':4,5]pyrrolo[2,3-d]pyrimidin-2-yl}propan-2-ol

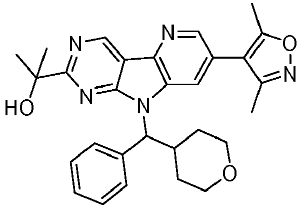

A 3 mol/L diethyl ether solution of methylmagnesium bromide (0.24 mL, 0.72 mmol) was added dropwise slowly to a tetrahydrofuran (5 mL) solution of methyl 7-(3,5-dimethylisoxazol -4-yl)-9-[phenyl(tetrahydro-2H-pyran-4-yl)methyl]-9H-pyridino[2',3':4,5]pyrrolo[2,3-d]pyrimidine-2-carboxylate (25mg, 0.050 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=500:25:1 as the developing solvent, to obtain the target product (14 mg, 56%).
¹H NMR (400 MHz, CDCl₃) δ 9.54 (s, 1H), 8.51 (d, 1H), 7.68 (d, 1H), 7.54-7.60 (m, 2H), 7.31-7.40 (m, 3H), 5.68 (d, 1H), 4.99 (br, 1H), 3.96-4.06 (m, 1H), 3.86-3.94 (m, 1H), 3.40-3.58 (m, 2H), 3.33-3.41 (m, 1H), 2.42 (s, 3H), 2.26 (s, 3H), 1.78 (s, 6H), 1.44-1.63 (m, 2H), 1.19-1.43 (m, 2H).

### Example 31

### 4-{9-Fluoro-5-[phenyl(tetrahydro-2H-pyran-4-yl)methyl]-5H-pyrrolo[3,2-b:4,5-c']dipyridin-3-yl}- 3,5-dimethylisoxazole

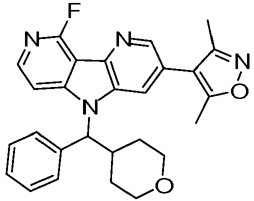

### Step A: 5-Bromo-2'-fluoro-3-nitro-2,3'-bipyridine

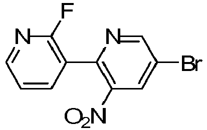

Under the protection of N₂, PdCl₂(dppf) (0.25 g, 0.34 mmol) was added to an anhydrous THF (50 mL) solution of 5-bromo-2-chloro-3-nitropyridine (1.69 g, 0.0071mol), (2-fluoropyridin-3-yl)boric acid (1.10 g, 0.0078 mol) and anhydrous potassium phosphate (1.66 g, 0.0078 mol), and the resulting mixture was heated to 70 °C to react for 4h. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated through a silica gel column (ethyl acetate/petroleum ether=1:10-1:2) to obtain a light yellow solid product (0.916 g, 43%).
¹H NMR (400 MHz, CDCl₃) δ 9.00 (d, J=2.0 Hz, 1H), 8.56 (d, J=2.0 Hz, 1H), 8.36 (ddd, J=4.9 Hz, J=2.0 Hz, J=1.2 Hz, 1H), 8.15 (ddd, J=9.4 Hz, J=7.4 Hz, J=2.0 Hz, 1H), 7.41 (ddd, J=7.3 Hz, J=4.9 Hz, J=2.0 Hz, 1H).

### Step B: 4-(2'-Fluoro-3-nitro-[2,3'-bipyridin]-5-yl)-3,5-dimethylisoxazole

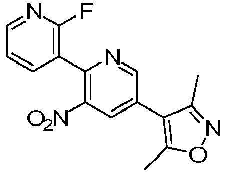

Under the protection of N₂, PdCl₂(dppf) (0.15 g, 0.0002 mol) was added to an anhydrous THF (40 mL) solution of 5-bromo-2'-fluoro-3-nitro-2,3'-bipyridine (0.90 g, 0.0030 mol), (3,5-dimethylisoxazol-4-yl)boric acid (0.46 g, 0.0033 mol) and anhydrous potassium phosphate (0.70 g, 0.0030 mol), and the resulting mixture was heated to 70°C to react overnight. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated through a silica gel column (ethyl acetate/petroleum ether=1 :10-1:2) to obtain a brown solid product (0.84 g, 89%).
¹H NMR (400 MHz, CDCl₃) δ 8.86 (d, J=2.0 Hz, 1H), 8.38 (ddd, J=4.9 Hz, J=2.0 Hz, J=1.2 Hz, 1H), 8.30 (d, J=2.0 Hz, 1H), 8.22 (ddd, J=9.4 Hz, J=7.4 Hz, J=2.0 Hz, 1H), 7.44 (ddd, J=7.3 Hz, J=4.9 Hz, J=2.0 Hz, 1H), 2.55 (s, 3H), 2.39 (s, 3H).

### Step C: 4-(9-Fluoro-5H-pyrrolo[3,2-b:4,5-c']dipyridin-3-yl)-3,5-dimethylisoxazole

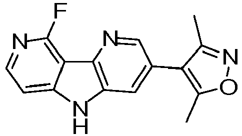

Under the protection of N₂, an o-dichlorobenzene (4 mL) solution of 4-(2'-fluoro-3-nitro-[2,3'-bipyridin]-5-yl)-3,5-dimethylisoxazole (0.40 g, 0.0013mol) and bis(diphenylphosphino)ethane (1.02 g, 0.0025 mol) was heated to 180 °C to react for 1h, and then cooled to room temperature. The residue of the system was separated through a silica gel column (ethyl acetate/dichloromethane=1:20-1:2) to obtain a light yellow solid product (0.15 g, 42%).
¹H NMR (400 MHz, CDCl₃) δ 9.67 (dd, 1H), 8.64 (d, 1H), 8.19 (dd, 1H), 7.65 (d, 1H), 7.30 (d, 1H), 2.46 (s, 3H), 2.30 (s, 3H).

### Step D: 4- {9-Fluoro-5-[phenyl(tetrahydro-2H-pyran-4-yl)methyl]-5H-pyrrolo[3,2-b:4,5-c']dipyridin -3-yl}-3,5-dimethylisoxazole

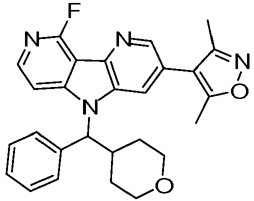

Diisopropyl azodiformate (57 mg, 0.28 mmol) was added dropwise to an anhydrous tetrahydrofuran (2 mL) solution containing 4-(9-fluoro-5H-pyrrolo[3,2-b: 4,5-c']dipyridin-3-yl)-3,5- dimethylisoxazole (40 mg, 0.14 mmol), phenyl(tetrahydro-2H-pyran-4-yl)methanol (54mg, 0.28 mmol) and triphenylphosphine (73 mg, 0.28mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain a light yellow solid product (15 mg, 23%).
¹H NMR (400 MHz, CDCl₃) δ 8.60 (d, 1H), 8.26 (d, 1H), 7.62 (d, 1H), 7.50 (d, 1H), 7.45-7.32 (m, 5H), 5.47 (d, 1H), 4.04-4.07 (m, 1H), 3.86-3.90 (m, 1H), 3.51-3.56 (m, 1H), 3.33-3.39 (m, 1H), 3.02-3.13 (m, 1H), 2.39 (s, 3H), 2.22 (s, 3H), 2.00-2.05 (m, 1H), 1.52-1.63 (m, 1H), 1.33-1.43 (m, 1H), 1.05-1.09 (m, 1H).

### Example 32

### 3,5-Dimethyl-4-{7-methylsulfonyl-5-[phenyl(tetrahydro-2H-pyran-4-yl)methyl]-5H-pyrrolo [2,3-b:4,5-b']dipyridin-3-yl}isoxazole

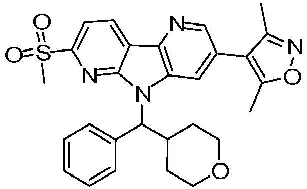

### Step A: 5-Bromo-2-methylthiopyridine

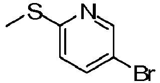

Under the protection of N₂, a 2.4 mol/L n-hexane solution of n-butyllithium (10.5 mL) was added dropwise slowly to a methylbenzene (250 mL) solution of 2,5-dibromopyridine (5.00 g, 0.021 mol) at -78°C, and stirred for 2h. Then dimethyl disulfide (3.8 mL) was added dropwise slowly, and stirred for additional 1h. The resulting mixture was warmed to room temperature, a saturated of ammonium chloride aqueous solution (40 mL) was added, then ethyl acetate (100 mL) was added, and the resulting mixture was stirred for 15min. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated through a silica gel column (ethyl acetate/petroleum ether=1:10-1:2) to obtain a white solid product (3.8 g, 88%).
¹H NMR (400 MHz, CDCl₃,) δ 8.49 (d, J=1.6 Hz, 1H), 7.58 (dd, J=7.6 Hz, J=1.6 Hz, 1H), 7.07 (d, J=7.6 Hz, 1H), 2.56 (s, 3H).

### Step B: 5-Bromo-2-methylsulfonyl pyridine

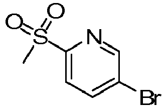

A water (20 mL) solution of potassium peroxymonosulfate (9.12 g, 0.015 mol) was added to a methanol (20 mL) solution of 5-bromo-2-methylthiopyridine (2.00 g, 0.0098 mol), and the resulting mixture reacted at room temperature for 1h, then was cooled and concentrated under reduced pressure to remove methanol. The residual aqueous phase was extracted with ethyl acetate (20 mL) twice. The ethyl acetate phases were combined, washed with a saturated sodium chloride aqueous solution, dried with anhydrous sodium sulfate, and concentrated to obtain a white solid product (2.0 g, 86%).
¹H NMR (400 MHz, CDCl₃) δ 8.79 (dd, 1H), 8.11 (dd, 1H), 7.99 (dd, 1H), 3.22 (s, 3H).

### Step C: 2-Methylsulfonyl-5-(1,3,2-dioxaborolan-2-yl)pyridine

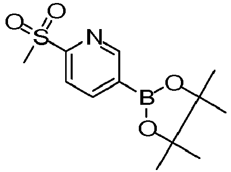

Under the protection of N₂, PdCl₂(dppf) (0.31 g, 0.0004 mol) was added to an anhydrous 1,4-dioxane (50 mL) solution of 5-bromo-2-methylsulfonylpyridine (2.00 g, 0.0085 mol), bis(pinacolato)diboron (2.58 g, 0.010 mol) and anhydrous potassium acetate (1.66 g, 0.017 mol), and the resulting mixture was heated to 100 °C to react overnight. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated through a silica gel column (ethyl acetate/petroleum ether=1:5-1:2) to obtain a white solid product (2.10 g, 88%).
¹H NMR (400 MHz, CDCl₃) δ 9.01 (d, 1H), 8.33 (d, 1H), 8.06 (dd, 1H), 3.24 (s, 3H), 1.37 (s, 12H).

### Step D: 3,5-Dimethyl-4-(6'-methylsulfonyl-3-nitro-[2,3'-bipyridin]-5-yl)isoxazole

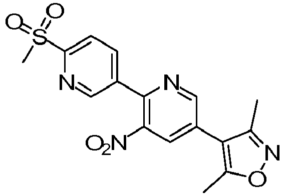

Under the protection of N₂, PdCl₂(dppf) (0.12g, 0.16 mmol) was added to an anhydrous tetrahydrofuran (30 mL) solution of 2-methylsulfonyl-5-(1,3,2-dioxaborolan-2-yl)pyridine (0.79 g, 0.0028 mol), 4-(6-chloro-5-nitropyridin-3-yl)-3,5-dimethylisoxazole (0.67 g, 0.00264 mol, step A in Example 7) and anhydrous potassium phosphate (1.20 g, 0.0056 mol), and the resulting mixture was heated to 70 °C to react overnight. After cooling and concentration under reduced pressure, water and ethyl acetate were added to the residue, stirred for 15 min, and filtered through Celite filler, and the Celite filler was rinsed with ethyl acetate. After the filtrate was layered, the aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated through a silica gel column (ethyl acetate/petroleum ether=1:10-1:2) to obtain a light yellow solid product (0.80 g, 80%).
¹H NMR (400 MHz, CDCl₃), δ 8.85 (dd, J=2.0 Hz, J=0.8 Hz, 1H), 8.81 (d, J=2.0 Hz, 1H), 8.18 (d, J=2.0 Hz, 1H), 8.13 (d, J=0.8 Hz, 1H), 8.11 (d, J=2.0 Hz, 1H), 3.23 (s, 3H), 2.48 (s, 3H), 2.32 (s, 3H).

### Step E: 3,5-Dimethyl-4-(7-methylsulfonyl-5H-pyrrolo[2,3-b:4,5-b']dipyridin-3-yl)isoxazole

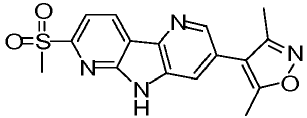

Under the protection of N₂, an o-dichlorobenzene (3 mL) solution of 3,5-dimethyl-4-(6'-methylsulfonyl-3-nitro-[2,3'-bipyridin]-5-yl)isoxazole (270 mg, 0.72 mmol) and 1,2-bis(diphenylphosphino)ethane (575 mg, 1.44 mmol) was heated to 180 °C and reacted under stirring for 1h. The resulting mixture was cooled to room temperature, and then separated through a silica gel column (ethyl acetate/dichloromethane=1:20-1:2) to obtain a light yellow solid product (50 mg, 20%).
¹H NMR (400 MHz, CDCl₃) δ 12.68 (s, 1H), 8.89 (d, J=8.0 Hz, 1H), 8.64 (d, J=1.8 Hz, 1H), 8.00 (d, J=1.8 Hz, 1H), 7.97 (d, J=8.0 Hz, 1H), 3.36 (s, 3H), 2.48 (s, 3H), 2.30 (s, 3H). Step F: 3,5-Dimethyl-4- {7-methylsulfonyl-5-[phenyl(tetrahydro-2H-pyran-4-yl)methyl]-5H-pyrrolo

### [2,3-b:4,5-b']dipyridin-3-yl}isoxazole

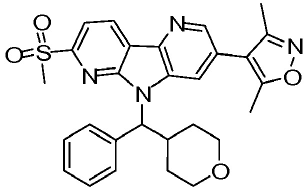

Diisopropyl azodiformate (29 mg, 0.146 mmol) was added dropwise to an anhydrous tetrahydrofuran (2 mL) solution containing 3,5-dimethyl-4-(7- methylsulfonyl-5H-pyrrolo [2,3-b:4,5-b']dipyridin-3-yl)isoxazole (25 mg, 0.073 mmol), phenyl(tetrahydro-2H-pyran-4-yl) methanol (28 mg, 0.146 mmol) and triphenylphosphine (38 mg, 0.146 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (10 mg, 26%).
¹H NMR (400 MHz, CDCl₃) δ 8.84 (d, J=8.0 Hz, 1H), 8.54 (d, J=1.6 Hz, 1H), 8.13 (d, J=8.0 Hz, 1H), 7.53 (d, J=1.6 Hz, 1H), 7.49-7.32 (m, 5H), 5.86 (d, J=11.4 Hz, 1H), 4.01-4.05 (m, 1H), 3.79-3.95 (m, 2H), 3.45-3.54 (m, 1H), 3.38 (s, 3H), 3.33-3.36 (m, 1H), 2.43 (s, 3H), 2.27 (s, 3H), 1.81-1.89 (m, 1H), 1.33-1.60 (m, 2H), 1.16-1.20 (m, 1H).

### Example 33

### Ethyl 5-[4,4-difluorocyclohexyl)(phenyl)methyl]-3-(3,5-dimethylisoxazol-4-yl)-5H-pyrrolo[2,3-b:4,5-b']dipyridine-7-carboxylate

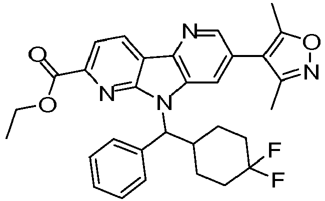

### Step A: 4,4-Difluoro-N-methoxy-N-methylcyclohexane-1-carboxamide

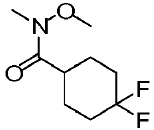

N,O-dimethylhydroxylamine hydrochloride (0.90 g, 9.2 mmol) was added to an anhydrous DMF (10 mL) solution of 4,4-difluorocyclohexanecarboxylic acid (1.00g, 6.1 mmol), diisopropyl ethylamine (1.92 ml, 12.2 mmol) and HATU (2.78 g, 7.3 mmol) at 0°C, and the resulting mixture was warmed to room temperature to react overnight. Water and ethyl acetate were added to the resulting mixture, stirred for 15min, and then layered. The aqueous phase was extracted with ethyl acetate once. The ethyl acetate phases were combined, washed with a saturated sodium chloride aqueous solution twice, dried with anhydrous sodium sulfate, and concentrated to obtain a black residue. The residue was separated through a silica gel column (ethyl acetate/petroleum ether=1:10-1:2) to obtain a white solid product (500 mg, 40%).
¹H NMR(400 MHz, CDCl₃,) δ 3.71 (s, 3H), 3.18 (s, 3H), 2.71-2.77 (m, 1H), 2.14-2.22 (m, 2H), 1.70-1.88 (m, 6H).

### Step B: (4,4-Difluorocyclohexyl)(phenyl)methanone

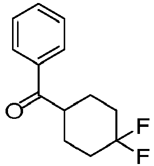

Under the protection of N₂, phenyllithium (1.8 mol/L in dibutyl ether, 4.69 mL, 8.45 mmol) was added to an anhydrous THF (200 mL) solution of 4,4-difluoro-N-methoxy-N-methylcyclohexane-1-carboxamide (500 mg, 2.41 mmol) at -78 °C, and the resulting mixture was kept at -78 °C under stirring for 1h. Then the resulting mixture was warmed up to 0°C, and a saturated ammonium chloride aqueous solution was added to quench the reaction. Water and ethyl acetate were added, and stirred for 15min. The resulting mixture was filtered through Celite, the filtrate was layered, and the aqueous phase was extracted with ethyl acetate once. The ethyl acetate phases were combined, washed with a saturated sodium chloride aqueous solution, dried with anhydrous sodium sulfate, and concentrated to obtain a white solid product (524 mg, 97%).

### Step C: (4,4-Difluorocyclohexyl)(phenyl)methanol

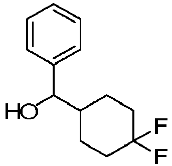

Sodium borohydride (68 mg, 1.75 mmol) was added to a methanol (15 mL) solution of (4,4-difluorocyclohexyl)(phenyl)methanone (261 mg, 1.17 mmol) at 0°C, and stirred for 30min. 1 mL of water was added, methanol was removed under reduced pressure, ethyl acetate and water were added to the residue, and the ethyl acetate phase was separated. The aqueous phase was extracted with ethyl acetate once. The ethyl acetate phases were combined, washed with a saturated sodium chloride aqueous solution, dried with anhydrous sodium sulfate, and concentrated to obtain a white solid product (170 mg, 65%).
¹H NMR(400 MHz, CDCl₃) δ 7.28-7.39 (m, 5H), 4.41 (dd, J=7.6 Hz, J=2.8 Hz, 1H), 1.97-2.18 (m, 3H), 1.84 (d, 1H), 1.58-1.73 (m, 2H), 1.24-1.49 (m, 4H).

### Step D: Ethyl 5-[4,4-difluorocyclohexyl)(phenyl)methyl]-3-(3,5-dimethylisoxazol-4-yl)-5H-pyrrolo [2,3-b:4,5-b']dipyridine-7-carboxylate

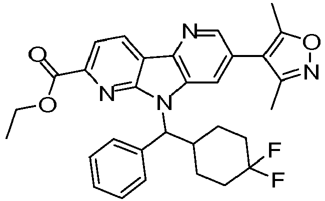

Diisopropyl azodiformate (36 mg, 0.179 mmol) was added dropwise to an anhydrous tetrahydrofuran (2 mL) solution containing ethyl 3-(3,5-dimethylisoxazol-4-yl)-5H-pyrrolo [2,3-b:4,5-b']dipyridine-7-carboxylate (30 mg, 0.089 mmol), (4,4-difluorocyclohexyl)(phenyl) methanol (40 mg, 0.179 mmol) and triphenylphosphine (47 mg, 0.179 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (17 mg, 35%).
¹H NMR (400 MHz, CDCl₃) δ 8.71 (d, J=8.0 Hz, 1H), 8.49 (d, J=1.6 Hz, 1H), 8.18 (d, J=8.0 Hz, 1H), 7.68 (d, J=1.6 Hz, 1H), 7.63-7.66 (m, 2H), 7.26-7.31 (m, 3H), 5.80 (d, J=10.9 Hz, 1H), 4.55 (q, J=7.1 Hz, 2H), 3.50-3.59 (m, 1H), 2.43 (s, 3H), 2.27 (s, 3H), 2.09-2.21 (m, 1H), 1.99-2.08 (m, 1H), 1.88-1.96 (m, 2H), 1.76-1.84 (m, 1H), 1.33-1.55 (m, 6H).

### Example 34

### 2-{5-[(4,4-Difluorocyclohexyl)(phenyl)methyl]-3-(3,5-dimethylisoxazol-4-yl)-5H-pyrrolo [2,3-b:4,5-b']dipyridin-7-yl}propan-2-ol

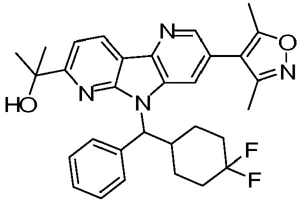

A 3 mol/L diethyl ether solution of methylmagnesium bromide (0.16 mL, 0.72 mmol) was added dropwise slowly to a tetrahydrofuran (2 mL) solution of the compound ethyl 5-[4,4-difluorocyclohexyl)(phenyl)methyl]-3-(3,5-dimethylisoxazol-4-yl)-5H-pyrrolo[2,3-b:4,5-b']dipyridine-7-carboxylate (17 mg, 0.031 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=500:25:1 as the developing solvent, to obtain the target product (7 mg, 42%).
¹H NMR(400 MHz, CDCl₃) δ 8.63 (d, J=8.1 Hz, 1H), 8.44 (d, J=1.6 Hz, 1H), 7.62 (d, J=1.6 Hz, 1H), 7.54 (d, J=7.2 Hz, 2H), 7.48 (d, J=8.1 Hz, 1H), 7.27-7.39 (m, 3H), 5.86 (d, J=10.1 Hz, 1H), 4.50 (br, 1H), 3.22-3.32 (m, 1H), 2.42(s, 3H), 2.26(s, 3H), 2.14-2.24 (m, 1H), 1.96-2.04 (m, 2H), 1.81-1.89 (m, 1H), 1.73 (s, 3H), 1.72 (s, 3H), 1.58-1.68 (m, 2H), 1.31-1.44 (m, 2H).

### Example 35

### Ethyl 5-[(4,4-difluorocyclohexyl)(phenyl)methyl]-3-(3,5-dimethylisoxazol-4-yl)-5H-pyrrolo [3,2-b:4,5-c']dipyridine-7-carboxylate

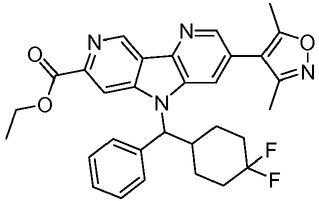

Diisopropyl azodiformate (48.1 mg, 0.238 mmol) was added dropwise to an anhydrous tetrahydrofuran (2 mL) solution containing ethyl 3-(3,5-dimethylisoxazol-4-yl)-5H-pyrrolo [2,3-b:4,5-c']dipyridine-7-carboxylate (40 mg, 0.119 mmol), (4,4-difluorocyclohexyl)(phenyl) methanol (54 mg, 0.238 mmol) and triphenylphosphine (62 mg, 0.238 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (18 mg, 28%).
¹H NMR(400 MHz, CDCl₃) δ 9.72 (s, 1H), 8.56 (br, 2H), 7.64 (s, 1H), 7.30-7.45 (m, 5H), 5.58(d, J=10.8 Hz, 1H), 4.55(q, J=7.1 Hz, 2H), 2.92- 2.98(m, 1H), 2.39 (s, 3H), 2.12-2.22(m, 4H), 1.82-2.02 (m, 3H), 1.51-1.68 (m, 5H), 1.21-1.44 (m, 2H).

### Example 36

### 2-{5-[(4,4-Difluorocyclohexyl)(phenyl)methyl]-3-(3,5-dimethylisoxazol-4-yl)-5H-pyrrolo [3,2-b:4,5-c']dipyridin-7-yl}propan-2-ol

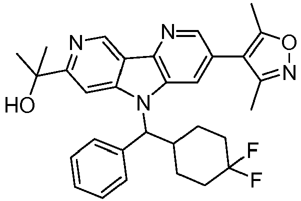

A 3 mol/L diethyl ether solution of methylmagnesium bromide (0.16 mL, 0.72 mmol) was added dropwise slowly to a tetrahydrofuran (2 mL) solution of the compound ethyl 5-[4,4-difluorocyclohexyl)(phenyl)methyl]-3-(3,5-dimethylisoxazol-4-yl)-5H-pyrrolo[3,2-b:4,5-c']dipyridine-7-carboxylate (17 mg, 0.031 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=500:25:1 as the developing solvent, to obtain the target product (10 mg, 60%).
¹H NMR(400 MHz, CDCl₃) δ 9.50 (s, 1H), 8.48 (d, J=1.6 Hz, 1H), 7.64 (s, 1H), 7.54 (d, J=1.6 Hz, 1H), 7.36 (m, 5H), 5.49 (d, J=10.5 Hz, 1H), 4.50 (br, 1H), 2.92-2.98 (m, 1H), 2.38 (s, 3H), 2.18-2.25 (m, 4H), 1.82-2.02 (m, 3H), 1.72 (s, 3H), 1.69 (s, 3H), 1.58-1.68 (m, 2H), 1.31-1.44 (m, 2H).

### Example 37

### 3,5-Dimethyl-4-{5-[phenyl(tetrahydro-2H-pyran-4-yl)methyl]-5H-pyrrolo[3,2-b:5,4-c']dipyridin -3-yl}isoxazole

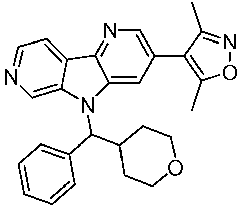

### Step A: 3,5-Dimethyl-4-(3-nitro-[2,4'-bipyridin]-5-yl)isoxazole

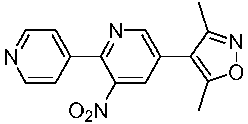

Under the protection of N₂, PdCl₂(dppf) (10 mg, 0.014 mmol) was added to a tetrahydrofuran and water (10:1, 3 mL) solution of 4-(6-chloro-5-nitropyridin-3-yl) -3,5-methylisoxazole (50 mg, 0.20 mmol), pyridin-4-ylboric acid (50 mg, 0.40 mmol) and anhydrous potassium phosphate (85 mg, 0.40 mmol), and the resulting mixture was heated to 70°C to react overnight. After cooling, water and ethyl acetate were added to the resulting mixture, and stirred for 15min. The aqueous phase was extracted with ethyl acetate once. The ethyl acetate phases were combined, washed with a saturated sodium chloride aqueous solution, dried with anhydrous sodium sulfate, and concentrated to obtain a black residue. The residue was separated with a preparative thin layer chromatography plate, using petroleum ether: ethyl acetate=1:1 as the developing solvent, to obtain 3,5-dimethyl-4-(3-nitro-[2,4'-bipyridin]-5-yl)isoxazole (50 mg, 85%).
¹H NMR (400 MHz, CDCl₃) δ 8.85 (d, 1H), 8.79-8.81 (m, 2H), 8.14 (d, 1H), 7.52-7.54 (m, 2H), 2.55 (s, 3H), 2.39 (s, 3H).

### Step B: 3,5-Dimethyl-4-(5H-pyrrolo[3,2-b:5,4-c']dipyridin-3-yl)isoxazole

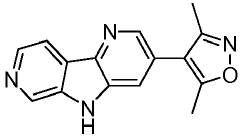

Under the protection of N₂, an o-dichlorobenzene (0.5 mL) solution of 3,5-dimethyl-4-(3-nitro-[2,4'-bipyridin]-5-yl)isoxazole (50 mg, 0.17 mmol) and 1,2-bis(diphenylphosphino)ethane (134 mg, 0.34 mmol) was heated to 180°C and reacted under stirring for 2h. After cooling to room temperature, dichloromethane (10 mL) was added, stirred for 30 min, and filtered. Solid was collected to obtain 3,5-dimethyl-4-(5H-pyrrolo[3,2-b:5,4-c']dipyridin-3-yl)isoxazole (16 mg, 35%).
¹H NMR (400 MHz, CDCl₃) δ 10.30 (s, 1H), 9.06 (s, 1H), 8.60 (d, 1H), 8.57 (d, 1H), 8.28 (d, 1H), 7.74 (d, 1H), 2.48 (s, 3H), 2.33 (s, 3H).

### Step C: 3,5-Dimethyl-4- {5-[phenyl(tetrahydro-2H-pyran-4-yl)methyl]-5H-pyrrolo[3,2-b:5,4-c'] dipyridin-3-yl}isoxazole

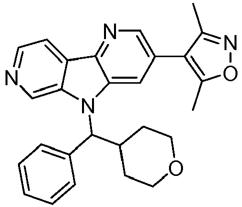

Diisopropyl azodiformate (27 mg, 0.13 mmol) was added dropwise to an anhydrous tetrahydrofuran (1 mL) solution containing 3,5-dimethyl-4-(5H-pyrrolo[3,2-b:5,4-c']dipyridin-3-yl) isoxazole (16 mg, 0.060 mmol), phenyl(tetrahydro-2H-pyran-4-yl)methanol (16 mg, 0.08 mmol) and triphenylphosphine (32 mg, 0.13 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=700:25:1 as the developing solvent, to obtain the target product (5 mg, 19%).
¹H NMR (400 MHz, CD₃OD) δ 9.40 (s, 1H), 8.58 (d, 1H), 8.49 (d, 1H), 8.33-8.36 (m, 2H), 7.64-7.66 (m, 2H), 7.34-7.38 (m, 2H), 7.28-7.31 (m, 1H), 5.90 (d, 1H), 3.97-4.00 (m, 1H), 3.79-3.83 (m, 1H), 3.58-3.64 (m, 1H), 3.33-3.42 (m, 2H), 2.47 (s, 3H), 2.31 (s, 3H), 1.94-2.00 (m, 1H), 1.61-1.68 (m, 1H), 1.38-1.43 (m, 1H), 1.04-1.08 (m, 1H).

### Example 38

### Ethyl 3-(3,5-dimethylisoxazol-4-yl)-5-(heptan-4-yl)-5H-pyrrolo[3,2-b:4,5-c']dipyridine-7-carboxylate

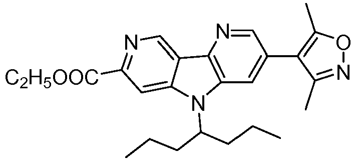

Diisopropyl azodiformate (36 mg, 0.179 mmol) was added dropwise to an anhydrous tetrahydrofuran (2 mL) solution containing ethyl 3-(3,5-dimethylisoxazol-4-yl)-5H-pyrrolo [2,3-b:4,5-c']dipyridine-7-carboxylate (30 mg, 0.089 mmol), 4-heptanol (20 mg, 1.72 mmol) and triphenylphosphine (47 mg, 0.179 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (25 mg, 64%).
¹H NMR (400 MHz, CD₃Cl) δ 9.72 (s, 1H), 8.60 (d, 1H), 8.40 (br, 1H), 7.75 (br, 1H), 4.65 (br, 1H), 4.58 (q, 2H), 2.51 (s, 3H), 2.36 (s, 3H), 2.07-2.15 (m, 2H), 1.92-2.02 (m, 2H), 1.56 (t, 3H), 1.19-1.25 (m, 2H), 1.02-1.17 (m, 2H), 0.85-0.92 (m, 6H).

### Example 39

### 2-[3-(3,5-Dimethylisoxazol-4-yl)-5-(heptan-4-yl)-5H-pyrrolo[3,2-b:4,5-c']dipyridin-7-yl]propan -2-ol

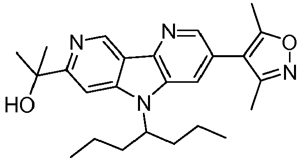

A 3 mol/L diethyl ether solution of methylmagnesium bromide (0.24 mL, 0.72 mmol) was added dropwise slowly to a tetrahydrofuran (5 mL) solution of ethyl 3-(3,5-dimethylisoxazol-4-yl)-5-(heptan-4-yl)-5H-pyrrolo[3,2-b:4,5-c']dipyridine-7-carboxylate (25 mg, 0.058 mmol) at -15°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=500:25:1 as the developing solvent, to obtain the target product (15 mg, 62%).
¹H NMR (400 MHz, CD₃Cl) δ 9.51 (s, 1H), 8.53 (d, 1H), 7.68 (br, 1H), 7.49 (br, 1H), 4.90 (br, 1H), 4.60 (br, 1H), 2.51 (s, 3H), 2.36 (s, 3H), 2.05-2.15 (m, 2H), 1.92-1.99 (m, 2H), 1.61-1.69 (m, 6H), 1.19-1.25 (m, 2H), 1.02-1.17 (m, 2H), 0.85-0.92 (m, 6H).

### Example 40

### Ethyl 3-(3,5-dimethylisoxazol-4-yl)-5-[phenyl(thiazol-4-yl)methyl]-5H-pyrrolo[3,2-b:4,5-c'] dipyridine-7-carboxylate

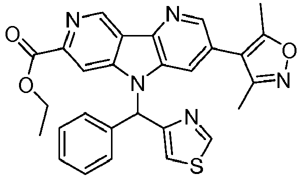

### Step A: Phenyl(thiazol-4-yl)methanol

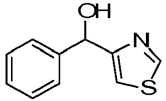

A tetrahydrofuran (10 mL) solution of bromobenzene (1.66 g, 10.57 mmol) was added dropwise slowly to a tetrahydrofuran (30 mL) solution of magnesium metal (0.25 g, 10.41 mmol) at room temperature, and the resulting mixture was slowly heated to 70°C until the magnesium metal was completely reacted. After cooling to 0°C, a tetrahydrofuran (10 mL) solution of thiazole-4-carboxaldehyde (1.00 g, 8.81 mmol) was added dropwise slowly. After the dropwise addition was completed, the resulting mixture was stirred at 0°C for additional 2h, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated by column chromatography through gradient elution (ethyl acetate:petroleum ether=1 :4-1:2) to obtain the target product (1.26 g, 75 %).
¹H NMR (400 MHz, CDCl₃) δ 8.76 (d, 1H), 7.43-7.46 (m, 2H), 7.35-7.39 (m, 2H), 7.29-7.35 (m, 1H), 7.10 (dd, 1H), 5.98 (d, 1H), 3.49 (d, 1H).

### Step B: Ethyl 3-(3,5-dimethylisoxazol-4-yl)-5-[phenyl(thiazol-4-yl)methyl]-5H-pyrrolo[3,2-b:4,5-c'] dipyridine-7-carboxylate

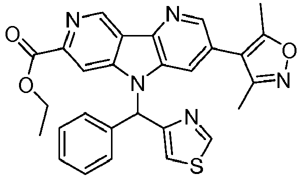

The target product was obtained referring to the synthetic method described in Example 3 with phenyl(thiazol-4-yl)methanol and ethyl 3-(3,5-dimethylisoxazol-4-yl)-5H-pyrrolo [2,3-b:4,5-c']dipyridine-7-carboxylate as starting materials.
¹H NMR (400 MHz, CD₃Cl) δ 9.74 (s, 1H), 8.91 (d, 1H), 8.53 (d, 1H), 8.19 (d, 1H), 7.36-7.42 (m, 5H), 7.29 (s, 1H), 7.15-7.18 (m, 2H), 4.53 (q, 2H), 2.32 (s, 3H), 2.13 (s, 3H), 1.49 (t, 3H).

### Example 41

### 2-{3-(3,5-Dimethylisoxazol-4-yl)-5-[phenyl(thiazol-4-yl)methyl]-5H-pyrrolo[3,2-b:4,5-c']dipyridin-7-yl}propan-2-ol

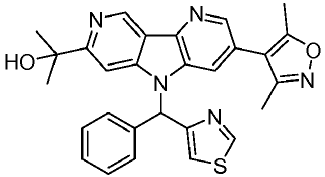

The target product was obtained referring to the synthetic method described in Example 4 with ethyl 3-(3,5-dimethylisoxazol-4-yl)-5-[phenyl(thiazol-4-yl)methyl]-5H-pyrrolo[3,2-b:4,5-c'] dipyridine-7-carboxylate as a starting material.
¹H NMR (400 MHz, CD₃Cl) δ 9.51 (d, 1H), 8.90 (d, 1H), 8.49 (d, 1H), 7.35-7.38 (m, 3H), 7.31 (s, 1H), 7.24-7.27 (m, 2H), 7.16-7.18 (m, 3H), 4.80 (br, 1H), 2.31 (s, 3H), 2.13 (s, 3H), 1.59 (s, 3H), 1.58(s, 3H).

### Example 42

### 4-{7-Methoxy-5-[phenyl(tetrahydro-2H-pyran-4-yl)methyl]-5H-pyrrolo[2,3-b:4,5-b']dipyridin-3-yl}-3,5-dimethylisoxazole

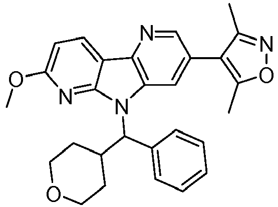

### Step A: 4-(6'-Methoxy-3-nitro-[2,3'-bipyridin]-5-yl)-3,5-dimethylisoxazole

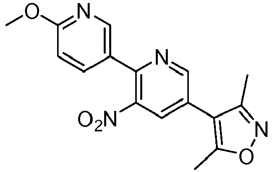

Under the protection of N₂, PdCl₂(dppf) (43 mg, 0.059 mmol) was added to a tetrahydrofuran and water (10:1, 20 mL) solution of 4-(6-chloro-5-nitropyridin-3-yl)-3,5-methylisoxazole (0.30 g, 1.18 mmol), (6-methoxypyridin-3-yl)boric acid (0.36 g, 2.35 mmol) and anhydrous potassium phosphate (1.00 g, 4.71 mmol), and the resulting mixture was heated to 70°C to react overnight. After cooling, water and ethyl acetate were added to the resulting mixture, and stirred for 15min. The aqueous phase was extracted with ethyl acetate once. The ethyl acetate phases were combined, washed with a saturated sodium chloride aqueous solution, dried with anhydrous sodium sulfate, and concentrated to obtain a black residue. The residue was purified by silica gel column chromatography eluting with ethyl acetate/petroleum ether (4:1) to obtain a brown solid product (0.30 g, 78%).
¹H NMR (400 MHz, CDCl₃) δ 9.78 (d, 1H), 8.45 (d, 1H), 8.05 (d, 1H), 7.81 (dd, 1H), 6.86 (dd, 1H), 4.02 (s, 3H), 2.53 (s, 3H), 2.36 (s, 3H).

### Step B: 4-(7-Methoxy-5H-pyrrolo[2,3-b:4,5-b']dipyridin-3-yl)-3,5-dimethylisoxazole

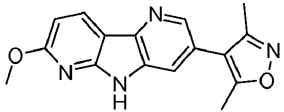

Under the protection of N₂, an o-dichlorobenzene (5 mL) solution of 4-(6'-methoxy-3-nitro-[2,3'-bipyridin]-5-yl)-3,5-dimethylisoxazole (0.30 g, 0.92 mmol) and 1,2-bis(diphenylphosphino)ethane (0.73 g, 1.84 mmol) was heated to 180°C and reacted under stirring for 1h. After cooling to room temperature, dichloromethane (30 mL) was added, stirred for 30 min, and filtered. Solid was collected to obtain 4-(7-methoxy-5H-pyrrolo[2,3-b:4,5-b']dipyridin -3-yl)-3,5-dimethylisoxazole (180 mg, 66%).
¹H NMR (400 MHz, DMSO-d6) δ 12.02 (br, 1H), 8.40-8.43 (m, 2H), 7.75 (d, 1H), 6.73 (d, 1H), 3.97 (s, 3H), 2.44 (s, 3H), 2.25 (s, 3H).

### Step C: 4- {7-Methoxy-5-[phenyl(tetrahydro-2H-pyran-4-yl)methyl]-5H-pyrrolo[2,3-b:4,5-b']dipyridin -3-yl}-3,5-dimethylisoxazole

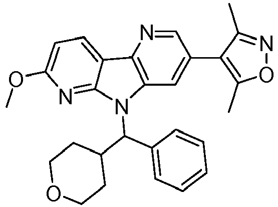

Diisopropyl azodiformate (27 mg, 0.13 mmol) was added dropwise to an anhydrous tetrahydrofuran (1 mL) solution containing 4-(7-methoxy-5H-pyrrolo[2,3-b: 4,5-b']dipyridin-3-yl) -3,5-dimethylisoxazole (20 mg, 0.068 mmol), phenyl(tetrahydro-2H-pyran-4-yl)methanol (26 mg, 0.14 mmol) and triphenylphosphine (32 mg, 0.13 mmol) at 0°C. After the addition was completed, the resulting mixture was slowly warmed to room temperature to react for 2h. After the reaction was completed, the reaction mixture was directly separated with a preparative thin layer chromatography plate, using dichloromethane:methanol:ammonia water=700:25:1 as the developing solvent, to obtain the target product (20 mg, 63%).
¹H NMR (400 MHz, CD₃Cl) δ 9.40 (s, 1H), 8.47 (d, 1H), 8.37 (d, 1H), 7.58-7.60 (m, 2H), 7.26-7.34 (m, 3H), 6.78 (d, 1H), 5.73 (d, 1H), 4.17 (s, 3H), 4.01-4.05 (m, 1H), 3.88-3.92 (m, 1H), 3.35-3.53 (m, 3H), 2.40 (s, 3H), 2.25 (s, 3H), 1.77-1.81 (m, 1H), 1.39-1.58 (m, 2H), 1.20-1.29 (m, 1H).

### Example 43

### 3-(3,5-Dimethylisoxazol-4-yl)-5-[phenyl(tetrahydro-2H-pyran-4-yl)methyl]-5H-pyrrolo [2,3-b:4,5-b']dipyridine-7-ol

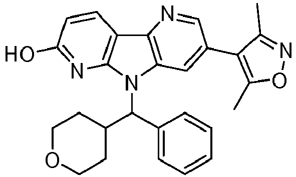

A 30% acetic acid solution of hydrogen bromide (0.2 mL) was added dropwise to an ethanol (1 mL) solution of 4-{7-methoxy-5-[phenyl(tetrahydro-2H-pyran-4-yl)methyl]-5H-pyrrolo [2,3-b:4,5-b']dipyridin-3-yl}-3,5-dimethylisoxazole (25 mg, 0.053 mmol). After the addition was completed, the resulting mixture was heated to 90°C to react for 7h. After the reaction was completed, the solvent was removed under reduced pressure, a 5% sodium bicarbonate aqueous solution was added, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using ethyl acetate:petroleum ether=1:1 as the developing solvent, to obtain the target product (20 mg, 82%).
¹H NMR (400 MHz, CDCl₃) δ 10.22 (br, 1H), 8.58 (br, 1H), 8.40 (s, 1H), 7.46-7.60 (m, 3H),7.22-7.38 (m, 3H), 6.76 (d, 1H), 5.92 (d, 1H), 3.98-4.03 (m, 1H),3.80-3.88 (m, 1H), 3.40-3.53 (m, 1H), 3.24-3.34 (m, 1H), 3.18-3.20 (m, 1H), 2.36 (s, 3H), 2.18 (s, 3H), 1.79-1.84 (m, 1H), 1.40-1.66 (m, 2H), 1.04-1.12 (m, 1H).

### Example 44

### 4-(5-((2-Fluorophenyl)(tetrahydro-2H-pyran-4-yl)methyl)-7-methoxy-5H-pyrrolo[2,3-b:4,5-b'] dipyridin-3-yl)-3,5-dimethylisoxazole

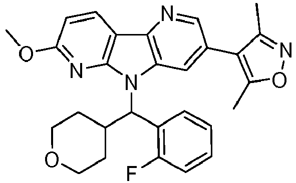

### Step A: N-methoxy-N-methyltetrahydro-2H-pyran-4-carboxamide

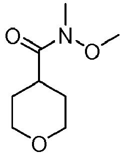

Tetrahydro-2H-pyran-4-carboxylic acid (13 g, 100 mmol) was dissolved in dichloromethane (200 mL), and cooled in an ice water bath. N,N-dimethyl formamide (0.1 mL) was added, and then oxalyl chloride (15.2 g, 120 mmol) was added dropwise slowly. After the dropwise addition was completed, the resulting mixture was stirred at 0°C for additional 1h. The solvent was removed under vacuum to obtain an oily substance, which was dissolved in dichloromethane (100 mL). The resulting solution was added to a mixed solution of N,N-dimethylhydroxylamine hydrochloride (9.75 g, 100 mmol), diisopropyl ethylamine (39 g, 300 mmol) and dichloromethane (200 mL). The reaction mixture was stirred at room temperature overnight. The reaction mixture was poured into water, and extracted with ethyl acetate. The organic phase was washed with a saturated sodium chloride aqueous solution, and dried with anhydrous sodium sulfate. The solvent was removed under vacuum to obtain the product (15 g, 87%).
¹H NMR (400 MHz, CDCl₃): δ 4.00-4.05 (m, 2H), 3.72 (s, 3H), 3.44-3.50 (m, 2H), 3.20 (s, 3H), 2.89-2.95 (m, 1H), 1.82-1.92 (m, 2H), 1.64-1.68 (m, 2H).

### Step B: (2-Fluorophenyl)(tetrahydro-2H-pyran-4-yl)methanone

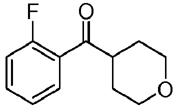

1-Bromo-2-fluorobenzene (3.5 g, 20 mmol) was dissolved in dry tetrahydrofuran (40 mL), and cooled to -78°C under the protection of nitrogen. A 2.4 mol/L n-hexane solution of n-BuLi (10 mL, 24 mmol) was added dropwise slowly. The reaction mixture was stirred at - 78°C for additional 30 min, and then a tetrahydrofuran (10 mL) solution of N-methoxy-N-methyltetrahydro-2H-pyran-4-carboxamide (4.16 g, 24 mmol) was added dropwise. The reaction mixture was slowly warmed to room temperature, and stirred overnight. An ammonium chloride aqueous solution was added to quench the reaction. The reaction mixture was poured into water, and extracted with ethyl acetate. The organic phase was separated, washed with a saturated saline solution, and dried. The solvent was removed under vacuum to obtain a crude product. The crude product was purified by column chromatography to obtain the product (2 g, 48%).
¹H NMR (400 MHz, CDCl₃): δ 7.76-7.80 (m, 1H), 7.49-7.54 (m, 1H), 7.22-7.27 (m, 1H), 7.10-7.15 (m, 1H), 4.00-4.05 (m, 2H), 3.49-3.55 (m, 2H), 3.32-3.40 (m, 1H), 1.73-1.86 (m, 4H).

### Step C: (2-Fluorophenyl)(tetrahydro-2H-pyran-4-yl)methanol

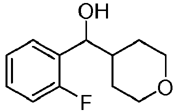

(2-Fluorophenyl)(tetrahydro-2H-pyran-4-yl)methanone (2 g, 9.6 mmol) was dissolved in methanol, and the resulting mixture was cooled in an ice water bath, to which sodium borohydride (0.36 g, 9.6 mmol) was added in batches until the reaction was completed. The reaction mixture was poured into water, and extracted with ethyl acetate. The extract was washed with a saturated saline solution, and dried. The solvent was removed under vacuum to obtain the product (2 g, 99%).

### Step D: 4-(5-((2-Fluorophenyl)(tetrahydro-2H-pyran-4-yl)methyl)-7-methoxy-5H-pyrrolo [2,3-b:4,5-b']dipyridin-3-yl)-3,5-dimethylisoxazole

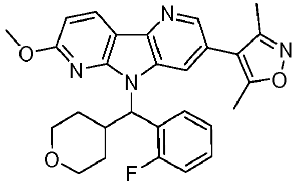

Diisopropyl azodiformate (28 mg, 0.136 mmol) was added dropwise to an anhydrous tetrahydrofuran (1 mL) solution containing 4-(7-methoxy-5H-pyrrolo[2,3-b: 4,5-b']dipyridin-3-yl) -3,5-dimethylisoxazole (20 mg, 0.068 mmol), 2-fluorophenyl(tetrahydro-2H-pyran-4-yl)methanol (14.3 mg, 0.068 mmol) and triphenylphosphine (36 mg, 0.136 mmol) at 0°C. After the addition was completed, the resulting mixture was slowly warmed to 30°C to react for 2h. After the reaction was completed, the reaction mixture was directly separated with a preparative thin layer chromatography plate, using ethyl acetate:petroleum ether=1:1 as the developing solvent, to obtain the target product (18 mg, 54%).
¹H NMR (400 MHz, CDCl₃) δ 8.48 (d, 1H), 8.45 (s, 1H), 8.04-8.08 (m, 1H), 7.83 (d, 1H), 7.24-7.27 (m, 1H), 7.11-7.15 (m, 1H), 7.01-7.06 (m, 1H), 6.78 (d, 1H), 5.79 (d, 1H), 4.17 (s, 3H), 4.00-4.05 (m, 1H), 3.84-3.94 (m, 1H), 3.60-3.78 (m, 1H), 3.32-3.45 (m, 2H), 2.48 (s, 3H), 2.33 (s, 3H), 1.59-1.64 (m, 1H), 1.47-1.58 (m, 1H), 1.21-1.38 (m, 2H).

### Example 45

### 4-{5-[(3-Fluoropyridin-2-yl)(tetrahydro-2H-pyran-4-yl)methyl)-7-methoxy-5H-pyrrolo[2,3-b:4,5-b']dipyridin-3-yl}-3,5-dimethylisoxazole

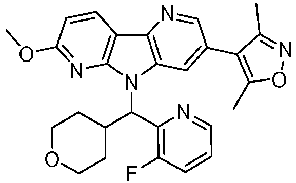

### Step A: (3-Fluoropyridin-2-yl)(tetrahydro-2H-pyran-4-yl)methanone

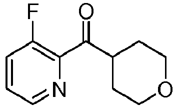

3-Fluoropyridine (1.94 g, 20 mmol) was dissolved in dry tetrahydrofuran (40 mL), and cooled to -78°C under the protection of nitrogen. A 2.4 mol/L n-hexane solution of n-BuLi (10 mL, 24 mmol) was added dropwise slowly. The reaction mixture was stirred at -78°C for additional 30 min, and then a tetrahydrofuran (10 mL) solution of N-methoxy-N-methyltetrahydro-2H-pyran-4- carboxamide (4.16 g, 24 mmol) was added dropwise. The reaction mixture was slowly warmed to room temperature, and stirred overnight. An ammonium chloride aqueous solution was added to quench the reaction. The reaction mixture was poured into water, and extracted with ethyl acetate. The extract was washed with a saturated saline solution, and dried. The solvent was removed under vacuum to obtain a crude product. The crude product was purified by column chromatography to obtain the product (1.5 g, 36%).
¹H NMR (400 MHz, CDCl₃): δ 8.61 (d, J=2.0 Hz, 1H), 8.55-8.57 (m, 1H), 7.56-7.59 (m, 1H), 4.00-4.04 (m, 2H), 3.48-3.54 (m, 2H), 3.27-3.34 (m, 1H), 1.73-1.86 (m, 4H).

### Step B: (3-Fluoropyridin-2-yl)(tetrahydro-2H-pyran-4-yl)methanol

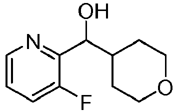

(3-Fluoropyridin-2-yl)(tetrahydro-2H-pyran-4-yl)methanone (1.5 g, 7.2 mmol) was dissolved in methanol (10 mL), and the resulting mixture was cooled in an ice water bath, to which sodium borohydride (0.27 g, 7.2 mmol) was added until the reaction was completed. The reaction mixture was poured into water, and extracted with ethyl acetate. The extract was washed with a saturated saline solution, and dried. The solvent was removed under vacuum to obtain the product (1.5 g, 99%).
¹H NMR (400 MHz, CDCl₃) δ 8.40-8.42 (m, 2H), 7.42-7.45 (m, 1H), 4.84 (d, J=6.4 Hz, 1H), 3.93-4.03 (m, 2H), 3.29-3.37 (m, 2H), 2.40 (br, 1H), 1.85-1.93 (m, 1H), 1.69-1.74 (m, 1H), 1.48-1.54 (m, 2H), 1.26-1.33 (m, 1H).

### Step C: 4-{5-[(3-Fluoropyridin-2-yl)(tetrahydro-2H-pyran-4-yl)methyl]-7-methoxy-5H-pyrrolo [2,3-b:4,5-b']dipyridin-3-yl}-3,5-dimethylisoxazole

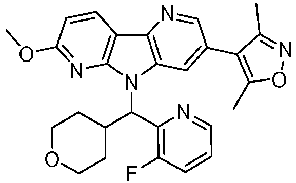

Diisopropyl azodiformate (28 mg, 0.136 mmol) was added dropwise to anhydrous tetrahydrofuran (1 mL) containing 4-(7-methoxy-5H-pyrrolo[2,3-b:4,5-b']dipyridin-3-yl)-3,5-dimethylisoxazole (20 mg, 0.068 mmol), 3-fluoropyridine(tetrahydro-2H-pyran-4-yl)methanol (14.3 mg, 0.068 mmol) and triphenylphosphine (36 mg, 0.136 mmol) at 0°C. After the addition was completed, the resulting mixture was slowly warmed to 30°C to react for 2h. After the reaction was completed, the reaction mixture was directly separated with a preparative thin layer chromatography plate, using ethyl acetate:petroleum ether=1:1 as the developing solvent, to obtain the target product (6 mg, 18%).
¹H NMR (400 MHz, CDCl³) δ 8.40-8.54 (m, 4H), 8.03 (br, 1H), 7.73 (s, 1H), 6.81 (d, 1H), 5.76 (d, 1H), 4.16 (s, 3H), 4.01-4.06 (m, 1H), 3.88-3.96 (m, 1H), 3.61-3.78 (m, 1H), 3.32-3.51 (m, 2H), 2.48 (s, 3H), 2.34 (s, 3H), 1.42-1.58 (m, 2H), 1.20-1.38 (m, 2H).

### Example 46

### Ethyl 5-(diphenylmethyl)-3-(3,5-dimethylisoxazol-4-yl)-5H-pyrrolo[2,3-b:4,5-c']dipyridine-7-carboxylate

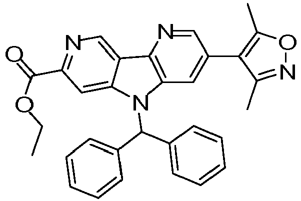

Diisopropyl azodiformate (48 mg, 0.24 mmol) was added dropwise to an anhydrous tetrahydrofuran (2 mL) solution containing ethyl 3-(3,5-dimethylisoxazol-4-yl)-5H-pyrrolo [2,3-b:4,5-c']dipyridine-7-carboxylate (40 mg, 0.12 mmol), phenyl(tetrahydro-2H-pyran-4-yl) methanol (43 mg, 0.24 mmol) and triphenylphosphine (62 mg, 0.24 mmol) at 0°C. After the addition was completed, the resulting mixture was slowly warmed to 30°C to react for 2h. After the reaction was completed, the reaction mixture was directly separated with a preparative thin layer chromatography plate, using dichloromethane:methanol:ammonia water=500:25:1 as the developing solvent, to obtain the target product (5 mg, 8%).
¹H NMR (400 MHz, CD₃OD) δ 9.49 (s, 1H), 8.53 (d, 1H), 7.87 (s, 1H), 7.57 (s, 1H), 7.44 (d, 1H), 7.31-7.35 (m, 6H), 7.17-7.19 (m, 4H), 4.32 (q, J=7.10 Hz, 2H), 2.21(s, 3H), 2.02 (s, 3H), 1.30 (t, J=7.10 Hz, 3H).

### Example 47

### 2-{5-(Diphenylmethyl)-3-(3,5-dimethylisoxazol-4-yl)-5H-pyrrolo[3,2-b:4,5-c']dipyridin-7-yl} propan-2-ol

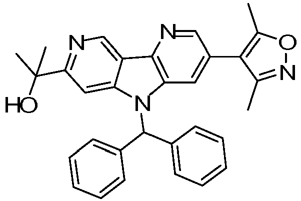

A 3 mol/L diethyl ether solution of methylmagnesium bromide (0.05 mL, 0.15 mmol) was added dropwise slowly to a tetrahydrofuran (2 mL) solution of ethyl 5-(diphenylmethyl)-3- (3,5-dimethylisoxazol-4-yl)-5H-pyrrolo[2,3-b:4,5-c']dipyridine-7-carboxylate (5 mg, 0.01 mmol) at -20°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=500:25:1 as the developing solvent, to obtain the target product (3.8 mg, 78%).
¹H NMR (400 MHz, CD₃OD) δ 9.37 (s, 1H), 8.44 (d, 1H), 7.54 (s, 1H), 7.46 (d, 1H), 7.30-7.32 (m, 6H), 7.25 (s, 1H), 7.16-7.19 (m, 4H), 2.19 (s, 3H), 1.99 (s, 3H), 1.46 (s, 6H).

### Example 48

### Ethyl 3 -(3,5 -dimethylisoxazol-4-yl)-5 - [(2-fluorophenyl)(tetrahydro-2H-pyran-4-yl)methyl] -5H-pyrrolo[2,3-b:4,5-c']dipyridine-7-carboxylate

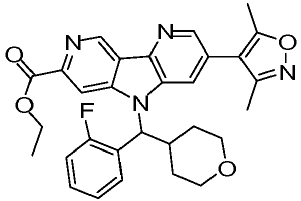

Diisopropyl azodiformate (48 mg, 0.24 mmol) was added dropwise to an anhydrous tetrahydrofuran (2 mL) solution containing ethyl 3-(3,5-dimethylisoxazol-4-yl)-5H-pyrrolo [2,3-b:4,5-c']dipyridine-7-carboxylate (40 mg, 0.12mmol), phenyl(tetrahydro-2H-pyran-4-yl) methanol (43 mg, 0.24 mmol) and triphenylphosphine (62 mg, 0.24 mmol) at 0°C. After the addition was completed, the resulting mixture was slowly heated to 50°C to react for 12h. After the reaction was completed, the reaction mixture was directly separated with a preparative thin layer chromatography plate, using dichloromethane:methanol:ammonia water=500:25:1 as the developing solvent, to obtain the target product (6 mg, 9.4%).
¹H NMR (400 MHz, CD₃OD) δ 9.53 (s, 1H), 8.60 (d, 1H), 8.48 (s, 1H), 8.43 (d, 1H), 8.20 (d, 1H), 7.82-7.86 (m, 1H), 7.64-7.68 (m, 1H), 7.32-7.36 (m, 1H), 6.25 (d, J=11.4Hz, 1H), 4.52 (q, J=7.10Hz, 2H), 3.72-4.03 (m, 3H), 3.36-3.43 (m, 2H), 2.45 (s, 3H), 2.30 (s, 3H), 1.62-1.95 (m, 2H), 1.42-1.52 (m, 4H), 1.20-1.38 (m,1H).

### Example 49

### 2-{3-(3,5-Dimethylisoxazol-4-yl)-5-[2-fluorophenyl(tetrahydro-2H-pyran-4-yl)methyl]-5H-pyrrolo[3,2-b:4,5-c']dipyridin-7-yl}propan-2-ol

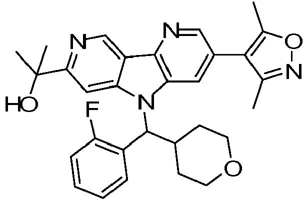

A 3 mol/L diethyl ether solution of methylmagnesium bromide (0.056 mL, 0.72 mmol) was added dropwise slowly to a tetrahydrofuran (2 mL) solution of ethyl 3-(3,5-dimethylisoxazol -4-yl)-5-[2-fluorophenyl(tetrahydro-2H-pyran-4-yl)methyl]-5H-pyrrolo[2,3-b:4,5-c']dipyridine-7- carboxylate (6 mg, 0.011 mmol) at -20°C. After the addition was completed, the resulting mixture was slowly warmed to room temperature to react for 2h. After the reaction was completed, a saturated ammonium chloride aqueous solution was added to quench the reaction, and the resulting mixture was extracted with ethyl acetate. The ethyl acetate phases were combined, washed with a saturated sodium chloride aqueous solution, dried with anhydrous sodium sulfate, and concentrated. The residue was separated with a preparative thin layer chromatography plate, using dichloromethane:methanol:ammonia water=500:25:1 as the developing solvent, to obtain the target product (1.73 mg, 30%).
¹H NMR (400 MHz, CD₃OD) δ 9.41 (s, 1H), 8.53 (d, 1H), 8.28 (s, 1H), 8.10-8.14 (m, 1H), 8.20 (d, 1H), 7.35-7.38 (m, 2H), 7.06-7.11 (m, 1H), 6.04 (d, J=11.4Hz, 1H), 3.98-4.05 (m, 1H), 3.79-3.83 (m, 1H), 3.57-3.64 (m, 1H), 3.36-3.42 (m, 2H), 2.47 (s, 3H), 2.31 (s, 3H), 1.92-1.97 (m,1H) 1.58-1.65 (m, 7H), 1.42-1.52 (m, 1H), 1.20-1.38 (m,1H).

### Example 50

### 3-(3,5-Dimethylisoxazol-4-yl)-5-[phenyl(tetrahydro-2H-pyran-4-yl)methyl]-5H-pyrrolo [2,3-b:4,5-b']dipyridine-7-carboxamide

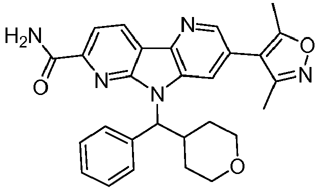

Ethyl 3-(3,5-dimethylisoxazol-4-yl)-5-[phenyl(tetrahydro-2H-pyran-4-yl)methyl]-5H- pyrrolo[2,3-b:4,5-b']dipyridine-7-carboxylate (20 mg, 0.040 mmol, Example 5) was dissolved in a methanol solution (2 mL) of ammonia gas, and the resulting mixture was kept in a sealed tube at 80°C to react for 48h. The solvent was removed by vacuum concentration to obtain a white solid (18 mg, 95%).
1H NMR (400 MHz, CDCl3,) δ 8.77 (d, J=8.0 Hz, 1H), 8.49 (d, J=1.6 Hz, 1H), 8.31 (d, J=8.0 Hz, 1H), 7.84 (br, 1H), 7.63 (d, J=1.6 Hz, 1H), 7.52 (d, J=8.0 Hz, 2H), 7.29-7.31 (m, 3H), 6.01 (d, J=10.4 Hz, 1H), 5.75 (br, 1H), 4.03-4.07 (m, 1H), 3.86-3.89 (m, 1H), 3.52 (t, J=12.0 Hz, 1H), 3.35-3.38 (m, 2H), 2.40 (s, 3H), 2.24 (s, 3H), 1.91-1.94 (m, 1H), 1.58-1.62 (m, 1H), 1.41-1.45 (m, 1H), 1.17-1.32 (m, 1H).

### Example 51

### 3-(3,5-Dimethylisoxazol-4-yl)-5-[phenyl(tetrahydro-2H-pyran-4-yl)methyl]-5H-pyrrolo [2,3-b:4,5-b']dipyridine-7-carboxylic acid

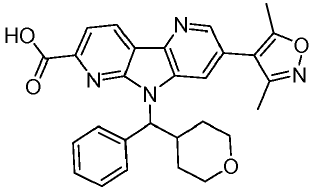

Ethyl 3-(3,5-dimethylisoxazol-4-yl)-5-[phenyl(tetrahydro-2H-pyran-4-yl)methyl]-5H- pyrrolo[2,3-b:4,5-b']dipyridine-7-carboxylate (20 mg, 0.040 mmol) was dissolved in a methanol solution (2 mL) of ammonia gas, and the resulting mixture was kept in a sealed tube at 80°C to react for 48h. The solvent was removed by vacuum concentration to obtain a white solid (18 mg, 95%).
¹H NMR (400 MHz, CDCl₃,) δ 8.86 (d, J=8.0 Hz, 1H), 8.55 (d, J=2.0 Hz, 1H), 8.32 (d, J=8.0 Hz, 1H), 7.70 (d, J=2.0 Hz, 1H), 7.52 (d, J=7.2 Hz, 2H), 7.31-7.38 (m, 3H), 6.01 (d, J=10.4 Hz, 1H), 4.04-4.10 (m, 1H), 3.87-3.90 (m, 1H), 3.51-3.57 (m, 1H), 3.25-3.40 (m, 2H), 2.42 (s, 3H), 2.26 (s, 3H), 1.93-1.96 (m, 1H), 1.54-1.65 (m, 1H), 1.39-1.49 (m, 1H), 1.12-1.19 (m, 1H).

### Example 52

### 3-(3,5-Dimethylisoxazol-4-yl)-5-[phenyl(tetrahydro-2H-pyran-4-yl)methyl]-5H-pyrrolo[2,3-b:4,5-b']dipyridine-7-amine

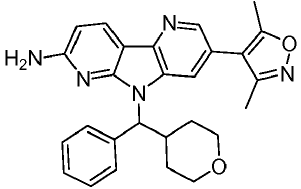

3-(3,5-Dimethylisoxazol-4-yl)-5-[phenyl(tetrahydro-2H-pyran-4-yl)methyl]-5H-pyrrolo[2,3-b:4,5-b']dipyridine-7-carboxylic acid (18 mg, 0.037 mmol) was dispersed in anhydrous methylbenzene (2 mL), and triethylamine (12 mg, 0.11 mmol) and DPPA (20 mg, 0.074 mmol) were added, and the resulting mixture was stirred at room temperature for 4h. Water (1 mL) was added, and heated at 60°C for 5h. The solvent was removed by vacuum concentration, and then dichlorometahne (10 mL) was added. The resulting mixture was washed with water, and then washed with a saturated saline solution. The solvent was removed by vacuum concentration. The residue was separated with a silica gel prefabricated plate (ethyl acetate/dichloromethane=2:1) to obtain a light yellow solid (10 mg, 60%).
¹H NMR (400 MHz, CDCl₃,) δ 8.30 (d, J=8.4 Hz, 1H), 8.28 (d, J=2.0 Hz, 1H), 7.55 (d, J=7.2 Hz, 2H), 7.45 (d, J=2.0 Hz, 1H), 7.26-7.33 (m, 3H), 6.51 (d, J=8.4 Hz, 1H), 5.85 (d, J=10.4 Hz, 1H), 4.81 (s, 2H), 3.99-4.03 (m, 1H), 3.85-3.89 (m, 1H), 3.47-3.53 (m, 1H), 3.23-3.38 (m, 2H), 2.37 (s, 3H), 2.21 (s, 3H), 1.82-1.85 (m, 1H), 1.52-1.59 (m, 1H), 1.40-1.44 (m, 1H), 1.13-1.17 (m, 1H).

### Example 53

### N-{3-(3,5-dimethylisoxazol-4-yl)-5-[phenyl(tetrahydro-2H-pyran-4-yl)methyl]-5H-pyrrolo[2,3-b:4,5-b']dipyridin-7-yl}-N-(methylsulfonyl)methylsulfamide

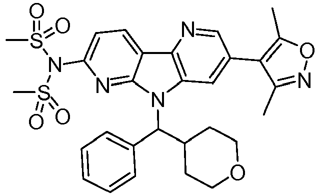

3-(3,5-Dimethylisoxazol-4-yl)-5-[phenyl(tetrahydro-2H-pyran-4-yl)methyl]-5H-pyrrolo[2,3-b:4,5-b']dipyridine-7-amine (8 mg, 0.018 mmol) was dissolved in anhydrous dichloromethane (1 mL), and triethylamine (6 mg, 0.059 mmol) and methylsulfonyl chloride (5 mg, 0.044 mmol) were added. The resulting mixture was stirred at room temperature for 2h, washed with water, and then washed with a saturated saline solution. The solvent was removed by vacuum concentration. The residue was separated with a silica gel prefabricated plate (ethyl acetate/dichloromethane=1:1) to obtain a white solid (10 mg, 92%).
¹H NMR (400 MHz, CDCl₃,) δ 8.76 (d, J=8.0 Hz, 1H), 8.51 (d, J=1.6 Hz, 1H), 7.69 (d, J=1.6 Hz, 1H), 7.58 (d, J=7.2 Hz, 2H), 7.40 (d, J=8.0 Hz, 1H), 7.28-7.41 (m, 3H), 5.72 (d, J=10.4 Hz, 1H), 4.01-4.04 (m, 1H), 3.86-3.90 (m, 1H), 3.65 (s, 6H), 3.34-3.52 (m, 3H), 2.44 (s, 3H), 2.28 (s, 3H), 1.80-1.83 (m, 1H), 1.45-1.55 (m, 1H), 1.31-1.41 (m, 1H), 1.19-1.23 (m, 1H).

### Example 54

### N-{3-(3,5-dimethylisoxazol-4-yl)-5-[phenyl(tetrahydro-2H-pyran-4-yl)methyl]-5H-pyrrolo[2,3-b:4,5-b']dipyridin-7-yl}methylsulfamide

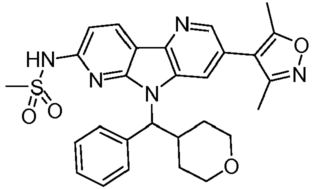

N-{3-(3,5-dimethylisoxazol-4-yl)-5-[phenyl(tetrahydro-2H-pyran-4-yl)methyl]-5H-pyrrolo[2,3-b:4,5-b']dipyridin-7-yl}-N-(methylsulfonyl)methylsulfamide (8 mg, 0.013 mmol) was dispersed in a mixed solution of tetrahydrofuran/4N potassium hydroxide aqueous solution (1 mL/lmL), and the resulting mixture was stirred at room temperature for 3h. 6N hydrochloric acid was added dropwise to adjust the pH to about 6, and the resulting mixture was extracted with dichloromethane. The extract was washed with water, and then washed with a saturated saline solution. The solvent was removed by vacuum concentration. The residue was separated with a silica gel prefabricated plate (ethyl acetate/dichloromethane=1:1) to obtain a white solid (5 mg, 71%).
¹H NMR (400 MHz, CDCl₃,) δ 8.60 (s, 1H), 8.43 (s, 1H), 7.63 (s, 1H), 7.56 (d, J=7.2 Hz, 2H), 7.41 (s, 1H), 7.28-7.36 (m, 3H), 7.07 (d, J=8.4 Hz, 1H), 5.78-5.81 (m, 1H), 4.02-4.05 (m, 1H), 3.87-3.90 (m, 1H), 3.48-3.54 (m, 1H), 3.45 (s, 3H), 3.32-3.42 (m, 2H), 2.41 (s, 3H), 2.25 (s, 3H), 1.82-1.86 (m, 1H), 1.55-1.66 (m, 1H), 1.43-1.53 (m, 1H), 1.18-1.21 (m, 1H).

### Example 55

### 3-(3,5-Dimethylisoxazol-4-yl)-N-methoxy-N-methyl-5-[phenyl(tetrahydro-2H-pyran-4-yl)methyl]-5H-pyrrolo[2,3-b:4,5-b']dipyridine-7-carboxamide

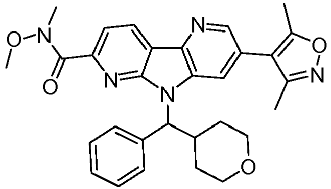

3-(3,5-Dimethylisoxazol-4-yl)-5-[phenyl(tetrahydro-2H-pyran-4-yl)methyl]-5H-pyrrolo[2,3-b:4,5-b']dipyridine-7-carboxylic acid (100 mg, 0.21 mmol) was dissolved in anhydrous DMF (1 mL), and dimethylhydroxylamine hydrochloride (52 mg, 0.53 mmol), DIPEA (82 mg, 0.63 mmol) and HATU (158 mg, 0.42 mmol) were added. The resulting mixture was stirred at room temperature for 2h. Then dichloromethane (10 mL) was added, and the resulting mixture was washed with water three times, and then washed with a saturated saline solution. The solvent was removed by vacuum concentration. The residue was separated with a silica gel prefabricated plate (ethyl acetate/dichloromethane=1:1) to obtain a white solid (89 mg, 81%).
¹H NMR (400 MHz, CDCl₃,) δ 8.70 (d, J=8.0 Hz, 1H), 8.46 (d, J=2.0 Hz, 1H), 7.70 (br, 1H), 7.62 (d, J=2.0 Hz,1H), 7.54 (d, J=7.2 Hz, 2H), 7.28-7.34 (m, 3H), 6.09 (br, 1H), 4.00-4.04 (m, 1H), 3.83-3.86 (m, 4H), 3.48-3.53 (m, 4H), 3.24-3.36 (m, 2H), 2.30 (s, 3H), 2.23 (s, 3H), 1.89-1.93 (m, 1H), 1.52-1.55 (m, 1H), 1.37-1.43 (m, 1H), 1.06-1.10 (m, 1H).

### Example 56

### 1-{3-(3,5-Dimethylisoxazol-4-yl)-5-[phenyl(tetrahydro-2H-pyran-4-yl)methyl]-5H-pyrrolo[2,3-b:4,5-b']dipyridin-7-yl}ethyl-1-one

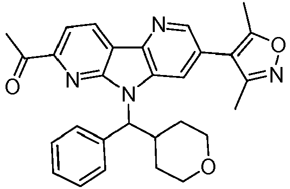

3-(3,5-Dimethylisoxazol-4-yl)-N-methoxy-N-methyl-5-[phenyl(tetrahydro-2H-pyran-4-yl)methyl]-5H-pyrrolo[2,3-b:4,5-b']dipyridine-7-carboxamide (26 mg, 0.050 mmol) was dissolved in anhydrous tetrahydrofuran (1 mL). After substituting the air with nitrogen three times, the resulting mixture was cooled to -20°C in an ice salt bath, and a diethyl ether solution of methylmagnesium bromide (0.3 mol/L, 0.1 mL) was added dropwise. The resulting mixture was kept at the temperature under stirring for 1h, warmed to 0°C, and stirred for additional 2h. A saturated ammonium chloride aqueous solution was added to quench the reaction. The resulting mixture was extracted with dichloromethane twice. The extract was washed with water, and then washed with a saturated saline solution. The solvent was removed by vacuum concentration to obtain a white solid (14 mg, 58%).
¹H NMR (400 MHz, CDCl₃,) δ 8.72 (d, J=8.0 Hz, 1H), 8.49 (d, J=2.0 Hz, 1H), 8.14 (d, J=8.0 Hz, 1H), 7.69 (d, J=2.0 Hz, 1H), 7.60 (d, J=7.2 Hz, 2H), 7.28-7.36 (m, 3H), 5.86 (d, J=10.4 Hz, 1H), 4.03-4.07 (m, 1H), 3.88-3.92 (m, 1H), 3.49-3.56 (m, 2H), 3.35-3.42 (m, 1H), 2.94 (s, 3H), 2.44 (s, 3H), 2.28 (s, 3H), 1.81-1.85 (m, 1H), 1.50-1.61 (m, 2H), 1.38-1.45 (m, 1H).

### Example 57

### 1-{3-(3,5-Dimethylisoxazol-4-yl)-5-[phenyl(tetrahydro-2H-pyran-4-yl)methyl]-5H-pyrrolo[2,3-b:4,5-b']dipyridin-7-yl}ethan-1-ol

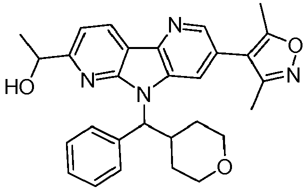

1-{3-(3,5-Dimethylisoxazol-4-yl)-5-[phenyl(tetrahydro-2H-pyran-4-yl)methyl]-5H-pyrrolo[2,3-b:4,5-b']dipyridin-7-yl}ethyl-1-one (14 mg, 0.029 mmol) was dissolved in anhydrous methanol (1 mL), and then sodium borohydride (5 mg, 0.13 mmol) was added. The resulting mixture was stirred at room temperature for 2h. Then water (10 mL) was added, and the resulting mixture was extracted with dichloromethane. The extract was washed with water, and then washed with a saturated saline solution. The solvent was removed by vacuum concentration to obtain a white solid (13 mg, 93%).
¹H NMR (400 MHz, CDCl3,) δ 8.62 (d, J=8.0 Hz, 1H), 8.43 (d, J=1.6 Hz, 1H), 7.63 (d, J=1.6 Hz, 1H), 7.55 (d, J=8.0 Hz, 2H), 7.28-7.36 (m, 4H), 5.94 (d, J=10.8 Hz, 1H), 5.12-5.18 (m, 1H) 4.02-4.07 (m, 2H), 3.86-3.89 (m, 1H), 3.49-3.55 (m, 1H), 3.33-3.39 (m, 2H), 2.41 (s, 3H), 2.25 (s, 3H), 1.84-1.88 (m, 1H), 1.67 (d, J=6.4 Hz, 3H), 1.50-1.61 (m, 1H), 1.35-1.43 (m, 1H), 1.16-1.21 (m, 1H).

### Example 58

### 4-{7-(2-Fluoropropyl-2-yl)-5-[phenyl(tetrahydro-2H-pyran-4-yl)methyl]-5H-pyrrolo[2,3-b:4,5-b']dipyridin-3-yl}-3,5-dimethylisoxazole

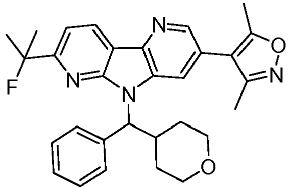

1-{3-(3,5-Dimethylisoxazol-4-yl)-5-[phenyl(tetrahydro-2H-pyran-4-yl)methyl]-5H-pyrrolo[2,3-b:4,5-b']dipyridin-7-yl}ethan-1-ol (20 mg, 0.040 mmol) was dissolved in anhydrous dichloromethane (1 mL), and cooled in an ice water bath under the protection of nitrogen, and then diethylaminosulphur trifluoride (13 mg, 0.080 mmol) was added. The resulting mixture was warmed to room temperature, and stirred for additional 2h. Then water (10 mL) was added, and the resulting mixture was extracted with dichloromethane. The extract was washed with water, and then washed with a saturated saline solution. The solvent was removed by vacuum concentration. The residue was separated with a silica gel prefabricated plate (ethyl acetate/dichloromethane=1:1) to obtain a white solid (3 mg, 15%).
¹H NMR (400 MHz, CDCl₃,) δ 9.25 (br, 1H), 8.42 (s, 1H), 7.96 (s, 1H), 7.75-7.77 (m, 1H), 7.55-7.57 (m, 2H), 7.29-7.37 (m, 3H), 5.86 (d, J=10.4 Hz, 1H), 4.02-4.05 (m, 1H), 3.87-3.91 (m, 1H), 3.46-3.54 (m, 2H), 3.33-3.38 (m, 1H), 2.43 (s, 3H), 2.25 (s, 3H), 1.94 (s, 3H), 1.88 (s, 3H), 1.79-1.82 (m, 1H), 1.48-1.59 (m, 1H), 1.33-1.43 (m, 1H), 1.16-1.19 (m, 1H).

### Example 59

### 3,5-Dimethyl-4- {5-[phenyl(tetrahydro-2H-pyran-4-yl)methyl]-7-(propan-1-en-2-yl)-5H-pyrrolo [2,3-b:4,5-b']dipyridin-3-yl}isoxazole

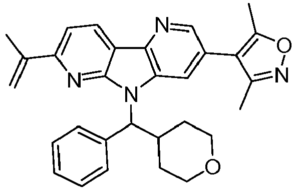

The title compound (5 mg, 26%) can be obtained simultaneously by separation in Example 58.
¹H NMR (400 MHz, CDCl₃,) δ 8.56 (d, J=8.0 Hz, 1H), 8.41 (d, J=2.0 Hz, 1H), 7.61-7.63 (m, 4H), 7.26-7.33 (m, 3H), 6.14 (s, 1H), 5.80 (d, J=10.4 Hz, 1H), 5.49 (s, 1H), 4.01-4.05 (m, 1H), 3.86-3.90 (m, 1H), 3.47-3.62 (m, 2H), 3.35-3.41 (m, 1H), 2.43 (s, 6H), 2.27 (s, 3H), 1.76-1.79 (m, 1H), 1.50-1.54 (m, 1H), 1.36-1.43 (m, 1H), 1.28-1.30 (m, 1H).

### Example 60

### 2-{3-(3,5-Dimethylisoxazol-4-yl)-5-[(3-fluoropyridin-2-yl)(tetrahydro-2H-pyran-4-yl)methyl]-5H-pyrrolo[3,2-b:4,5-b']dipyridin-7-yl}propan-2-ol

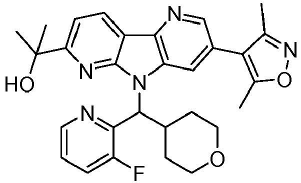

The target product was obtained referring to the synthetic method described in Example 5 and Example 6 with 3-fluoropyridine(tetrahydro-2H-pyran-4-yl)methanol and ethyl 3-(3,5-dimethylisoxazol-4-yl)-5H-pyrrolo[2,3-b:4,5-b']dipyridine-7-carboxylate as starting materials.
¹H NMR (400 MHz, CDCl₃) δ 8.63 (d, 1H), 8.50 (d, 1H), 8.48 (d, 1H), 8.42 (d, 1H), 7.93 (br, 1H), 7.79 (s, 1H), 7.54 (d, 1H), 5.82 (br, 1H), 4.03-4.06 (m, 1H),3.88-3.91 (m, 1H), 3.61-3.78 (m, 2H), 3.43-3.53 (m, 1H), 3.33-3.39 (m, 1H), 2.50 (s, 3H), 2.36 (s, 3H), 1.74 (s, 3H), 1.73 (s, 3H), 1.42-1.58 (m, 2H), 1.20-1.38(m, 2H).

### Bioactivity Assay

### 1. In Vitro Enzymatic Activity Assay of Compounds

IC₅₀ values of the compounds of the present application in inhibiting BRD4 enzyme binding reaction were determined by homogeneous time resolved fluorescence (HTRF). A compound was serially diluted 5-fold with 100% DMSO starting from 1 mM (totally 7 concentrations). 2 µL of the compound at each concentration was added into 48 µL of a reaction buffer solution (20 mM HEPES pH7.5, 150 mM NaCl, 5 mM DTT, 0.005% Tween 20 and 100 µg/ml BSA) to be diluted and fully mixed. Then 2.5 µL of the resulting mixture was added to a 384-well plate (OptiPlate-384, purchased from PerkinElmer), 5 µL of GST-BRD4 (51-462) (purchased from Cisbio) (final concentration: 1 nM) was added, and the the resulting mixture was centrifuged, and fully mixed. Then 2.5 µL of a short peptide Biotin-AHA-SGRGK(Ac)GGK-(Ac)GLGK(Ac)GGAK(Ac)RHRKV) (synthesized by GL Biochem (Shanghai) Ltd) (final concentration: 100 nM) was added to initiate the reaction (total reaction volume: 10 µL). The 384-well plate was placed in an incubator at 23°C to react for 60min, and then 5 µL of Eu3+ cryptate-labled anti-GST antibody (purchased from Cisbio) and 5 µL of Streptavidin-XL-665 (purchased from Cisbio) were added to terminate the reaction. After an additional 1 hour incubation in the incubator, the fluorescence values (excited at 320 nm, emitted light at 665 nm and 620 nm being detected, and the ratio of the two being the enzyme binding signal) were read on Envision (purchased from PerkinElmer). The binding strength of each compound to the BRD4 protein was determined respectively at 7 concentrations, and the data were calculated with GraphPad Prism software to obtain the IC₅₀ value of the compound.

| Compound No. | Compound Structure | BRD4 IC₅₀ (nM) |
|---|---|---|
| 1 | 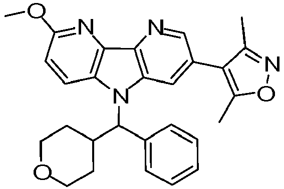 | <2 |
| 3 | 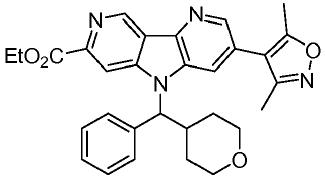 | <2 |
| 4 | 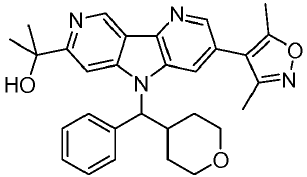 | <2 |
| 6 | 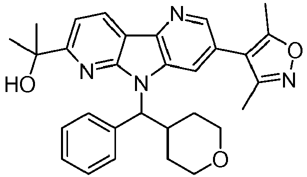 | <2 |
| 9 | 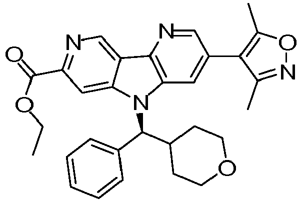 | <2 |
| 10 | 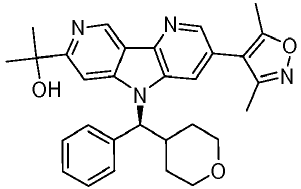 | <2 |
| 12 | 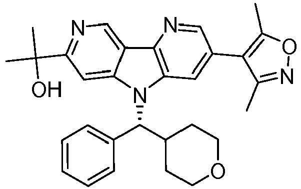 | <2 |
| 16 | 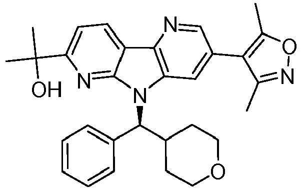 | <2 |
| 18 | 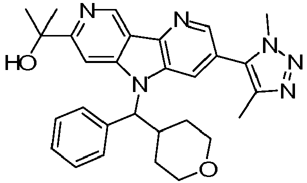 | <2 |
| 20 | 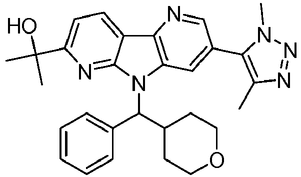 | <2 |
| 22 | 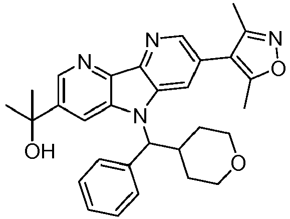 | <2 |
| 24 | 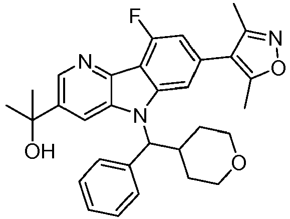 | <2 |
| 26 | 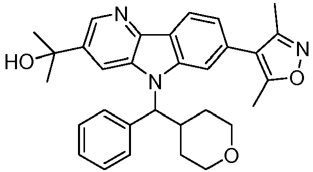 | <2 |
| 37 | 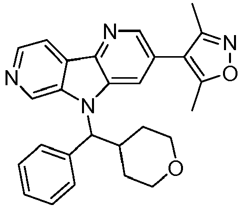 | <2 |
| 39 | 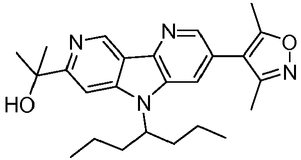 | <2 |
| 41 | 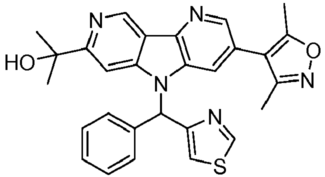 | <2 |
| 43 | 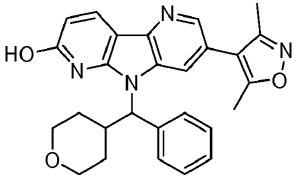 | <2 |
| 50 | 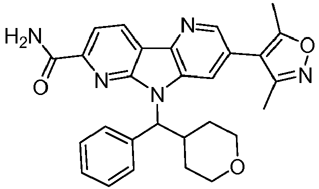 | <2 |
| 52 | 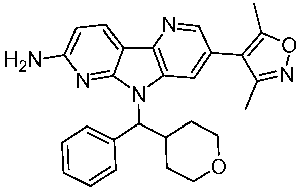 | <2 |
| 55 | 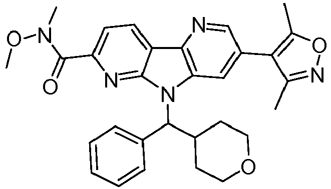 | <2 |
| 56 | 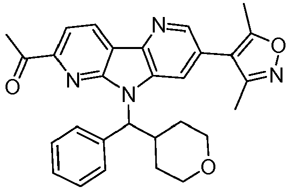 | <2 |
| 57 | 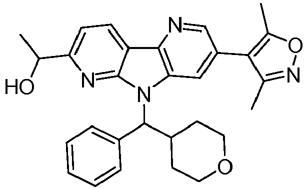 | <2 |
| 60 | 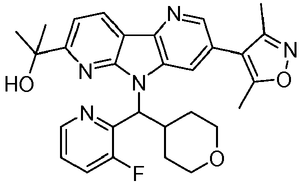 | <2 |

### 2. Cell Proliferation Activity Assay of Compounds

Human acute lymphoblastic leukemia cell line MV4-11 cells were cultured in PRIM1640 medium plus 10% fetal bovine serum (FBS, purchased from Biological Industries, BI) and 1% penicillin/streptomycin double antibody solution (P/S, purchased from Life Technology) at 37°C with 5% CO₂. On the day before the compound was tested, the MV4-11 cells were spread in a 96-well plate (purchased from Corning) at a concentration of 8,000 cells/195µL/well. On the next day, the compound was serially diluted 3-fold with 100% DMSO starting from 10 mM (totally 10 concentrations). 2 µL of the resulting mixture at each concentration was diluted with 48 µL of PRIM1640 medium (purchased from Life Technology). 5 µL of the diluted compound at each concentration was added to the spread cell suspension, and the compound and cells were co-incubated in a cell incubator for 72h (3 days), and then incubated for additional 4h after adding 35 µL of Cell-Titer Blue reagent (purchased from Promega). Then the fluorescence values (excited at 560 nm, detected at 590 nm) were read on Flexstation III (purchased from Molecular Devices), and the data were calculated with GraphPad Prism software to obtain the IC₅₀ value of the compound in inhibiting cell proliferation.

### Biological Data of Some Selected Compounds

The selected compounds prepared above were analyzed according to the biological methods herein. The results are shown in the table below:
IC₅₀ values of the compounds:

| Compound No. | Compound Structure | MV4-11 IC₅₀ (nM) |
|---|---|---|
| 4 | 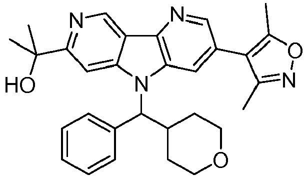 | <10 |
| 10 | 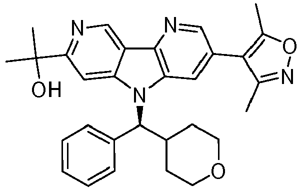 | <10 |
| 12 | 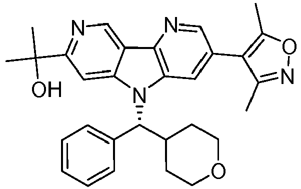 | <10 |
| 16 | 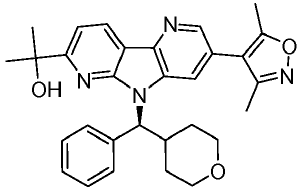 | <10 |
| 18 | 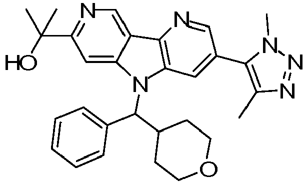 | <10 |
| 22 | 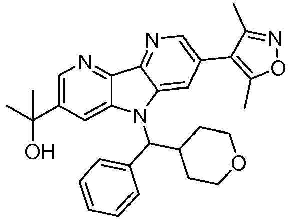 | <10 |
| 24 | 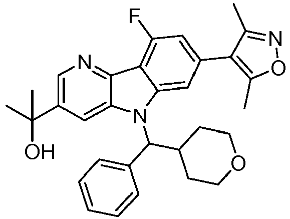 | <10 |
| 26 | 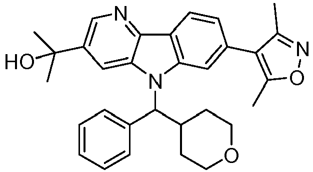 | <10 |
| 43 | 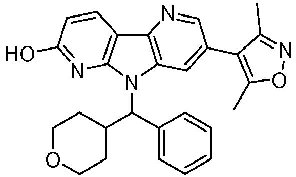 | <10 |
| 50 | 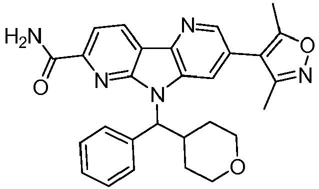 | <10 |
| 52 | 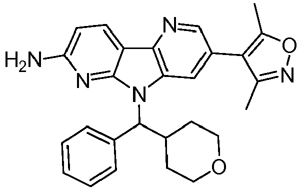 | <10 |

## Claims

1. A compound represented by Formula (III) or a pharmaceutically acceptable salt, solvate, active metabolite, polymorph, ester, isomer or prodrug thereof, 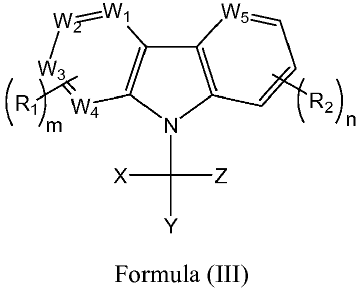 wherein
W₁, W₂, W₃ and W₄ are each independently selected from the group consisting of CH and N, and at least one of them is N;
W₅ is N or CR₃;
X and Y are each independently selected from the group consisting of optionally substituted C₁₋₆ alkyl, optionally substituted C₃₋₈ cycloalkyl, optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S;
Z is selected from the group consisting of hydrogen and C₁₋₆ alkyl;
R₁ is independently selected from the group consisting of halogen, -CN, -OH, -NH₂, -NH-optionally substituted C₁₋₆ alkyl, -C(=O)NH₂, -C(=O)-optionally substituted C₁₋₆ alkyl, - C(=O)N(CH₃)-OCH₃, -C(=O)NH-optionally substituted C₁₋₆ alkyl, -COOH, -C(=O)O-optionally substituted C₁₋₆ alkyl, -OC(=O)NH-optionally substituted C₁₋₆ alkyl, -NHOC(=O)-optionally substituted C₁₋₆ alkyl, -NHC(=O)NH-optionally substituted C₁₋₆ alkyl, -NHSO₂NH-optionally substituted C₁₋₆ alkyl, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₈ cycloalkyl, optionally substituted C₃₋₈ cycloalkyl-CO-, optionally substituted C₃₋₈ cycloalkyl-SO₂-, optionally substituted aryl C₁₋₆ alkoxy, optionally substituted C₃₋₈ cycloalkyl-C₁₋₆ alkoxy, optionally substituted heterocycloalkyl-CO-, optionally substituted heterocycloalkyl, optionally substituted C₁₋₆ alkyl-SO₂-, -NHSO₂-optionally substituted C₁₋₆ alkyl, -N(SO₂-optionally substituted C₁₋₆ alkyl)₂, -NHSO₂-optionally substituted heterocycloalkyl, optionally substituted C₁₋₆ alkyl-NHSO₂- and optionally substituted heterocycloalkyl-NHSO₂-;
R₂ is independently selected from the group consisting of optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S;
R₃ is selected from the group consisting of hydrogen, halogen, -CN, -OH, -NH₂, - NH-C₁₋₆ alkyl, -C(=O)NH-C₁₋₆ alkyl, -COOH, -C(=O)O-C₁₋₆ alkyl, C₁₋₆ alkyl, C₁₋₆ alkoxy and C₃₋₈ cycloalkyl;
m is selected from the group consisting of 0, 1, 2 and 3; and
n is selected from the group consisting of 1, 2 and 3.

2. The compound or a pharmaceutically acceptable salt, solvate, active metabolite, polymorph, ester, isomer or prodrug thereof according to claim 1, wherein W₁ and W₃ are CH, and one of W₂ and W₄ is N and the other is CH.

3. The compound or a pharmaceutically acceptable salt, solvate, active metabolite, polymorph, ester, isomer or prodrug thereof according to claim 1 or 2, wherein
X and Y are each independently selected from the group consisting of C₁₋₆ alkyl, C₃₋₈ cycloalkyl, 6- to 10-membered aryl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, aryl, heteroaryl and heterocycloalkyl may be optionally substituted by halogen, C₁₋₆ alkyl or C₁₋₆ alkoxy;
preferably, X and Y are each independently selected from the group consisting of C₁₋₆ alkyl, C₃₋₈ cycloalkyl, phenyl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, phenyl, heteroaryl and heterocycloalkyl may be optionally substituted by halogen, C₁₋₆ alkyl or C₁₋₆ alkoxy; and
more preferably, X and Y are each independently selected from the group consisting of C₁₋₆ alkyl, C₃₋₈ cycloalkyl, phenyl, pyridyl, thiazolyl and 3- to 8-membered heterocycloalkyl containing 1-3 heteroatoms independently selected from the group consisting of N, O and S, wherein the alkyl, cycloalkyl, phenyl, pyridyl, thiazolyl and heterocycloalkyl may be optionally substituted by halogen, C₁₋₆ alkyl or C₁₋₆ alkoxy.

4. The compound or a pharmaceutically acceptable salt, solvate, active metabolite, polymorph, ester, isomer or prodrug thereof according to any one of claims 1-3, wherein Z is hydrogen.

5. The compound or a pharmaceutically acceptable salt, solvate, active metabolite, polymorph, ester, isomer or prodrug thereof according to any one of claims 1-4, wherein
R₁ is independently selected from the group consisting of halogen, -CN, -OH, -NH₂, -NH-C₁₋₆ alkyl, -C(=O)NH₂, -C(=O)-C₁₋₆ alkyl, -C(=O)N(CH₃)-OCH₃, -C(=O)NH-C₁₋₆ alkyl, - COOH, -C(=O)O-C₁₋₆ alkyl, C₁₋₆ alkyl, C₂₋₆ alkenyl, -C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or -NH₂;
preferably, R₁ is independently selected from the group consisting of -OH, -NH₂, - NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, - SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or -NH₂;
more preferably, R₁ is independently selected from the group consisting of -OH, - NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and -N(SO₂-C₁₋₆ alkyl)₂, wherein the alkyl and alkenyl may be optionally substituted by -F, -Cl, -Br, -OH or - NH₂; and
further preferably, R₁ is selected from the group consisting of -F, -OH,

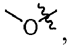

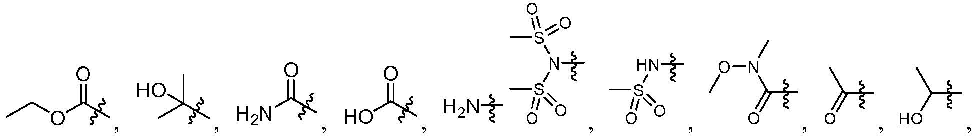

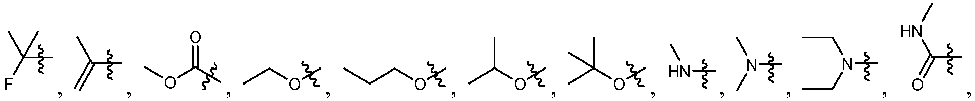

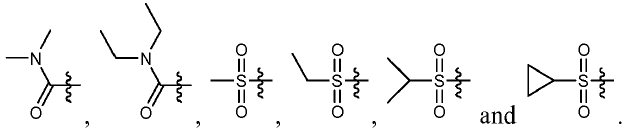

6. The compound or a pharmaceutically acceptable salt, solvate, active metabolite, polymorph, ester, isomer or prodrug thereof according to any one of claims 1-5, wherein
R₂ is independently selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl, C₂₋₆ alkenyl or C₃₋₈ cycloalkyl;
preferably, R₂ is independently selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl; and
more preferably, R₂ is independently selected from the group consisting of the following structures: 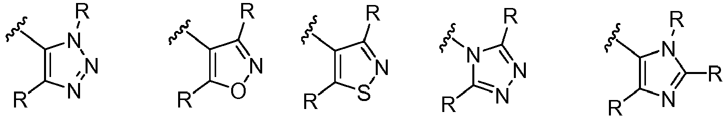 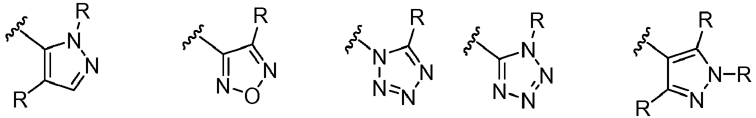 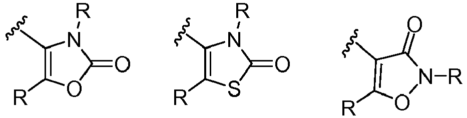 wherein R is independently selected from the group consisting of hydrogen, optionally substituted C₁₋₆ alkyl, optionally substituted C₃₋₈ cycloalkyl-C₁₋₆ alkyl, optionally substituted aryl-C₁₋₆ alkyl, optionally substituted heteroaryl-C₁₋₆ alkyl, optionally substituted heterocycloalkyl-C₁₋₆ alkyl, optionally substituted C₁₋₆ alkyl-CO-, optionally substituted aryl-CO-, optionally substituted C₃₋₈ cycloalkyl-CO-, optionally substituted heteroaryl, optionally substituted heterocycloalkyl-CO-, optionally substituted aryl-SO₂-, optionally substituted C₁₋₆ alkyl-SO₂-, optionally substituted C₃₋₈ cycloalkyl-SO₂-, optionally substituted heteroaryl-SO₂-, optionally substituted C₁₋₆ alkyl-OCO- and optionally substituted C₃₋₈ cycloalkyl-OCO-; preferably, R is independently selected from the group consisting of hydrogen, C₁₋₆ alkyl, C₃₋₈ cycloalkyl, C₁₋₆ alkyl-CO-, aryl-CO-, C₃₋₈ cycloalkyl-CO-, heteroaryl, heterocycloalkyl-CO-, aryl-SO₂-, C₁₋₆ alkyl-SO₂-, C₃₋₈ cycloalkyl-SO₂-, heteroaryl-SO₂-, C₁₋₆ alkyl-OCO- and C₃₋₈ cycloalkyl-OCO-; more preferably, R is independently selected from the group consisting of hydrogen, C₁₋₆ alkyl and heteroaryl; and most preferably, R is independently selected from the group consisting of hydrogen and C₁₋₆ alkyl.

7. The compound or a pharmaceutically acceptable salt, solvate, active metabolite, polymorph, ester, isomer or prodrug thereof according to any one of claims 1-6, wherein R₃ is selected from the group consisting of hydrogen, halogen, C₁₋₆ alkyl and C₃₋₈ cycloalkyl; and preferably, R₃ is selected from the group consisting of hydrogen, halogen and C₁₋₆ alkyl.

8. The compound or a pharmaceutically acceptable salt, solvate, active metabolite, polymorph, ester, isomer or prodrug thereof according to any one of claims 1-7, wherein m is selected from the group consisting of 0, 1 and 2, and n is selected from the group consisting of 1 and 2; and preferably, m and n are 1.

9. The compound or a pharmaceutically acceptable salt, solvate, active metabolite, polymorph, ester, isomer or prodrug thereof according to claim 1, wherein the compound is represented by the following Formula (IV): 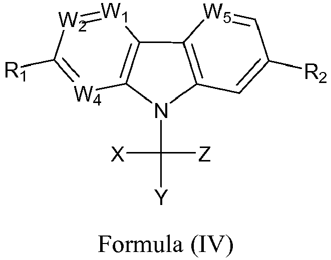 wherein
W₁, W₂ and W₄ are each independently selected from the group consisting of CH and N, and at least one of them is N;
W₅ is N or CR₃;
X and Y are each independently selected from the group consisting of optionally substituted C₁₋₆ alkyl, optionally substituted C₃₋₈ cycloalkyl, optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S;
Z is selected from the group consisting of hydrogen and C₁₋₆ alkyl;
R₁ is selected from the group consisting of halogen, -CN, -OH, -NH₂, -NH-optionally substituted C₁₋₆ alkyl, -C(=O)NH₂, -C(=O)-optionally substituted C₁₋₆ alkyl, - C(=O)N(CH₃)-OCH₃, -C(=O)NH-optionally substituted C₁₋₆ alkyl, -COOH, -C(=O)O-optionally substituted C₁₋₆ alkyl, -OC(=O)NH-optionally substituted C₁₋₆ alkyl, -NHOC(=O)-optionally substituted C₁₋₆ alkyl, -NHC(=O)NH-optionally substituted C₁₋₆ alkyl, -NHSO₂NH-optionally substituted C₁₋₆ alkyl, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₈ cycloalkyl, optionally substituted C₃₋₈ cycloalkyl-CO-, optionally substituted C₃₋₈ cycloalkyl-SO₂-, optionally substituted aryl C₁₋₆ alkoxy, optionally substituted C₃₋₈ cycloalkyl-C₁₋₆ alkoxy, optionally substituted heterocycloalkyl-CO-, optionally substituted heterocycloalkyl, optionally substituted C₁₋₆ alkyl-SO₂-, -NHSO₂-optionally substituted C₁₋₆ alkyl, -N(SO₂-optionally substituted C₁₋₆ alkyl)₂, -NHSO₂-optionally substituted heterocycloalkyl, optionally substituted C₁₋₆ alkyl-NHSO₂- and optionally substituted heterocycloalkyl-NHSO₂-;
R₂ is selected from the group consisting of optionally substituted 6- to 10-membered aryl, optionally substituted 5- to 7-membered heteroaryl containing 1-3 heteroatoms independently selected from the group consisting of N, O and S, and optionally substituted 3- to 8-membered heterocycloalkyl containing 1-3 heteroatoms independently selected from the group consisting of N, O and S; and
R₃ is selected from the group consisting of hydrogen, halogen, -CN, -OH, -NH₂, - NH-C₁₋₆ alkyl, -C(=O)NH-C₁₋₆ alkyl, -COOH, -C(=O)O-C₁₋₆ alkyl, C₁₋₆ alkyl, C₁₋₆ alkoxy and C₃₋₈ cycloalkyl.

10. The compound or a pharmaceutically acceptable salt, solvate, active metabolite, polymorph, ester, isomer or prodrug thereof according to claim 9, wherein W₁ is CH, and one of W₂ and W₄ is N and the other is CH.

11. The compound or a pharmaceutically acceptable salt, solvate, active metabolite, polymorph, ester, isomer or prodrug thereof according to claim 9 or 10, wherein
X and Y are each independently selected from the group consisting of C₁₋₆ alkyl, C₃₋₈ cycloalkyl, 6- to 10-membered aryl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, aryl, heteroaryl and heterocycloalkyl may be optionally substituted by halogen, C₁₋₆ alkyl or C₁₋₆ alkoxy;
preferably, X and Y are each independently selected from the group consisting of C₁₋₆ alkyl, C₃₋₈ cycloalkyl, phenyl, 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S, wherein the alkyl, cycloalkyl, phenyl, heteroaryl and heterocycloalkyl may be optionally substituted by halogen, C₁₋₆ alkyl or C₁₋₆ alkoxy; and
more preferably, X and Y are each independently selected from the group consisting of C₁₋₆ alkyl, C₃₋₈ cycloalkyl, phenyl, pyridyl, thiazolyl and 3- to 8-membered heterocycloalkyl containing 1-3 heteroatoms independently selected from the group consisting of N, O and S, wherein the alkyl, cycloalkyl, phenyl, pyridyl, thiazolyl and heterocycloalkyl may be optionally substituted by halogen, C₁₋₆ alkyl or C₁₋₆ alkoxy.

12. The compound or a pharmaceutically acceptable salt, solvate, active metabolite, polymorph, ester, isomer or prodrug thereof according to any one of claims 9-11, wherein Z is hydrogen.

13. The compound or a pharmaceutically acceptable salt, solvate, active metabolite, polymorph, ester, isomer or prodrug thereof according to any one of claims 9-12, wherein
R₁ is selected from the group consisting of halogen, -CN, -OH, -NH₂, -NH-C₁₋₆ alkyl, -C(=O)NH₂, -C(=O)-C₁₋₆ alkyl, -C(=O)N(CH₃)-OCH₃, -C(=O)NH-C₁₋₆ alkyl, -COOH, - C(=O)O-C₁₋₆ alkyl, C₁₋₆ alkyl, C₂₋₆ alkenyl, -C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by F, Cl, Br, -OH or - NH₂; preferably, R₁ is selected from the group consisting of -OH, -NH₂, -NH-C₁₋₆ alkyl, - C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, - NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or -NH₂;
more preferably, R₁ is selected from the group consisting of -OH, -NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and -N(SO₂-C₁₋₆ alkyl)₂, wherein the alkyl and alkenyl may be optionally substituted by -F, -Cl, -Br, -OH or -NH₂; and
further preferably, R₁ is selected from the group consisting of -F, -OH,

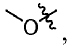

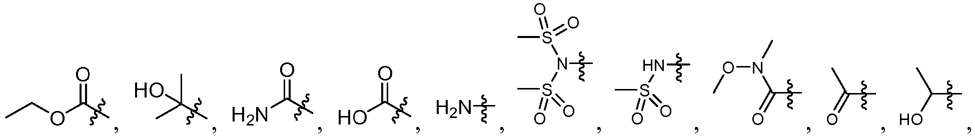

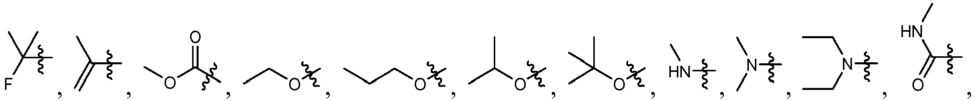

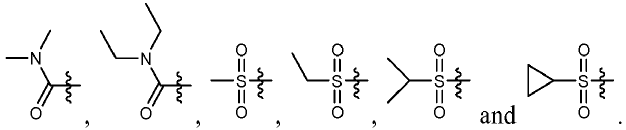

14. The compound or a pharmaceutically acceptable salt, solvate, active metabolite, polymorph, ester, isomer or prodrug thereof according to any one of claims 9-13, wherein
R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl, C₂₋₆ alkenyl or C₃₋₈ cycloalkyl;
preferably, R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl; and
more preferably, R₂ is selected from the group consisting of the following structures: 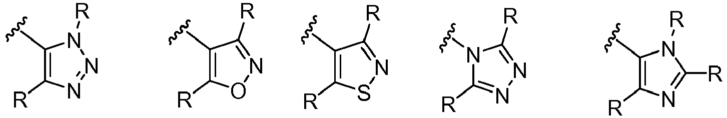 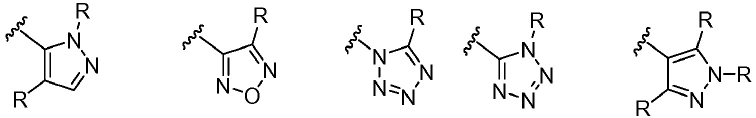 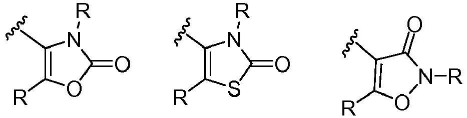 wherein R is selected from the group consisting of hydrogen, optionally substituted C₁₋₆ alkyl, optionally substituted C₃₋₈ cycloalkyl-C₁₋₆ alkyl, optionally substituted aryl-C₁₋₆ alkyl, optionally substituted heteroaryl-C₁₋₆ alkyl, optionally substituted heterocycloalkyl-C₁₋₆ alkyl, optionally substituted C₁₋₆ alkyl-CO-, optionally substituted aryl-CO-, optionally substituted C₃₋₈ cycloalkyl-CO-, optionally substituted heteroaryl, optionally substituted heterocycloalkyl-CO-, optionally substituted aryl-SO₂-, optionally substituted C₁₋₆ alkyl-SO₂-, optionally substituted C₃₋₈ cycloalkyl-SO₂-, optionally substituted heteroaryl-SO₂-, optionally substituted C₁₋₆ alkyl-OCO- and optionally substituted C₃₋₈ cycloalkyl-OCO-; preferably, R is selected from the group consisting of hydrogen, C₁₋₆ alkyl, C₃₋₈ cycloalkyl, C₁₋₆ alkyl-CO-, aryl-CO-, C₃₋₈ cycloalkyl-CO-, heteroaryl, heterocycloalkyl-CO-, aryl-SO₂-, C₁₋₆ alkyl-SO₂-, C₃₋₈ cycloalkyl-SO₂-, heteroaryl-SO₂-, C₁₋₆ alkyl-OCO- and C₃₋₈ cycloalkyl-OCO-; more preferably, R is selected from the group consisting of hydrogen, C₁₋₆ alkyl and heteroaryl; and most preferably, R is selected from the group consisting of hydrogen and C₁₋₆ alkyl.

15. The compound or a pharmaceutically acceptable salt, solvate, active metabolite, polymorph, ester, isomer or prodrug thereof according to any one of claims 9-14, wherein R₃ is selected from the group consisting of hydrogen, halogen, C₁₋₆ alkyl and C₃₋₈ cycloalkyl; and preferably, R₃ is selected from the group consisting of hydrogen, halogen and C₁₋₆ alkyl.

16. The compound or a pharmaceutically acceptable salt, solvate, active metabolite, polymorph, ester, isomer or prodrug thereof according to claim 1, wherein the compound is represented by the following Formula (V): 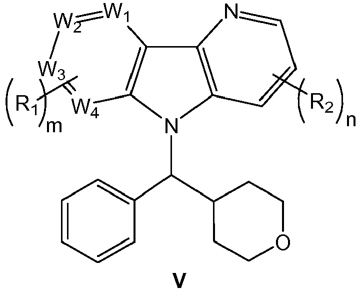 wherein
W₁, W₂, W₃ and W₄ are each independently selected from the group consisting of CH and N, and at least one of them is N;
R₁ is selected from the group consisting of halogen, -CN, -OH, -NH₂, -NH-optionally substituted C₁₋₆ alkyl, -C(=O)NH₂, -C(=O)-optionally substituted C₁₋₆ alkyl, - C(=O)N(CH₃)-OCH₃, -C(=O)NH-optionally substituted C₁₋₆ alkyl, -COOH, -C(=O)O-optionally substituted C₁₋₆ alkyl, -OC(=O)NH-optionally substituted C₁₋₆ alkyl, -NHOC(=O)-optionally substituted C₁₋₆ alkyl, -NHC(=O)NH-optionally substituted C₁₋₆ alkyl, -NHSO₂NH-optionally substituted C₁₋₆ alkyl, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₈ cycloalkyl, optionally substituted C₃₋₈ cycloalkyl-CO-, optionally substituted C₃₋₈ cycloalkyl-SO₂-, optionally substituted aryl C₁₋₆ alkoxy, optionally substituted C₃₋₈ cycloalkyl-C₁₋₆ alkoxy, optionally substituted heterocycloalkyl-CO-, optionally substituted heterocycloalkyl, optionally substituted C₁₋₆ alkyl-SO₂-, -NHSO₂-optionally substituted C₁₋₆ alkyl, -N(SO₂-optionally substituted C₁₋₆ alkyl)₂, -NHSO₂-optionally substituted heterocycloalkyl, optionally substituted C₁₋₆ alkyl-NHSO₂- and optionally substituted heterocycloalkyl-NHSO₂-;
R₂ is selected from the group consisting of optionally substituted 6- to 10-membered aryl, optionally substituted 5- to 7-membered heteroaryl containing 1-3 heteroatoms independently selected from the group consisting of N, O and S, and 3- to 8-membered heterocycloalkyl containing 1-3 heteroatoms independently selected from the group consisting of N, O and S;
m is selected from the group consisting of 0, 1, 2 and 3; and
n is selected from the group consisting of 1, 2 and 3.

17. The compound or a pharmaceutically acceptable salt, solvate, active metabolite, polymorph, ester, isomer or prodrug thereof according to claim 16, wherein one or two of W₁, W₂, W₃ and W₄ are N, and the others are CH; and preferably, one of W₁, W₂, W₃ and W₄ is N, and the others are CH.

18. The compound or a pharmaceutically acceptable salt, solvate, active metabolite, polymorph, ester, isomer or prodrug thereof according to claim 16 or 17, wherein
R₁ is selected from the group consisting of halogen, -CN, -OH, -NH₂, -NH-C₁₋₆ alkyl, -C(=O)NH₂, -C(=O)-C₁₋₆ alkyl, -C(=O)N(CH₃)-OCH₃, -C(=O)NH-C₁₋₆ alkyl, -COOH, - C(=O)O-C₁₋₆ alkyl, C₁₋₆ alkyl, C₂₋₆ alkenyl, -C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or - NH₂;
preferably, R₁ is selected from the group consisting of -OH, -NH₂, -NH-C₁₋₆ alkyl, - C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, - NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or -NH₂;
more preferably, R₁ is selected from the group consisting of -OH, -NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and -N(SO₂-C₁₋₆ alkyl)₂, wherein the alkyl and alkenyl may be optionally substituted by -F, -Cl, -Br, -OH or -NH₂; and
further preferably, R₁ is selected from the group consisting of -F, -OH,

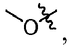

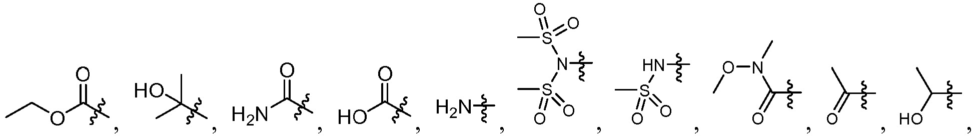

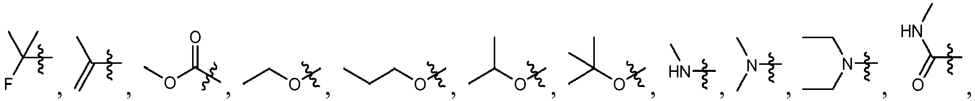

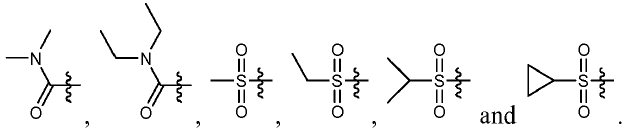

19. The compound or a pharmaceutically acceptable salt, solvate, active metabolite, polymorph, ester, isomer or prodrug thereof according to any one of claims 16-18, wherein R₁ is connected to W₁, W₂ or W₃; and preferably, R₁ is connected to W₂ or W₃.

20. The compound or a pharmaceutically acceptable salt, solvate, active metabolite, polymorph, ester, isomer or prodrug thereof according to any one of claims 16-19, wherein
R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl, C₂₋₆ alkenyl or C₃₋₈ cycloalkyl;
preferably, R₂ is selected from the group consisting of 5- to 7-membered heteroaryl containing 1-3 heteroatoms independently selected from the group consisting of N, O and S and 3- to 8-membered heterocycloalkyl containing 1-3 heteroatoms independently selected from the group consisting of N, O and S, wherein the heteroaryl and heterocycloalkyl may be optionally substituted by C₁₋₆ alkyl; and
more preferably, R₂ is independently selected from the group consisting of the following structures: 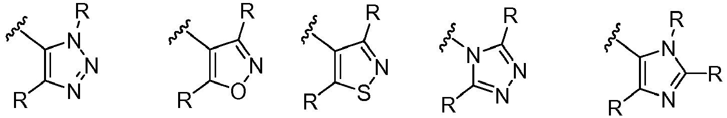 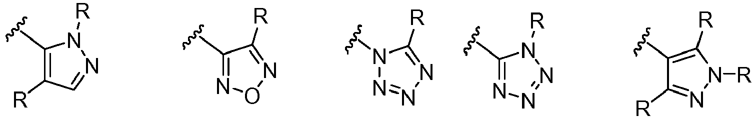 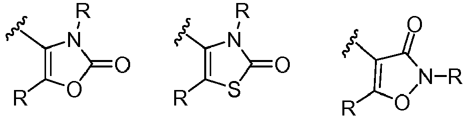 wherein R is selected from the group consisting of hydrogen, optionally substituted C₁₋₆ alkyl, optionally substituted C₃₋₈ cycloalkyl-C₁₋₆ alkyl, optionally substituted aryl-C₁₋₆ alkyl, optionally substituted heteroaryl-C₁₋₆ alkyl, optionally substituted heterocycloalkyl-C₁₋₆ alkyl, optionally substituted C₁₋₆ alkyl-CO-, optionally substituted aryl-CO-, optionally substituted C₃₋₈ cycloalkyl-CO-, optionally substituted heteroaryl, optionally substituted heterocycloalkyl-CO-, optionally substituted aryl-SO₂-, optionally substituted C₁₋₆ alkyl-SO₂-, optionally substituted C₃₋₈ cycloalkyl-SO₂-, optionally substituted heteroaryl-SO₂-, optionally substituted C₁₋₆ alkyl-OCO- and optionally substituted C₃₋₈ cycloalkyl-OCO-; preferably, R is selected from the group consisting of hydrogen, C₁₋₆ alkyl, C₃₋₈ cycloalkyl, C₁₋₆ alkyl-CO-, aryl-CO-, C₃₋₈ cycloalkyl-CO-, heteroaryl, heterocycloalkyl-CO-, aryl-SO₂-, C₁₋₆ alkyl-SO₂-, C₃₋₈ cycloalkyl-SO₂-, heteroaryl-SO₂-, C₁₋₆ alkyl-OCO- and C₃₋₈ cycloalkyl-OCO-; more preferably, R is selected from the group consisting of hydrogen, C₁₋₆ alkyl and heteroaryl; and most preferably, R is selected from the group consisting of hydrogen and C₁₋₆ alkyl.

21. The compound or a pharmaceutically acceptable salt, solvate, active metabolite, polymorph, ester, isomer or prodrug thereof according to claim 1, wherein the compound is represented by the following Formula (VI): 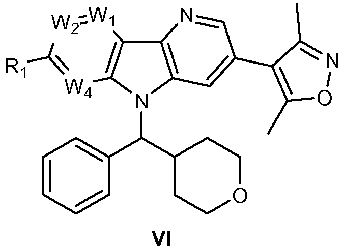 wherein
W₁, W₂ and W₄ are each independently CH or N, and at least one of them is N; and
R₁ is selected from the group consisting of halogen, -CN, -OH, -NH₂, -NH-optionally substituted C₁₋₆ alkyl, -C(=O)NH₂, -C(=O)-optionally substituted C₁₋₆ alkyl, - C(=O)N(CH₃)-OCH₃, -C(=O)NH-optionally substituted C₁₋₆ alkyl, -COOH, -C(=O)O-optionally substituted C₁₋₆ alkyl, -OC(=O)NH-optionally substituted C₁₋₆ alkyl, -NHOC(=O)-optionally substituted C₁₋₆ alkyl, -NHC(=O)NH-optionally substituted C₁₋₆ alkyl, -NHSO₂NH-optionally substituted C₁₋₆ alkyl, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₈ cycloalkyl, optionally substituted C₃₋₈ cycloalkyl-CO-, optionally substituted C₃₋₈ cycloalkyl-SO₂-, optionally substituted aryl C₁₋₆ alkoxy, optionally substituted C₃₋₈ cycloalkyl-C₁₋₆ alkoxy, optionally substituted heterocycloalkyl-CO-, optionally substituted heterocycloalkyl, optionally substituted C₁₋₆ alkyl-SO₂-, -NHSO₂-optionally substituted C₁₋₆ alkyl, -N(SO₂-optionally substituted C₁₋₆ alkyl)₂, -NHSO₂-optionally substituted heterocycloalkyl, optionally substituted C₁₋₆ alkyl-NHSO₂- and optionally substituted heterocycloalkyl-NHSO₂-.

22. The compound or a pharmaceutically acceptable salt, solvate, active metabolite, polymorph, ester, isomer or prodrug thereof according to claim 21, wherein W₁ is CH, and one of W₂ and W₄ is N and the other is CH; preferably, W₂ is N, and W₁ and W₄ are CH; or W₄ is N, and W₁ and W₂ are CH; or W₁ is N, and W₂ and W₄ are CH.

23. The compound or a pharmaceutically acceptable salt, solvate, active metabolite, polymorph, ester, isomer or prodrug thereof according to claim 21 or 22, wherein
R₁ is selected from the group consisting of halogen, -CN, -OH, -NH₂, -NH-C₁₋₆ alkyl, -C(=O)NH₂, -C(=O)-C₁₋₆ alkyl, -C(=O)N(CH₃)-OCH₃, -C(=O)NH-C₁₋₆ alkyl, -COOH,-C(=O)O-C₁₋₆ alkyl, C₁₋₆ alkyl, C₂₋₆ alkenyl, -C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl, -NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or-NH₂;
preferably, R₁ is selected from the group consisting of -OH, -NH₂, -NH-C₁₋₆ alkyl,-C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, C₃₋₈ cycloalkyl, 3- to 8-membered heterocycloalkyl containing 1-3 heteroatoms independently selected from the group consisting of N, O and S, -SO₂-C₁₋₆ alkyl,-NHSO₂-C₁₋₆ alkyl and -N(SO₂-C₁₋₆ alkyl)₂, wherein the alkyl, alkenyl, cycloalkyl and heterocycloalkyl may be optionally substituted by -F, -Cl, -Br, -OH or -NH₂;
more preferably, R₁ is selected from the group consisting of -OH, -NH₂, -NH-C₁₋₆ alkyl, -C(=O)NH₂, -C(=O)-C₁₋₆ alkyl, -C(=O)NH-C₁₋₆ alkyl, -COOH, -C(=O)O-C₁₋₆ alkyl, C₁₋₆ alkyl, C₂₋₆ alkenyl, C₁₋₆ alkoxy, -SO₂-C₁₋₆ alkyl, -NHSO₂-C₁₋₆ alkyl and -N(SO₂-C₁₋₆ alkyl)₂, wherein the alkyl and alkenyl may be optionally substituted by -F, -Cl, -Br, -OH or -NH₂; and
further preferably, R₁ is selected from the group consisting of -F, -OH,

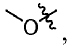

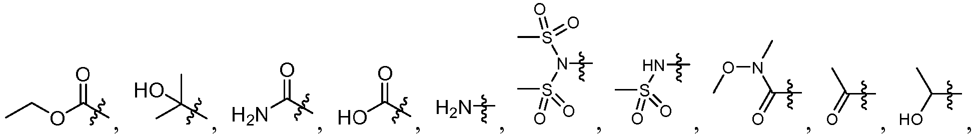

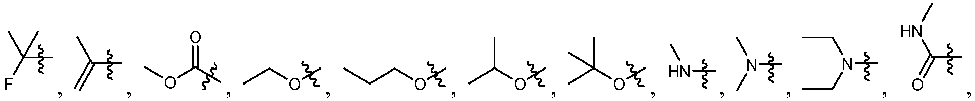

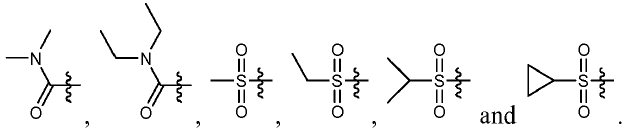

24. The compound or a pharmaceutically acceptable salt, solvate, active metabolite, polymorph, ester, isomer or prodrug thereof according to claim 1, wherein the compound is represented by the following structural formulae:

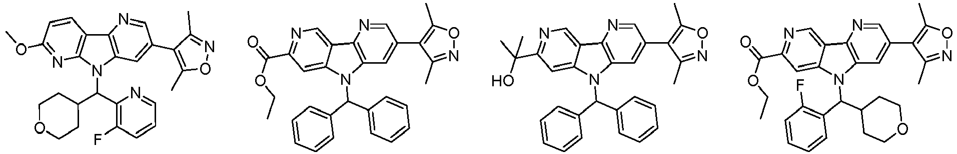

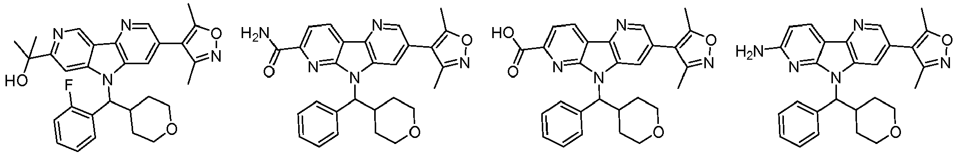

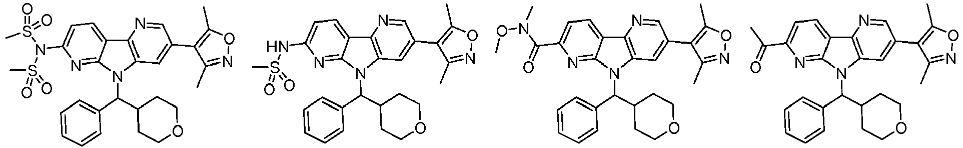

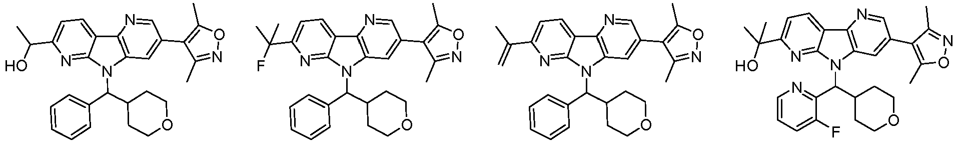

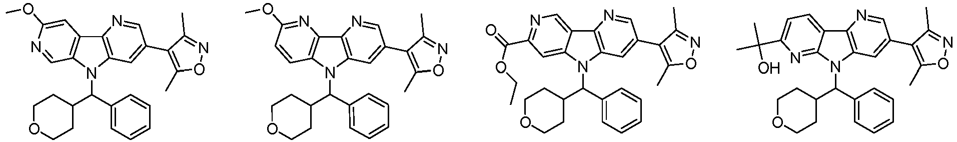

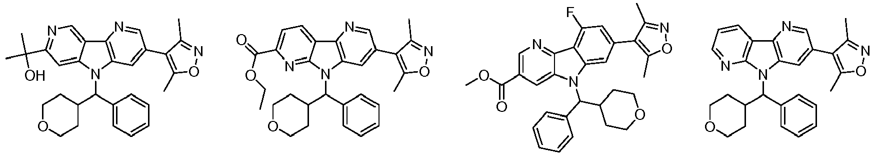

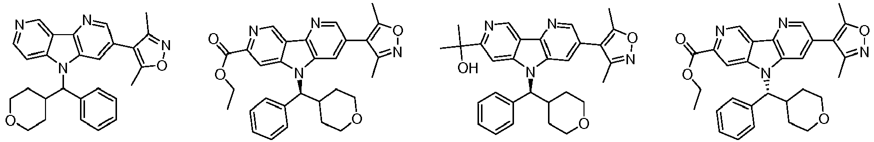

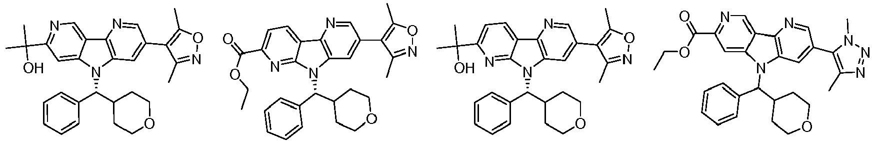

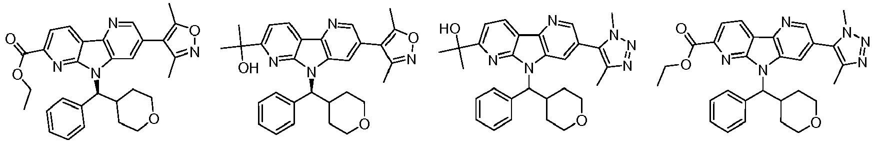

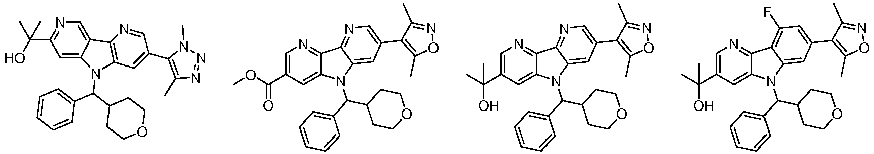

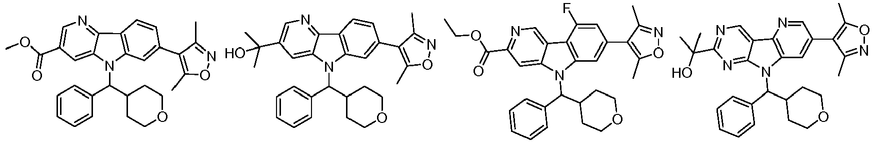

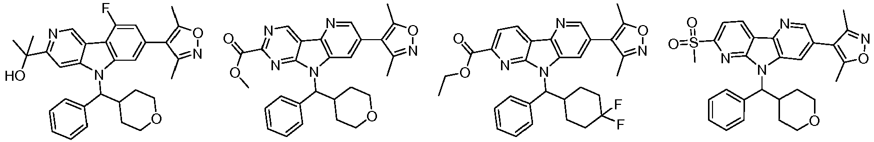

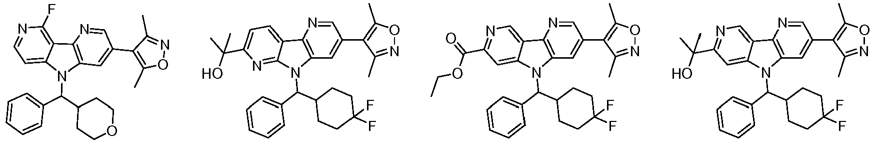

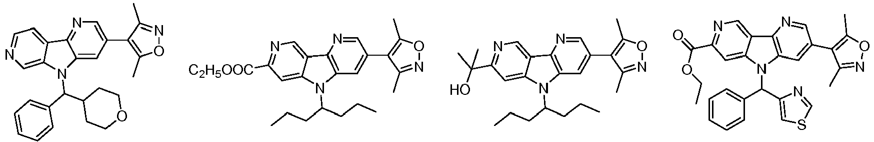

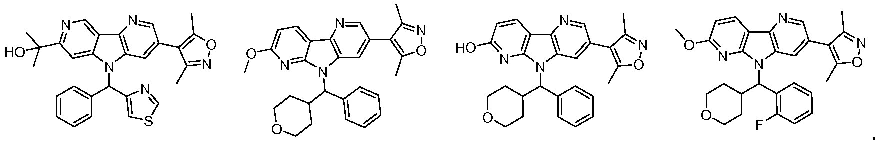

25. A pharmaceutical composition, comprising a) the compound or a pharmaceutically acceptable salt, solvate, active metabolite, polymorph, ester, isomer or prodrug thereof according to any one of claims 1-24, and b) a pharmaceutically acceptable carrier.

26. Use of the compound or a pharmaceutically acceptable salt, solvate, active metabolite, polymorph, ester, isomer or prodrug thereof according to any one of claims 1-24 or the pharmaceutical composition according to claim 25 in the preparation of a medicament for treating a disease associated with BET proteins.

27. A method for treating a disease associated with BET proteins, comprising administering to a patient in need thereof a therapeutically effective amount of the compound or a pharmaceutically acceptable salt, solvate, active metabolite, polymorph, ester, isomer or prodrug thereof according to any one of claims 1-24 or the pharmaceutical composition according to claim 25.

28. The compound or a pharmaceutically acceptable salt, solvate, active metabolite, polymorph, ester, isomer or prodrug thereof according to any one of claims 1-24 or the pharmaceutical composition according to claim 25 for use in treating a disease associated with BET proteins.
